# EUROPEAN PATENT APPLICATION

(11) **EP 3 858 836 A1**
(43) Date of publication of application: **04.08.2021**
(21) Application number: 19865985.6
(22) Date of filing: 18.06.2019
(51) Int. Cl.: C07D 493/22, C07D 493/04, C07D 239/553, A61K 31/365, A61K 31/505, A61K 31/385, A61P 35/00

(54) **COMPOUND AND USE THEREOF**

(30) Priority: 28.09.2018 WO PCT/CN2018/108232
(71) Applicant: Novagenesis Therapeutix (Suzhou) Limited, Suzhou, Jiangsu 215000 (CN)
(72) Inventor: QIAN, Feng, Beijing 100084 (CN); LIU, Zhengsheng, Beijing 100084 (CN)
(74) Representative: Gill, Siân Victoria
(86) International application number: PCT/CN2019/091678
(87) International publication number: WO 2020/062951

(57) **Abstract**

A compound represented by formula (I), or an isomer, a stereoisomer, a geometric isomer, a tautomer, a nitrogen oxide, a hydrate, a solvate, a metabolite, pharmaceutically acceptable salts or a prodrug thereof.

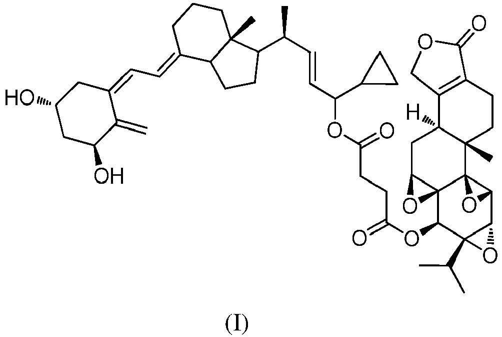

## Description

### TECHNICAL FIELD

The present disclosure relates to the field of medicine, and particularly, the present disclosure relates to a compound and use thereof. More specifically, the present disclosure relates to a compound, a compound represented by formula (I), a pharmaceutical composition containing the compound, a compound and use thereof, and a preparation method of the compound.

### BACKGROUND

Pancreatic cancer is a malignant tumor of the digestive tract that is highly malignant and difficult to diagnose and treat. About 90% thereof is ductal adenocarcinoma that originates in the glandular epithelium. Its morbidity and mortality rates have significantly increased in recent years. With a 5-year survival rate lower than 5%, pancreatic cancer is one of the malignant tumors with the worst prognosis. The early diagnosis rate of pancreatic cancer is not high, the surgical mortality is high, and the cure rate is very low.

The cause of pancreatic cancer is not clear yet. Its occurrence may be related to smoking, drinking, high-fat and high-protein diets, excessive drinking of coffee, environmental pollution, and genetic factors. The survey reports in recent years have found that the morbidity of pancreatic cancer in diabetic patients is significantly higher than that in the general population. Patients with pancreatitis may have a certain relation with the morbidity of pancreatic cancer, and it has been found that, among the patients with chronic pancreatitis, a proportion of suffering pancreatic cancer is increased significantly. In addition, many factors, such as occupation, environment, and geography, are related to the occurrence of this disease.

The clinical manifestations of pancreatic cancer depend on a location of the cancer, a course of the disease, presence or absence of metastasis, and involvement of adjacent organs. The clinical features thereof are short course, and rapid development and deterioration. The most common manifestations include fullness, discomfort and pain in the upper abdomen. Despite of the conscious pain, not all patients can feel tenderness. The location of tenderness, if exists, is consistent with that of the conscious pain.

The current research indicates that the pathological characteristics of pancreatic cancer tissues show that stroma occupies the vast majority of cancer tissues, about 80%, while cancer cells take the minority. Such pathological characteristics of pancreatic cancer tissues result in the extremely deficient vascular system, thereby leading to the difficulty in medicine administration and ineffective immunotherapy.

Therefore, it is major problem to be solved by scientists to search for an effective treatment for pancreatic cancer.

### SUMMARY

The present disclosure is based on Applicant's discovery and knowledge of the following facts and problems:

In pancreatic cancer tissues, cancer cells account for only about 20% thereof, and extracellular matrix (stroma) accounts for more than 70%. The extracellular matrix includes fibroblasts, immune and inflammatory cells, endothelial cells, and complex extracellular media. One clinical manifestation of the pancreatic cancer tissue is hard texture, and pathological examination reveals that a large amount of collagen fibers surrounds a relatively small amount of pancreatic cancer cells. The small amount of cancer cells, under a "protection" of the tumor stroma, can successfully escape the surveillance of the body's immune system, and thus rampantly proliferate, differentiate and metastasize.

The occurrence and development of stroma is directly related to pancreatic stellate cells (PSCs). PSCs are one type of fibroblast located at a base of pancreatic acinar cells. Under normal circumstances, the PSCs are in a dormant state, the cytoplasm thereof contains a large number of lipid droplets containing vitamin A, and the PSCs can normally secrete enzymes or inhibitors for synthesis and degradation of the extracellular matrix to maintain a balance between the secretion and degradation of the extracellular matrix. When the pancreas is damaged or pancreatic inflammation occurs, the PSCs will be activated by a stimulation of cellular kinases, growth factors, hypoxic pressure, etc. The activated PSCs will lose the lipid droplets in the cytoplasm, while rapidly proliferating and secreting a large amount of cell growth factor receptors (vascular endothelial growth factor receptor VEGFR, fibroblast growth factor receptor, etc.), extracellular matrix proteins (including collagen, laminin, integrin) and matrix protein degrading enzyme inhibitors (metalloproteinase inhibitors, hyaluronidase inhibitors, etc.), and the like. The activation of PSCs breaks the balance of the components outside the pancreatic cancer tissue, a large amount of connective tissue proliferates, and matrix degrading enzymes are less secreted, which in turns increases a content of extra-tumor matrix greatly. This complex stroma-rich microenvironment, on the one hand, protects tumor cells from the immune surveillance and invalid many immunotherapies; and on the other hand, it limits a formation of tumor blood vessels, and as a physical barrier, greatly limits the entry of anti-tumor chemotherapy agents into the tumor cells. Thus, the chemotherapy agents lose their efficacy, which ultimately promotes an evolution, invasion, and metastasis of the tumor.

In this regard, specifically targeting the "prime culprit' - the activated PSCs produced by the pancreatic cancer stroma, Applicant employs small molecule medicines to promote the PSCs to be in the "dormant state", so as to reduce the production of stroma. The small molecule medicines are chemically coupled at the same time to obtain a coupling molecule, which has a dual targeting function, i.e., simultaneously targeting pancreatic cancer cells and pancreatic stellate cells, and has better formulation and druggability. Further, by adopting appropriate formulations, the small molecule medicines can have improved pharmacokinetic properties, so as to have the optimal synergistic effect on the production of stroma and the inhibition of cancer cells, thereby improving the drug resistance environment of pancreatic tumors and enhancing the effective killing of tumor cells.

The specific scheme design and effects of the present disclosure are described as follows:
As an example, calcipotriol (Cal) is used as a small molecule inhibitor acting on the PSCs, and triptolide (TP) is used as a highly effective small molecule chemotherapy medicine. Applicant designed to connect triptolide and calcipotriol via an enzyme-degradable linker, to synthesize a brand new, dual-targeting coupling compound. This compound is named as "Callide" in the present disclosure. Compared with the original medicine TP, Callide has an increased molecular weight and a slower crystallization tendency, and it is more hydrophobic. Therefore, Callide can be easily loaded and delivered by means of a variety of nano-drug delivery systems (such as polymer micelles, albumin composite nanoparticles, liposomes, etc.), thereby optimizing pharmacokinetics of Callide and increasing a concentration of Callide at the tumor site, and reducing toxic and side effects of TP. Meanwhile, Callide can be only catalytically degraded by esterase in vivo to produce Cal and TP, such that the pharmacokinetics of Cal and TP can be better synchronized, thereby achieving the dual targeting and synergistic effects of promoting the PSC dormancy (by calcipotriol) and inhibiting the pancreatic cancer cells (by triptolide), and significantly reducing the toxic and side effects.

In view of the above, in a first aspect, the present disclosure provides a compound. According to the embodiments of the present disclosure, the compound includes a tumor stroma-regulating group and a cytotoxic group that are coupled to each other. The tumor stroma-regulating group is used to regulate the tumor stroma, and the cytotoxic group is used to kill the tumor cells. The compound according to the embodiments of the present disclosure can act on the tumor stroma and the tumor cells at the same time, so as to achieve the purpose of eliminating or reducing the tumor stroma while killing tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

According to the embodiments of the present disclosure, the above-mentioned compound can further have at least one of the following additional technical features.

According to the embodiments of the present disclosure, the tumor stroma-regulating group includes at least one selected from calcipotriol (Cal), cyclopamine, Ganciclovir (GCV), Fingolimod, all-trans retinoic acid (ATRA), and hyaluronidase (HA).

According to the embodiments of the present disclosure, the cytotoxic group includes at least one selected from triptolide (TP), paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, and Lapatinib.

The compound, which is obtained by arbitrarily combining the above-described tumor stroma-regulating groups and cytotoxic groups, can act on the tumor stroma and the tumor cells at the same time, thereby achieving the purpose of eliminating or reducing the tumor stroma while killing the tumor cells.

According to the embodiments of the present disclosure, the compound further includes an enzyme-degradable linker. In this way, the compound can be enzymatically degraded in vivo to produce medicines of the tumor stroma-regulating group and the cytotoxic group, which better synchronizes the pharmacokinetics of the medicines of the tumor stroma-regulating group and cytotoxic group, thereby achieving the synergistic effect of promoting PSC dormancy and inhibiting tumor cells.

According to the embodiments of the present disclosure, the enzyme-degradable linker has one of the following structures:

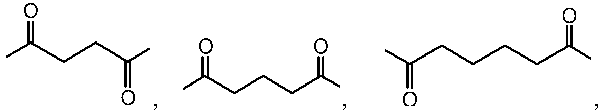

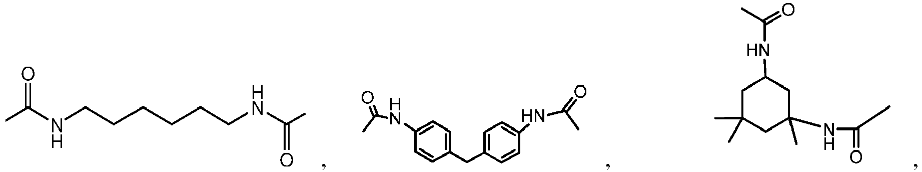

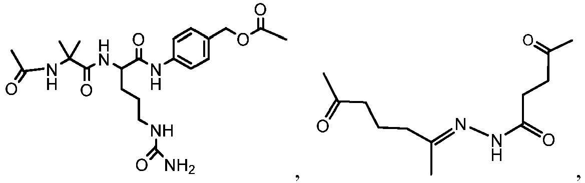

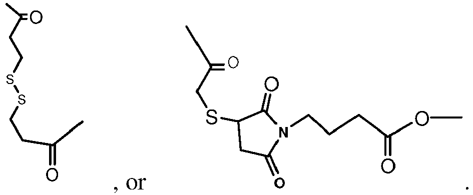

In a second aspect of the present disclosure, the present disclosure provides a compound. According to the embodiments of the present disclosure, the compound includes one of the following structures, or includes one of isomers, stereoisomers, geometric isomers, tautomers, nitrogen oxides, hydrates, solvates, metabolites, pharmaceutically acceptable salts or prodrugs of the following structures: 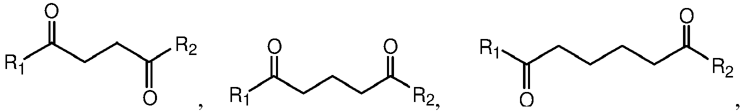 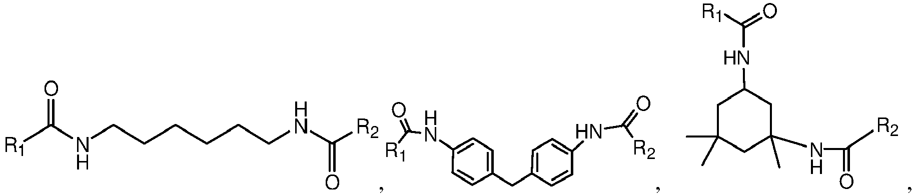 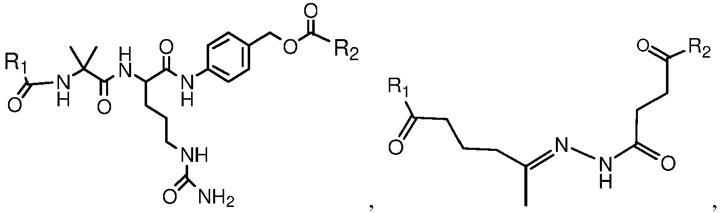 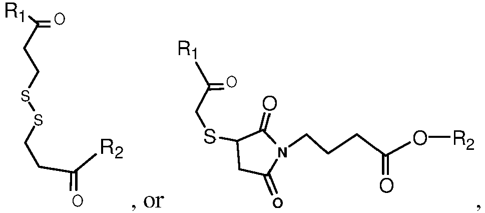
where R₁ is independently calcipotriol, cyclopamine, Ganciclovir, Fingolimod, all-trans retinoic acid or hyaluronidase; and
R₂ is independently triptolide, paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, or Lapatinib.

The compound according to the embodiments of the present disclosure can be enzymatically degraded in vivo to produce medicines of the tumor stroma-regulating group and the cytotoxic group. Thus, the compound can act on the tumor stroma and tumor cells at the same time, thereby achieving the purpose of eliminating or reducing the tumor stroma while killing the tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

According to the embodiments of the present disclosure, the compound has one of the following structures:

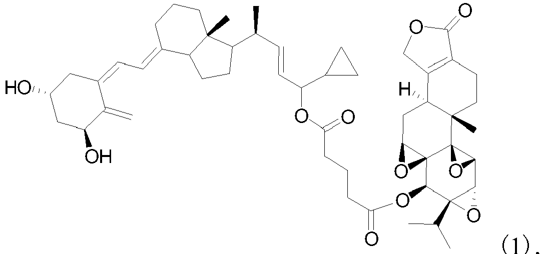

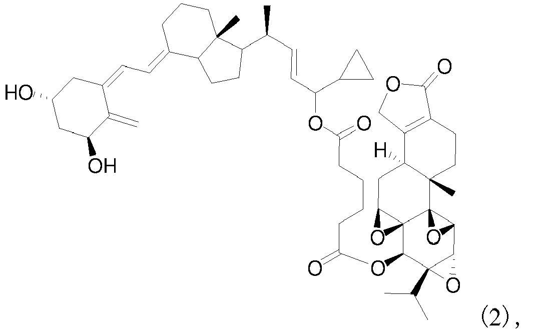

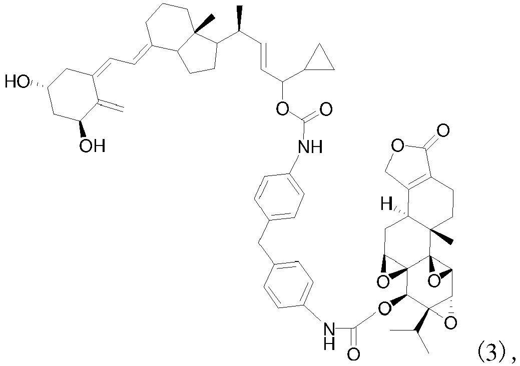

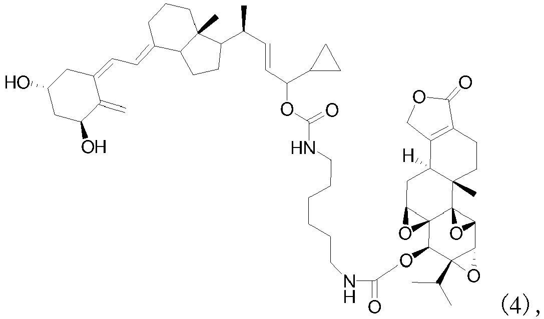

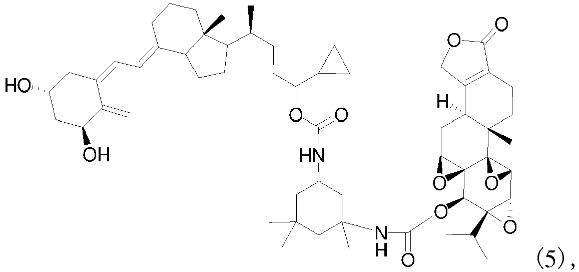

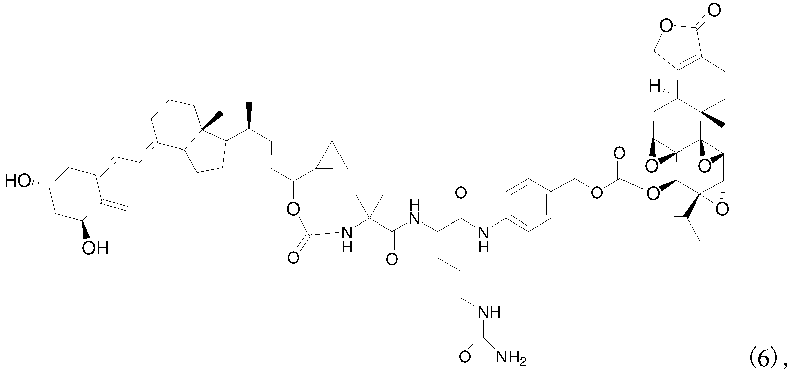

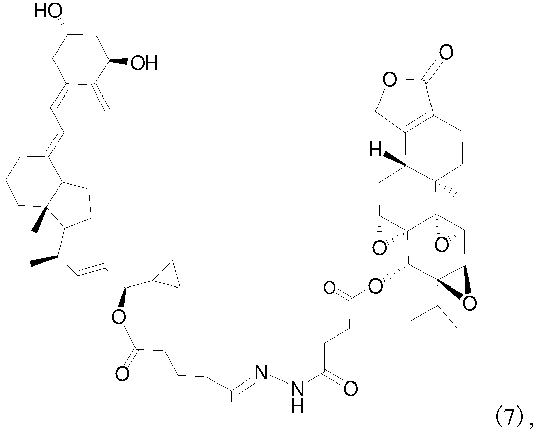

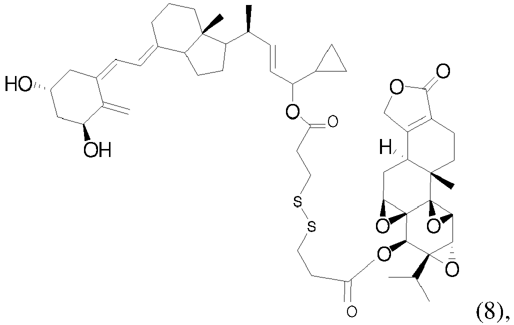

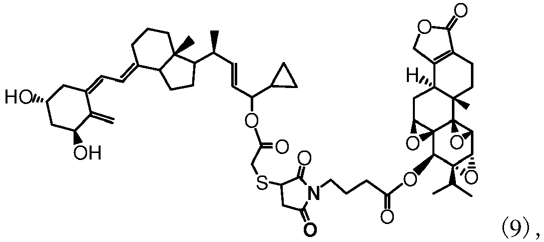

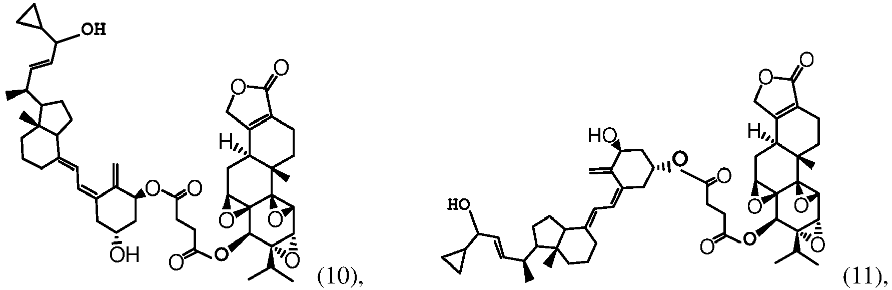

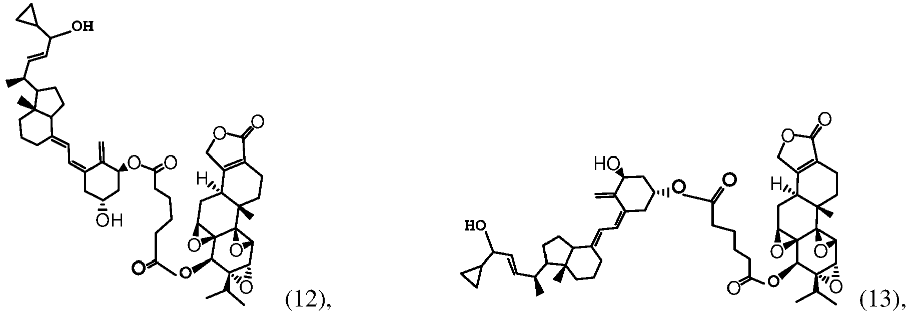

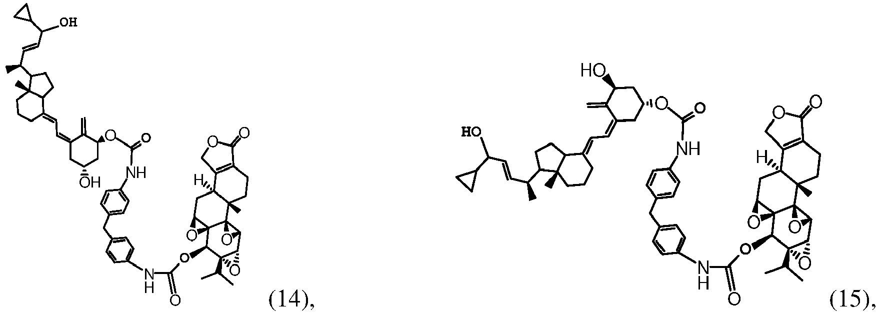

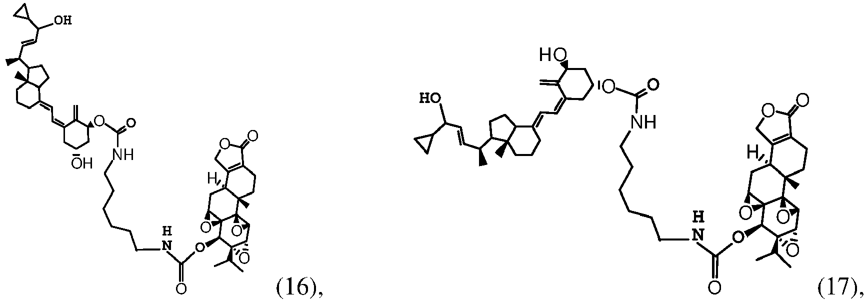

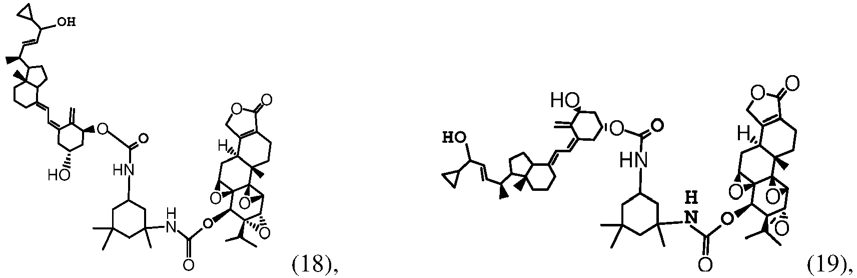

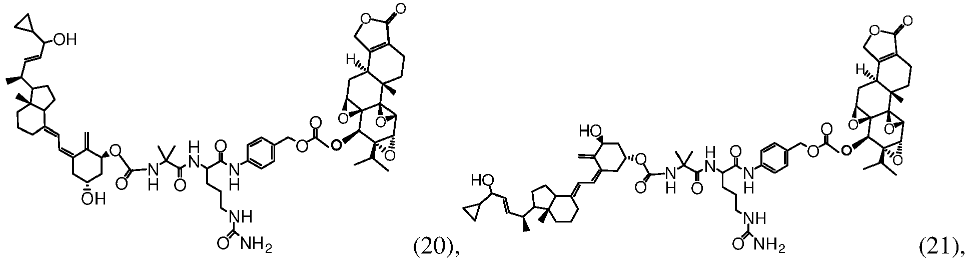

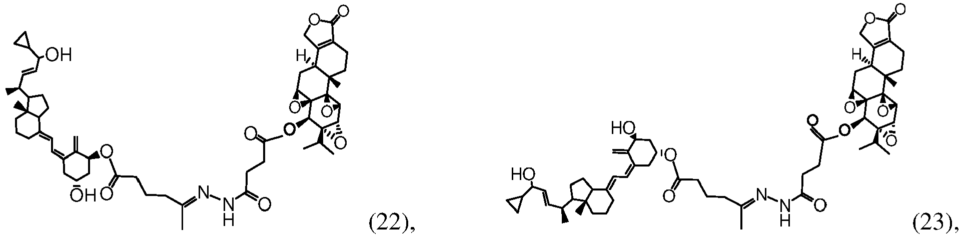

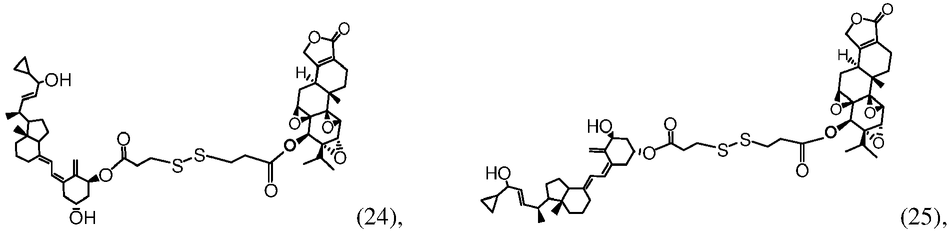

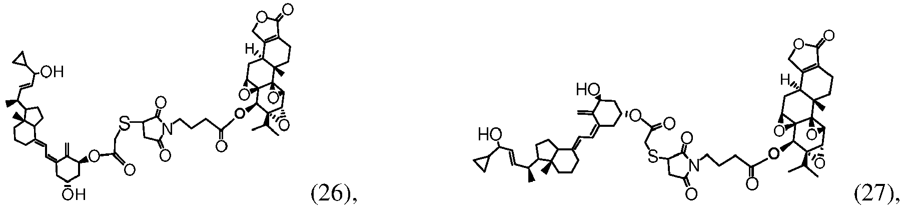

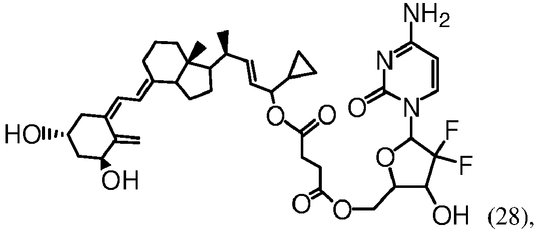

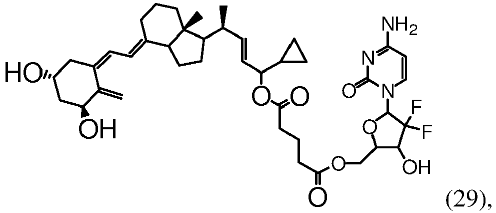

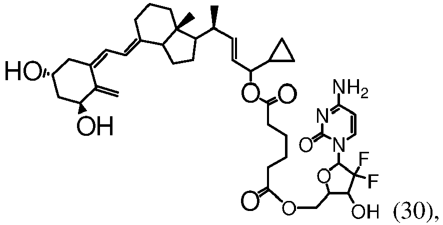

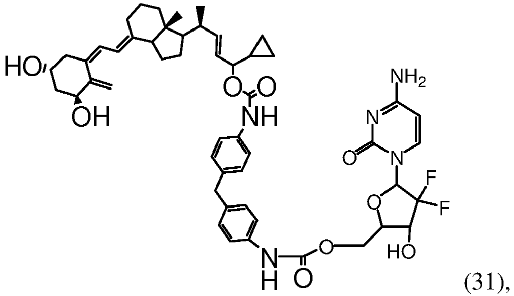

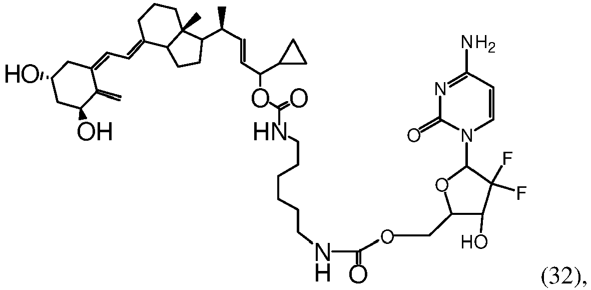

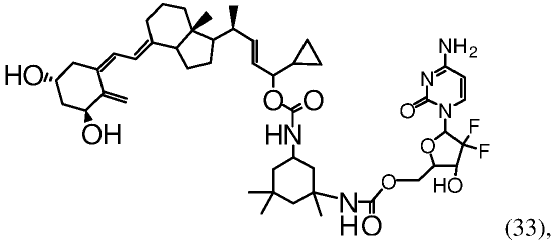

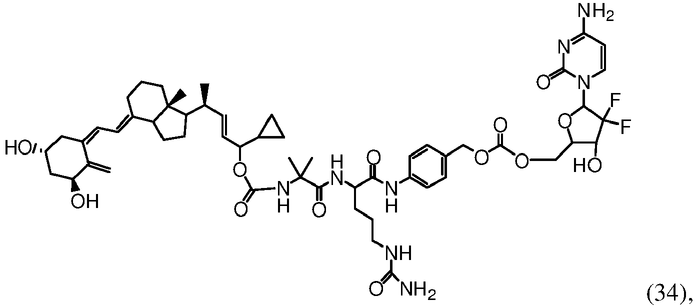

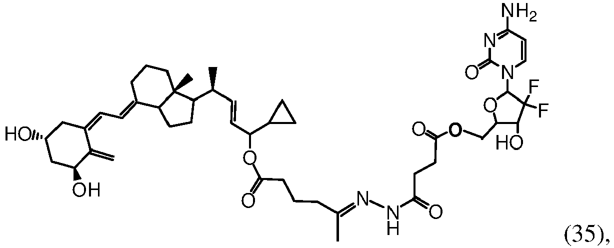

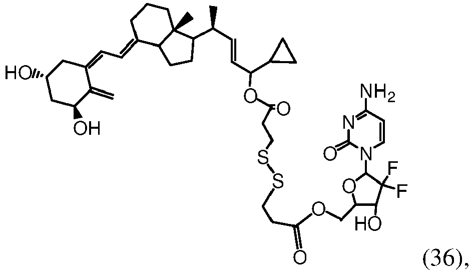

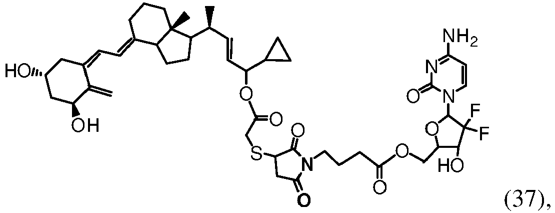

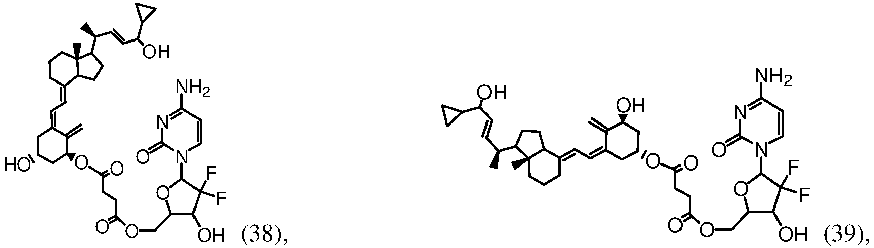

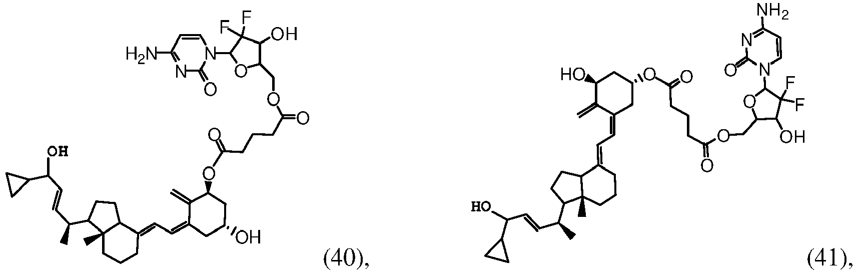

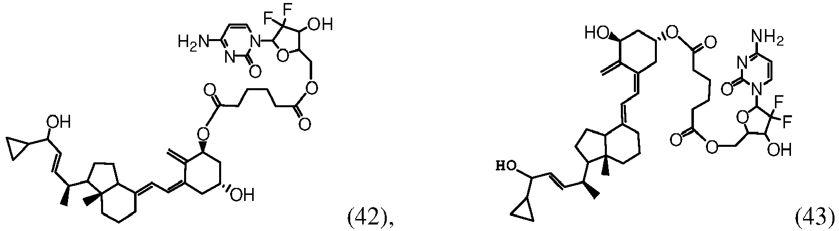

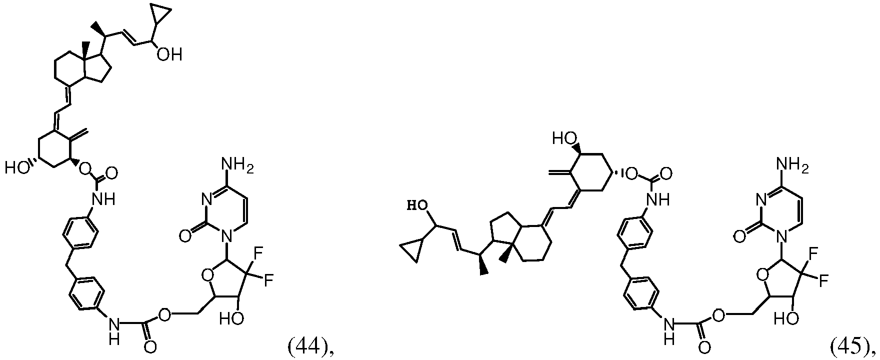

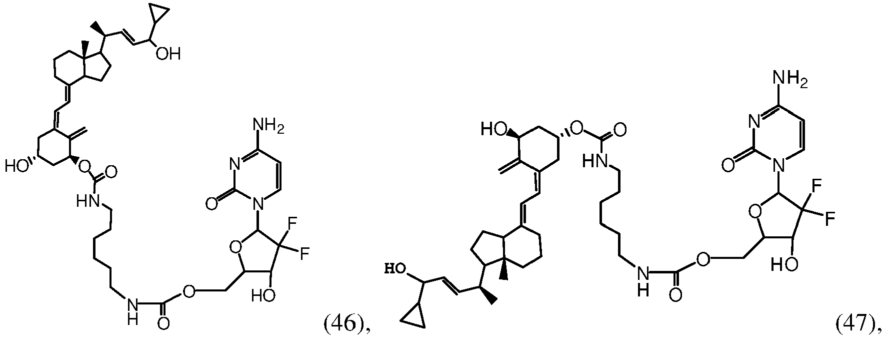

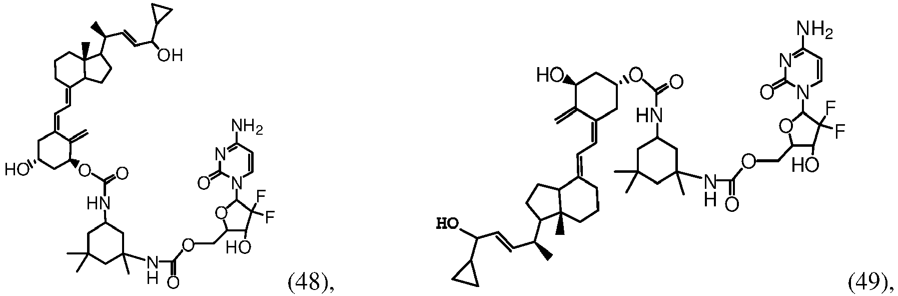

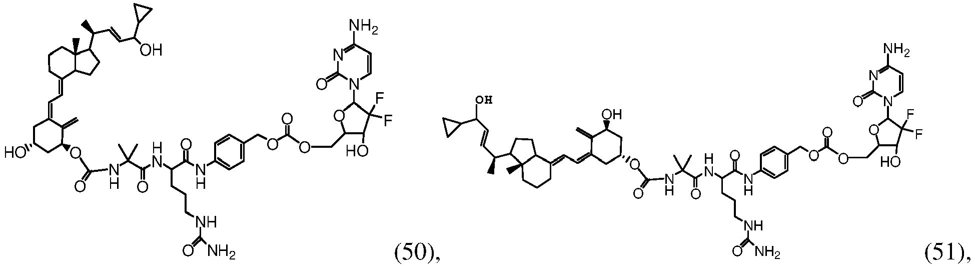

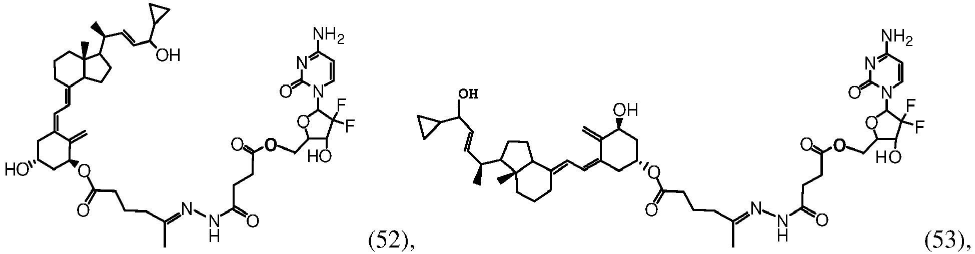

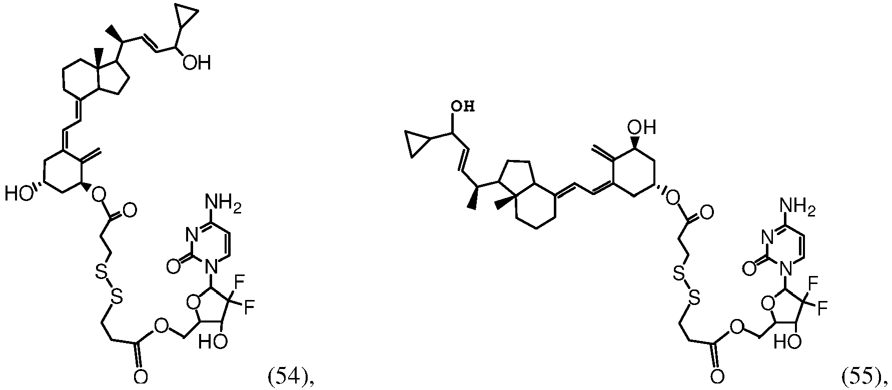

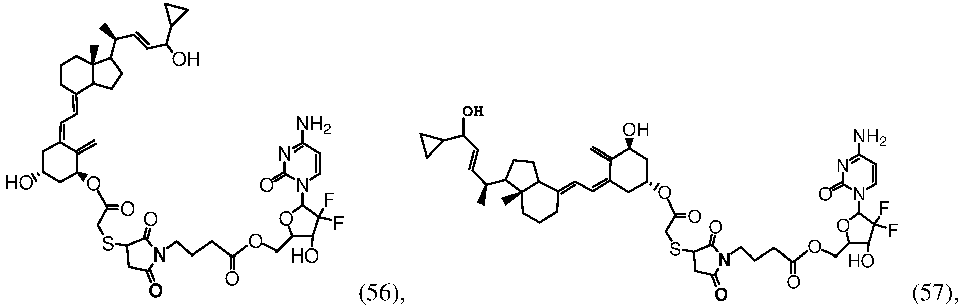

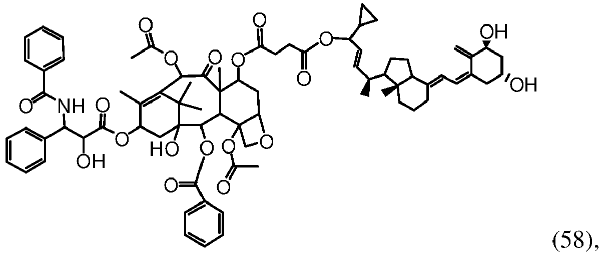

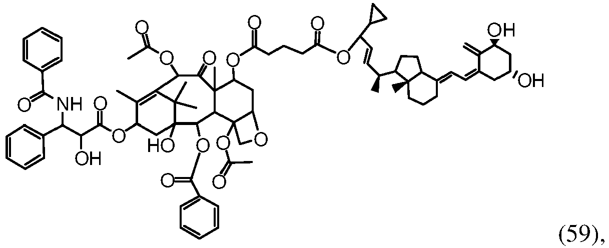

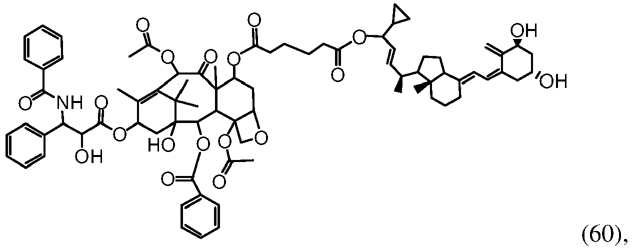

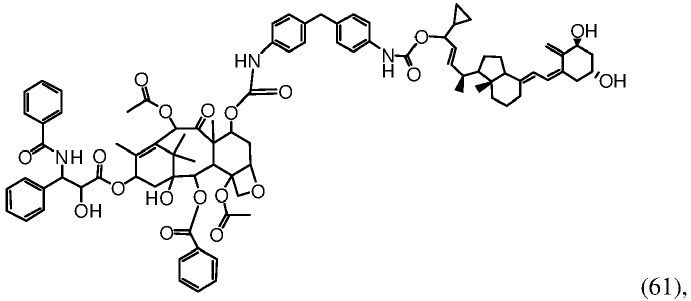

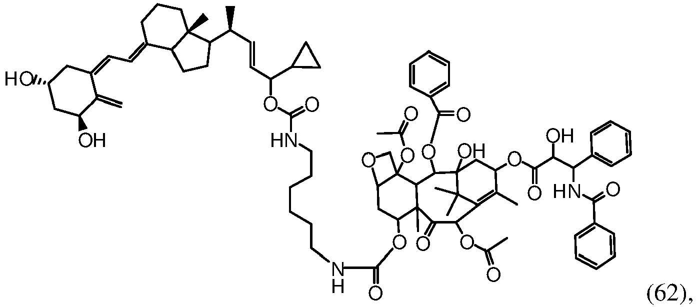

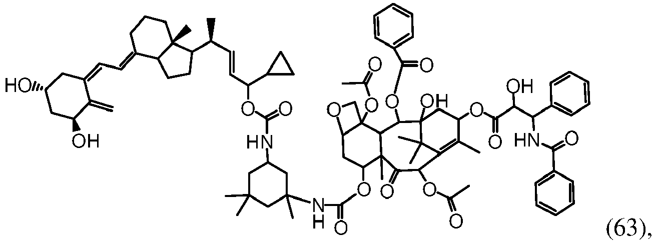

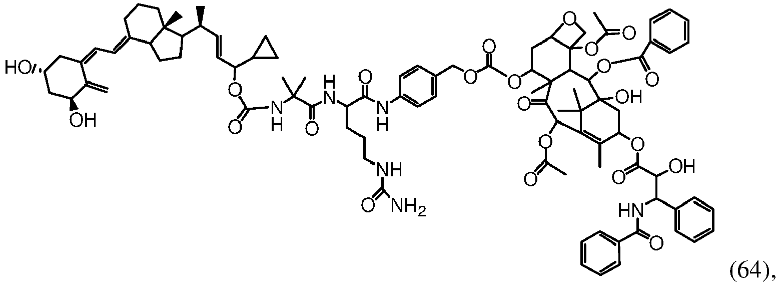

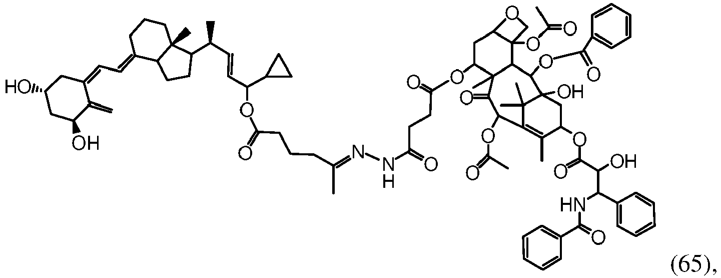

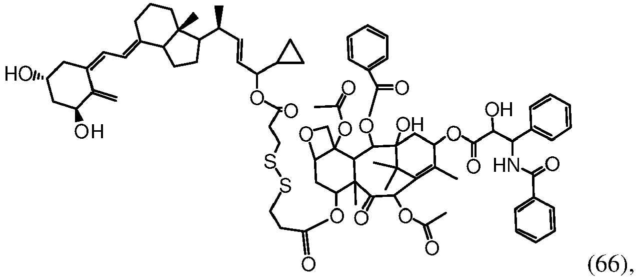

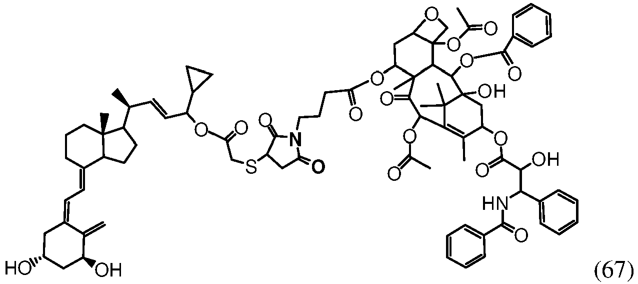

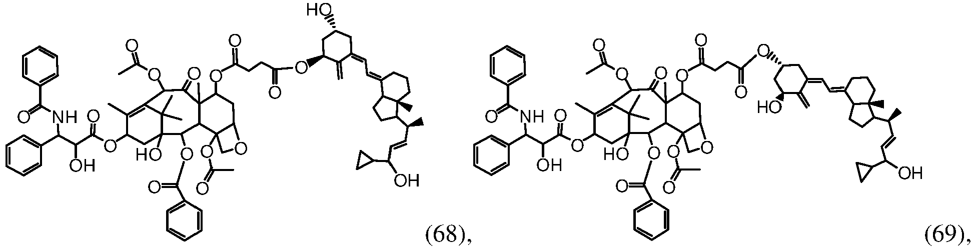

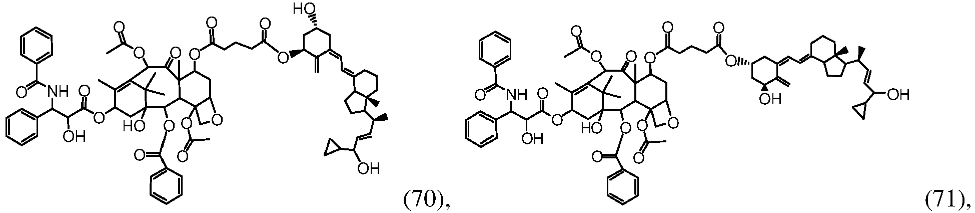

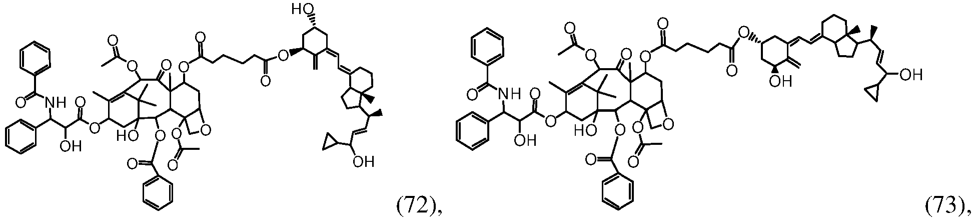

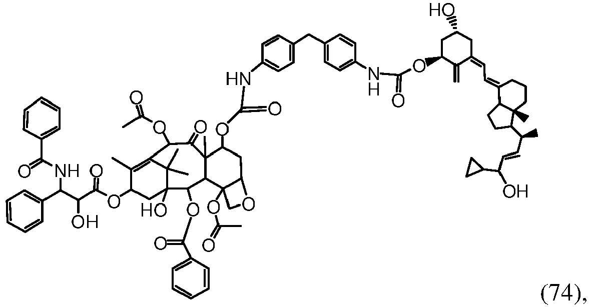

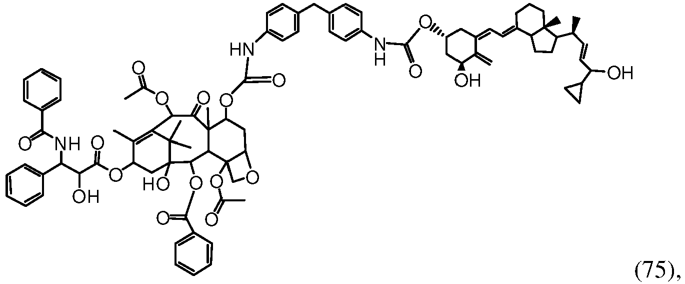

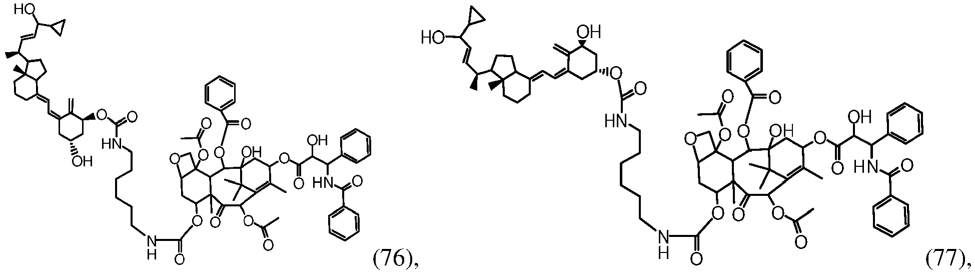

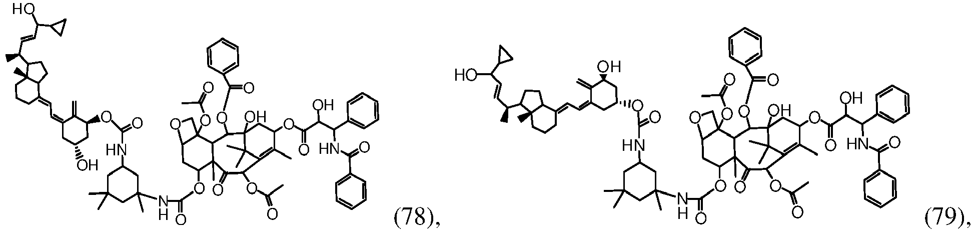

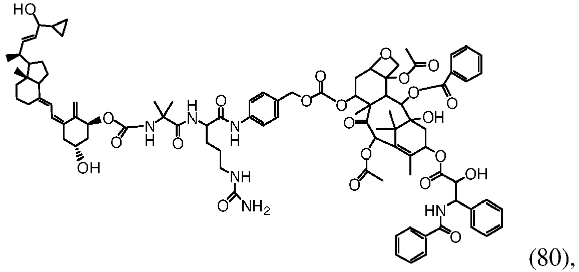

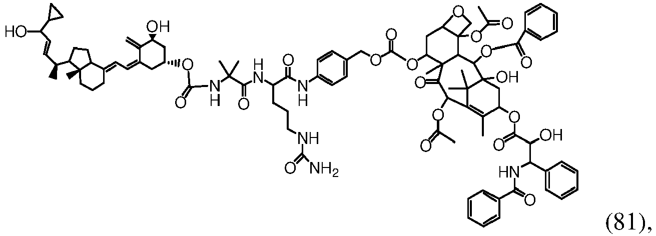

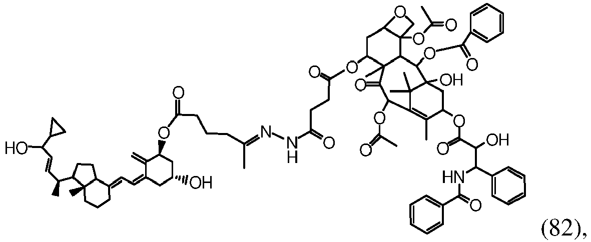

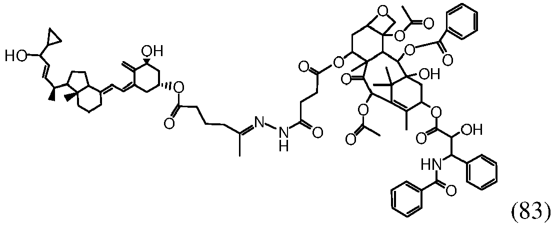

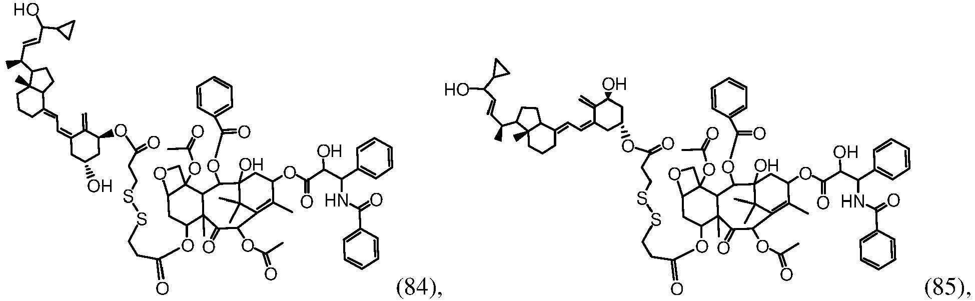

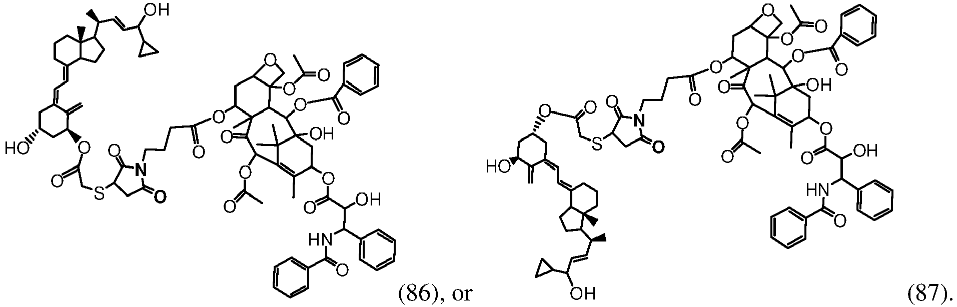

The compound according to the embodiments of the present disclosure includes the tumor stroma-regulating group and the cytotoxic group that are coupled to each other. The tumor stroma-regulating group is used to regulate the tumor stroma, and the cytotoxic group is used to kill the tumor cells. The compound according to the embodiments of the present disclosure can act on the tumor stroma and the tumor cells at the same time, so as to achieve the purpose of eliminating or reducing the tumor stroma while killing tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

In a third aspect of the present disclosure, the present disclosure provides a compound. According to the embodiments of the present disclosure, the compound is a compound represented by formula (I), or the compound is an isomer, a stereoisomer, a geometric isomer, a tautomer, a nitrogen oxide, a hydrate, a solvate, a metabolite, a pharmaceutically acceptable salt or a prodrug of the compound represented by formula (I):

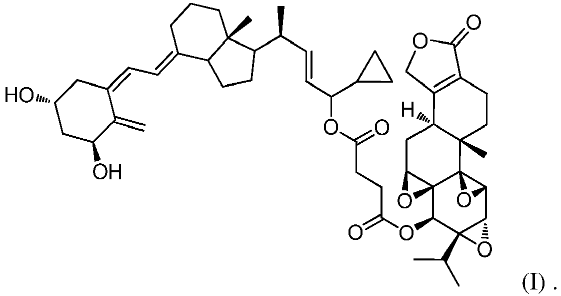

The compound according to the embodiment of the present disclosure: 1) effectively controls the pancreatic stellate cells and pancreatic cancer cells in the pancreatic cancer tissue simultaneously; 2) Callide's pharmacokinetic properties are significantly optimized, and its toxicity is significantly reduced, when compared with TP; and 3) in a pancreatic cancer transgenic mouse model with high clinical relevance, the compound significantly prolonged the median survival time of tumor animals compared with the existing first-line drugs for treating pancreatic cancer, such as Gemcitabine.

According to the embodiments of the present disclosure, the isomer of the compound represented by formula (I) has a structure represented by formula (II) or formula (III),

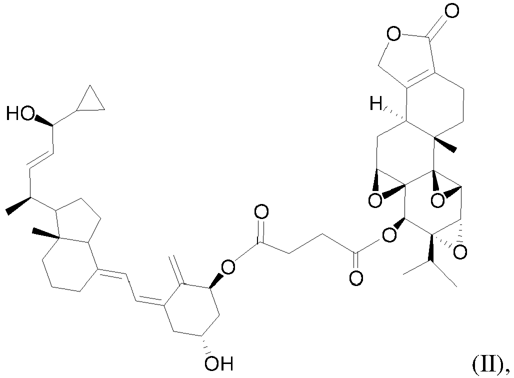

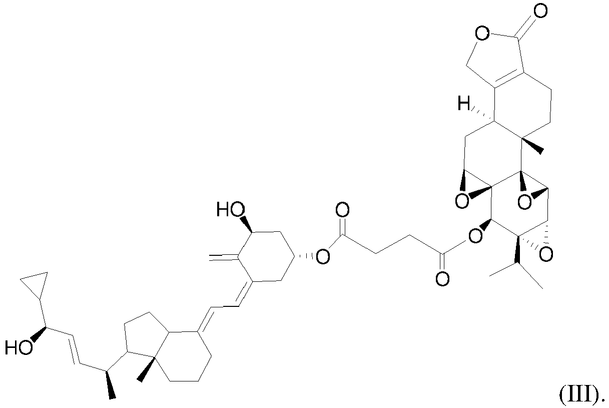

The compounds represented by the above formula (II) and formula (III) according to the embodiments of the present disclosure have similar properties as the compound represented by formula (I). That is, they can effectively control the pancreatic stellate cells and pancreatic cancer cells in the pancreatic cancer tissue at the same time. Compared with TP, the compounds represented by formula (II) and formula (III) have significantly optimized pharmacokinetic properties, and significantly reduced toxicity; and in a pancreatic cancer transgenic mouse model with high clinical relevance, a median survival time of tumor animals is significantly prolonged, when compared with the existing first-line drugs for treating pancreatic cancer such as Gemcitabine.

In a fourth aspect of the present disclosure, the present disclosure provides a pharmaceutical composition. According to the embodiments of the present disclosure, the pharmaceutical composition includes, as an active ingredient, the above-described compound. The pharmaceutical composition according to the embodiments of the present disclosure can effectively control the formation of stroma in cancer tissues and achieve more effective treatment of tumors. The pharmaceutical composition according to the embodiments of the present disclosure has a longer half-life and therapeutic window as well as significantly reduced therapeutic toxicity compared to the existing drugs for cancer treatment.

According to the embodiments of the present disclosure, the above-mentioned pharmaceutical composition can further have at least one of the following additional technical features:

According to the embodiments of the present disclosure, the pharmaceutical composition further includes pharmaceutically acceptable excipients.

According to the embodiments of the present disclosure, the pharmaceutical composition is formulated in a form of micelles, emulsion, albumin nanoparticles, liposomes, capsules, pills, tablets, granules, oral liquid, oral ointment, aerosol, or spray, allowing to be administrated easily.

According to the embodiments of the present disclosure, the pharmaceutical composition is formulated in the form of micelles, and the micelles are formed by at least one of copolymer including polyethylene glycol-polylactic acid blocks, monomethoxy polyethylene glycol polylactic acid, monomethoxy polyethylene-glycol polylactic acid-glycolic acid copolymer, monomethoxy polyethylene glycol polycaprolactone, monomethoxy polyethylene glycol polytrimethylene carbonate, and monomethoxy polyethylene glycol polyamino acid. In this way, the active ingredient can be protected from being affected by a phagocytosis of the human reticuloendothelial system and other human internal environment, and thus it can be slowly released at the lesion site, while a drug loading and bioavailability of the active ingredient can be effectively improved, so as to exerts an efficacy of treating or preventing cancer.

According to the embodiments of the present disclosure, the pharmaceutical composition further includes an additional anti-pancreatic cancer medicine, and the additional anti-pancreatic cancer medicine includes one or more of 5-fluorouracil, Gemcitabine, FOLFIRINOX, nano-paclitaxel/Gemcitabine combination, and ONIVYDE™. By combining with the additional medicine for treating pancreatic cancer, the pharmaceutical composition has a more significant therapeutic effect on pancreatic cancer.

In a fifth aspect of the present disclosure, the present disclosure provides a use of the compound as described above or the pharmaceutical composition as described above in a preparation of a medicine for treating or preventing cancer. The active ingredient in the coupling compound or the pharmaceutical composition according to the embodiments of the present disclosure can be effectively *in vivo* degraded into the tumor stroma-regulating group and the cytotoxic group drugs, the tumor stroma-regulating group can effectively inhibit the growth of tumor stroma, and the cytotoxic group exerts a cytotoxic effect to kill the tumor cells effectively. Therefore, the compound or pharmaceutical composition according to the embodiments of the present disclosure can be used to effectively treat or prevent cancer.

According to the embodiments of the present disclosure, the above use can further have at least one of the following additional technical features:

According to the embodiments of the present disclosure, the cancer is pancreatic cancer, liver cancer, breast cancer, skin cancer, prostate cancer, or fibroblastoma. Applicant found that the above-mentioned tumors have an obvious stromal microenvironment, and the compound or the pharmaceutical composition according to the embodiments of the present disclosure has a better therapeutic effect on the above-mentioned cancer.

According to the embodiments of the present disclosure, the cancer is pancreatic cancer. The compound or the pharmaceutical composition according to the embodiments of the present disclosure has more significant therapeutic effect on the pancreatic cancer.

In a sixth aspect of the present disclosure, the present disclosure provides a method for preparing the compound as described above. According to the embodiments of the present disclosure, the method includes, subjecting a first coupling component and a second coupling component to a ligation reaction. The first coupling component is configured to regulate tumor stroma, and the second coupling component is configured to kill tumor cells. The compound obtained by the above method according to the embodiments of the present disclosure can act on the tumor stroma and the tumor cells at the same time, so as to achieve the purpose of eliminating or reducing the tumor stroma while killing tumor cells. The compound obtained by the above method according to the embodiments of the present disclosure has the improved pharmacokinetic properties, synchronizing the pharmacokinetics of the tumor stroma-regulating group and the cytotoxic group, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

According to the embodiments of the present disclosure, the above-mentioned method can further have at least one of the following additional technical features.

According to the embodiments of the present disclosure, the ligation reaction is performed under a condition that the first coupling component, the second coupling component, and a linker coexist, and the linker is at least one of compounds represented by formula (88) to formula (97),

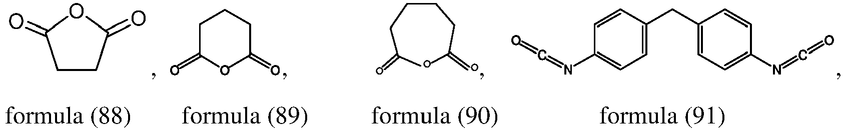

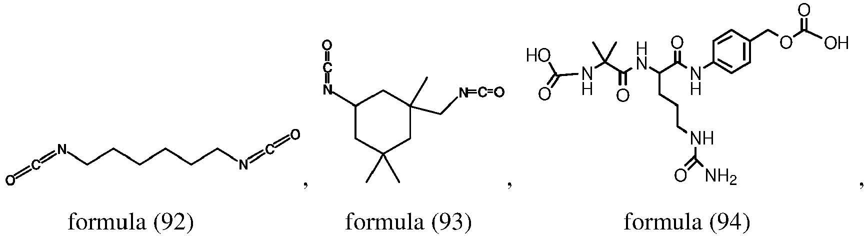

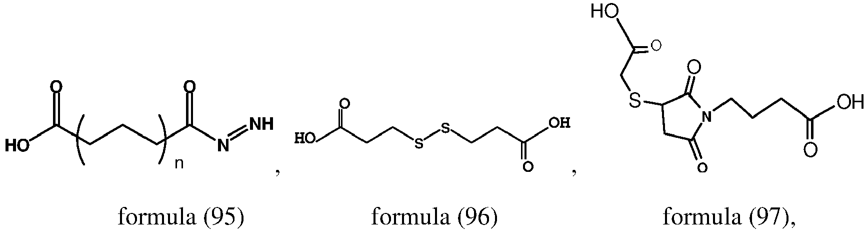

where, n is 1 to 10. The compounds represented by formula (88) to formula (97) can be used as effective linkers to chemically couple the first coupling component with the second coupling component to form the above-mentioned stable compound.

According to the embodiments of the present disclosure, the first coupling componentis Cal, the second coupling component is TP, and the liner is the compound represented by formula (88). In this way, the ligation reaction has a high efficiency.

According to the embodiments of the present disclosure, a molar ratio of the first coupling component to the second coupling component is 1: 1. In this way, the second coupling component has a significant effect of inhibiting tumor cells, and the first coupling component has a significant effect of inhibiting the formation of cancer stroma, such that the synergistic therapeutic effect of these two is significant.

### BRIEF DESCRIPTION OF DRAWINGS

FIG. 1 illustrates a synthesis scheme of a compound represented by formula (I) (referred to as Callide herein, i.e., Compound 5 in the synthesis scheme) according to an embodiment of the present disclosure, and a preparation of a Callide-loading micelles (referred to as Callide^{NP} herein);
FIG. 2 illustrates graphs of survival rates of cells after being treated with TP, Cal, TP/Cal, and Callide^{NP} in presence or absence of porcine liver esterase (PLE) according to an embodiment of the present disclosure;
FIG. 3 illustrates plasma drug-time curves and pharmacokinetic parameters in rats after an administration of medicine according to an embodiment of the present disclosure;
FIG. 4 illustrates graphs showing changes in body weight (top) and testicular index (bottom) of mice after an administration of medicine according to an embodiment of the present disclosure;
FIG. 5 illustrates diagrams showing expression of smooth actin and collagen in murine pancreatic stellate cells and pancreatic cancer cells in vitro after an administration of medicine according to an embodiment of the present disclosure;
FIG. 6 illustrates diagrams indicating an effect of tumor suppression and prolonged survival rate of mice in an orthotopic model of co-implantation of murine pancreatic stellate cells and pancreatic cancer cells in vivo after an administration of medicine according to an embodiment of the present disclosure;
FIG. 7 illustrates that, in a CDX model, TP according to an embodiment of the present disclosure inhibits tumor growth more effectively than Callide^{NP};
FIG. 8 illustrates that Callide^{NP} according to an embodiment of the present disclosure effectively reduces a growth of pancreatic tumors in a human xenotransplantation model; and
FIG. 9 illustrates photographs of a nude mouse before and after an administration of a medicine according to an embodiment of the present disclosure.

### DESCRIPTION OF EMBODIMENTS

Embodiments of the present disclosure are described in detail below. The embodiments described below are illustrative and merely for the purpose of explaining the present disclosure, and they cannot be construed as limitations of the present disclosure. Technologies or conditions, if not specifically indicated in the embodiments, are those described in the literatures in the related art or the product instruction. Reagents or instruments used are all conventional products that are commercially available, unless a manufacturer is specifically indicated.

Some embodiments of the present disclosure will now be described in detail, examples of which are illustrated by accompanying structural and chemical formulas. The present disclosure is intended to cover all alternatives, modifications and equivalent technical solutions, which are included in the scope of the present disclosure as defined by the claims. Those skilled in the art shall recognize that various methods and materials similar or equivalent to those described herein can be used in the practice of the present disclosure. The present disclosure is by no means limited to the methods and materials described herein. If one or more of the incorporated literatures, patents, and similar materials are different from or contradictory to the present disclosure (including but not limited to definitions of terms, terminology, described technology, etc.), the present application shall prevail.

It should be further recognized that, for purpose of clarity, some features of the present disclosure are described in multiple independent embodiments, but they may also be provided in combination in a single embodiment. On the contrary, for the sake of brevity, various features of the present disclosure are described in a single embodiment, but they can also be provided individually or in any suitable sub-combination.

Unless otherwise indicated, all technical and scientific terms used in the present disclosure have the same meaning as commonly understood by those skilled in the art to which the present disclosure belongs. All patents and publications related to the present disclosure are incorporated by reference in their entirety.

Unless otherwise indicated, the following definitions used in the present disclosure shall be applied. For the purposes of the present disclosure, chemical elements are consistent with the periodic table of elements of CAS version, and the "Handbook of Chemistry and Physics", 75th edition, 1994. In addition, the general principles of organic chemistry shall refer to the descriptions in "Organic Chemistry", Thomas Sorrell, University, Science Books, Sausalito: 1999, and "March's Advanced Organic Chemistry" by Michael B. Smith and Jerry March, John Wiley & Sons, New York: 2007, the entire contents of which are incorporated into the present disclosure by reference.

Unless otherwise indicated or there is a clear conflict in the context, the articles "a", "an" and "said" as used herein are intended to include "at least one" or "one or more". Therefore, the articles used herein refer to one or more than one (i.e., at least one) object articles. For example, "a component" refers to one or more components, that is, more than one component is considered to be employed or used in the implementation of the embodiments.

### Compound

In a first aspect, the present disclosure provides a compound. According to the embodiments of the present disclosure, the compound includes a tumor stroma-regulating group and a cytotoxic group that are coupled to each other. The tumor stroma-regulating group is used to regulate the tumor stroma, and the cytotoxic group is used to kill the tumor cells. The compound according to the embodiments of the present disclosure can act on the tumor stroma and the tumor cells at the same time, so as to achieve the purpose of eliminating or reducing the tumor stroma while killing tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

According to the embodiments of the present disclosure, the tumor stroma-regulating group includes at least one selected from calcipotriol (Cal), cyclopamine, Ganciclovir (GCV), Fingolimod, all-trans retinoic acid (ATRA), and hyaluronidase (HA). Among them, calcipotriol (Cal) is a VDR ligand, which is the main regulator of PSCs and participates in regulating PSCs to return the dormant state, so as to induce stroma content. Cyclopamine can bind to smoothened (Smo) protein in the Hedgehog signaling pathway, thereby inhibiting the activity of the protein and reducing the formation of the stroma. Ganciclovir, by competitively inhibiting a binding of the trivalent phosphate of deoxyguanosine and DNA polymerase, can inhibit a number of the excessively proliferated and α-SMA positive fibroblasts in the tumor stroma, so as to reduce the content of stroma content. Fingolimod, as a sphingosine phosphate receptor modulator, inhibits the activation of pancreatic stellate cells by inhibiting AMPK/mTOR pathway, inhibiting autophagy and promoting apoptosis. All-trans-retinoic acid is an analogue of vitamin A and has the same mechanism of action as calcipotriol, and it can act on the vitamin receptors of fibroblasts produced by tumors, so as to so the content of stroma. Hyaluronidase can effectively degrade the important component of the tumor stroma, hyaluronic acid, thereby directly reducing the density and content of the stroma.

According to the embodiments of the present disclosure, the cytotoxic group includes at least one selected from triptolide (TP), paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, and Lapatinib. Among them, triptolide (TP) is a potent inhibitor of TFIIAH, FIIAH is a universal transcription factor, which is responsible for recruiting DNAs to be transcribed to a promoter for transcription and unwinding the DNA. In addition, if the DNA of cells is damaged, TFIIAH is also used to unwind the helix DNA during gene repair. The excessive proliferation of cancer cells is accompanied by the transcription of DNA and the expression of the related mRNA, and TFIIAH is therefore more highly expressed than normal tissues, allowing it to be a target of chemotherapy drugs for treating cancer. TP is an effective medicine for the treatment of pancreatic cancer. Paclitaxel and docetaxel can be used to inhibit a synthesis of tumor cell tubulin, thereby inhibiting the proliferation of tumor cells and achieving tumor suppression, and with an increase of drug concentration, the cell survival rate in vitro gradually decreases. Adriamycin can inhibit the synthesis of RNAs and DNAs, and it has the strongest inhibitory effect on RNAs and a broad anti-tumor spectrum, so as to have an effect on a variety of tumors, and adriamycin belongs to the cycle non-specific drugs, having a killing effect on tumor cells of various growth cycles; through the analysis of cell proliferation experiments, a cell death rate is related to the drug concentration. Camptothecin and hydroxycamptothecin are cytotoxic quinoline alkaloids that can inhibit DNA topoisomerase (TOPO I), and they can combine with a complex formed by Topo I-DNA and stabilize the complex, such that the broken DNA chains cannot be rejoined, thereby preventing DNA replication and RNA synthesis. Camptothecin and hydroxycamptothecin are cell cycle S-phase specific drugs, and they have no effect on the cells in GO phase and a slight lethality on the cells in G1, G2 and M phases. In addition, it can also directly destroy the DNA structure, so as to limit the proliferation of cancer cells to a specific cell cycle, thereby inhibiting the tumor cell growth. Irinotecan is a semi-synthetic derivative of camptothecin. Camptothecin can specifically bind to topoisomerase I, which induces a reversible single-strand break, thereby unwinding the double-stranded DNA structure and promoting the death of cancer cells. 5-fluorouracil is a fluorouracil, i.e., an analogue of uracil, which can be converted into effective fluorouracil deoxynucleotides in the cells and interferes with DNA synthesis by blocking the conversion of deoxyribonucleic acid to thymidine by intracellular thymidylate synthase. Gemcitabine is a difluoronucleoside antimetabolite-anticancer drug, a water-soluble analog of deoxycytidine, and an inhibitory enzyme substitute for ribonucleotide reductase, which can be used to block DNA synthesis during tumor cell DNA replication and inhibit tumor cell growth. Cisplatin can bind to DNA and causes cross-linking, thereby destroying the function of DNA and inhibiting cell mitosis, and it is a cell non-specific drug that inhibits the proliferation of cancer cells. Oxaliplatin, by producing hydrated derivatives, can act on DNA, so as to form intra- and inter-chain cross-links, thereby inhibiting DNA synthesis and producing cytotoxicity and anti-tumor activity. Pemetrexed is an anti-folate preparation having a structure containing a pyrrolopyrimidine group, and it can inhibit the cell replication and the tumor growth by destroying the folate-dependent normal metabolic process in the cells. Capecitabine can be in vivo converted into antimetabolic fluoropyrimidine deoxynucleoside carbamates of 5-FU, which can inhibit cell division and interfere with the synthesis of RNA and protein, thereby inhibiting cancer cell proliferation. Epirubicin is an isomer of adriamycin and has the mechanism of action that it is directly embedded between DNA nucleobase pairs to interfere with transcription process and prevent the formation of mRNA, thereby inhibiting the synthesis of DNA and RNA.

The small molecule targeted drugs and their targets are as follows: Sorafenib (PDGF/VEGF), Gefitinib (EGFR), Erlotinib (EGFR/HER2), Imatinib (Bcr-Abl), Nilotini (Bcr-Abl), Dasatinib (Bcr-Abl), Everolimus (Bcr-Abl), Sunitinib (PDGF/VEGF), Ibrutinib (BTK), Crizotinib (ALK), Pazopanib (PDGF/VEGF), Carfilzomib (26S proteasom), Tofacitinib (JAK), Axitini (VEGFR), Regorafenib (multikinase inhibitor), Verofenib (B-Raf), Sirolimus (mTOR), Ponatinib (Bcr-Abl), Levatinib (RTK), Olaparib (PARP), Ceritinib (ALK), Romidepsin (HDAC), Alectinib (ALK), Belinostat (HDAC), Bosutinib (Src/Abl), Vandetanib (multikinase inhibitor), Cabozantinib (C-Met), Afatinib (EGFR), Trametinib (MEK1/2), Dabrafenib (BRAF), or Lapatinib (EGFR/erb2). Based on the above small molecule targeted drugs and their respective targets, these drugs can specifically bind to the cancer cell surface or related targets, allowing them to specifically kill the cancer cells and greatly reduce the toxic and side effects on normal cells.

The coupling compound of the present disclosure is based upon such a design concept: in order to simultaneously act on tumor stroma and tumor cells, Applicant has designed and synthesized a variety of different coupling drug molecules. After using different degradable linkers, the drugs can be degraded in vivo to different extents, a part of the coupling drug molecule has the inhibitory effect on the tumor stroma, and the other part kills the tumor cells. In addition, through the design of different preparations and treatment schemes, a balance between tumor stroma suppression and cancer cell killing has been achieved, thereby achieving the purpose of synergistic effect.

According to the embodiments of the present disclosure, the compound further includes an enzyme-degradable linker. In this way, the compound can be enzymatically degraded in vivo to produce medicines of the tumor stroma-regulating group and the cytotoxic group, which better synchronizes the pharmacokinetics of the medicines of the tumor stroma-regulating group and cytotoxic group, thereby achieving the synergistic effect of promoting PSC dormancy and inhibiting tumor cells.

According to the embodiments of the present disclosure, the enzyme-degradable linker has one of the following structures:

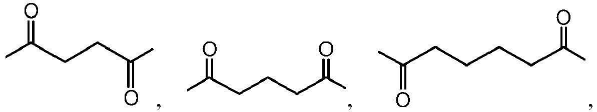

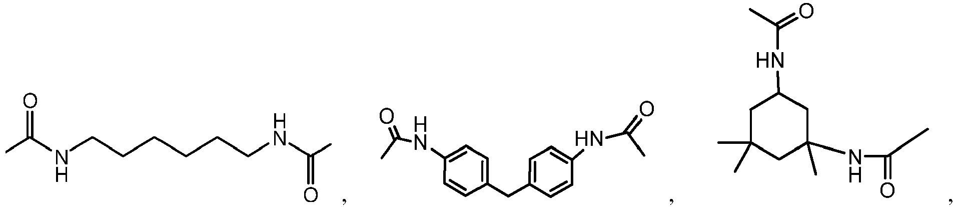

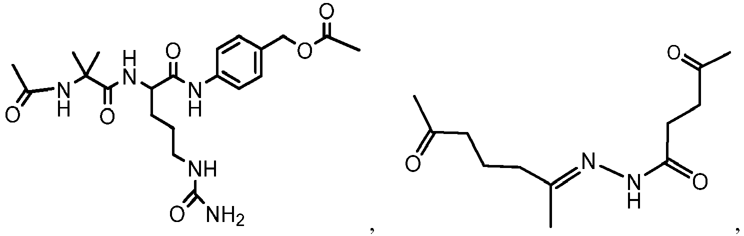

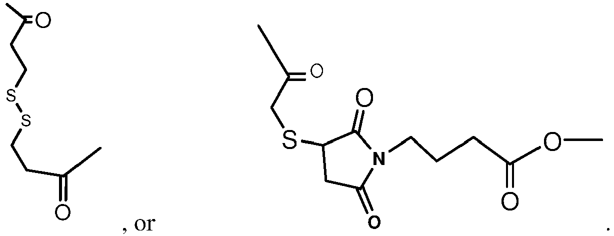

In another aspect of the present disclosure, the present disclosure provides a compound. According to the embodiments of the present disclosure, the compound includes one of the following structures, or includes one of isomers, stereoisomers, geometric isomers, tautomers, nitrogen oxides, hydrates, solvates, metabolites, pharmaceutically acceptable salts or prodrugs of the following structures: 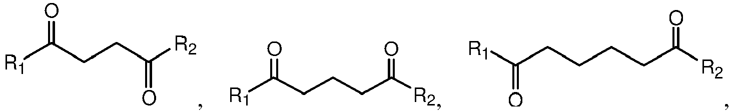 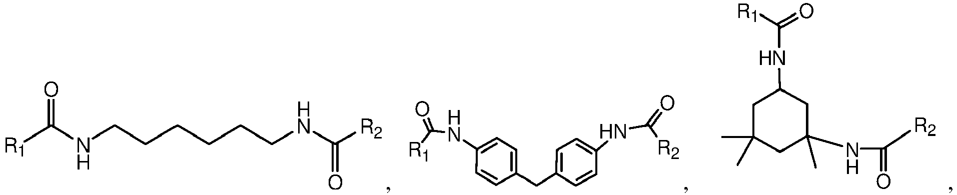 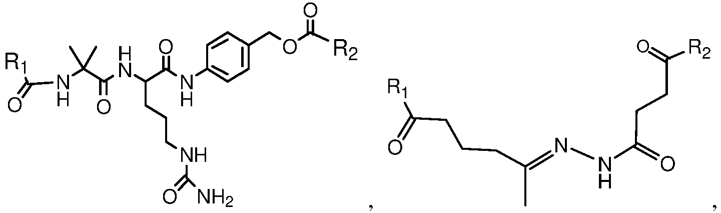 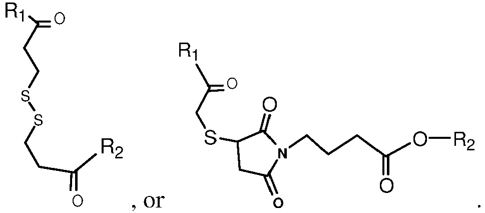
where R₁ is independently calcipotriol, cyclopamine, Ganciclovir, Fingolimod, all-trans retinoic acid or hyaluronidase; and
R2 is independently triptolide, paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, or Lapatinib.

The compound according to the embodiments of the present disclosure can be enzymatically degraded in vivo to produce medicines of the tumor stroma-regulating group and the cytotoxic group. Thus, the compound can act on the tumor stroma and tumor cells at the same time, thereby achieving the purpose of eliminating or reducing the tumor stroma while killing the tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

According to yet another embodiment of the present disclosure, the compound has one of the following structures:

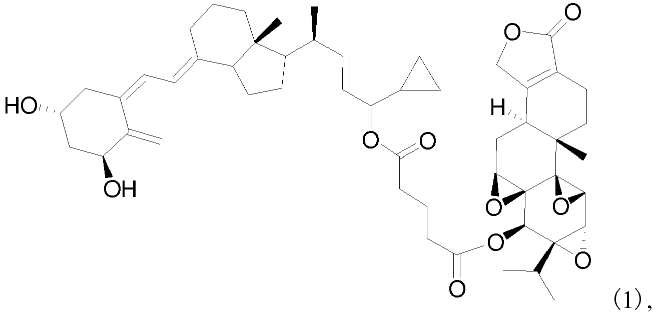

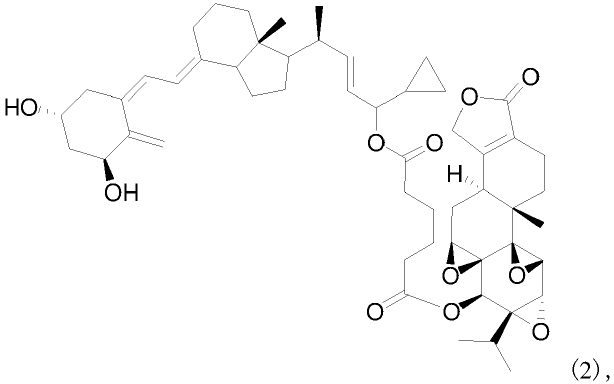

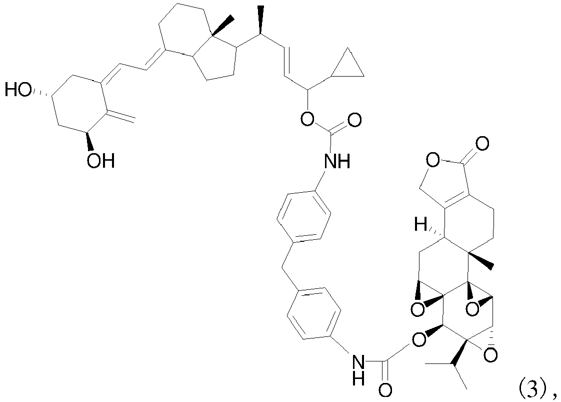

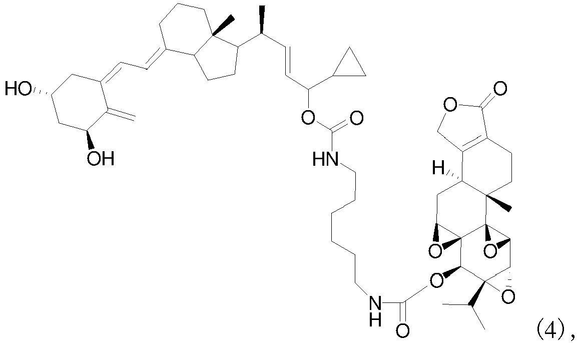

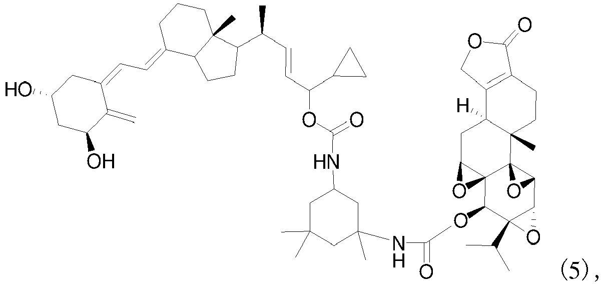

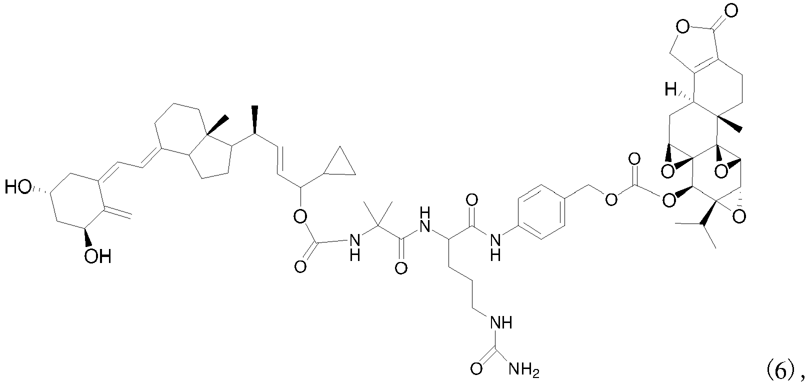

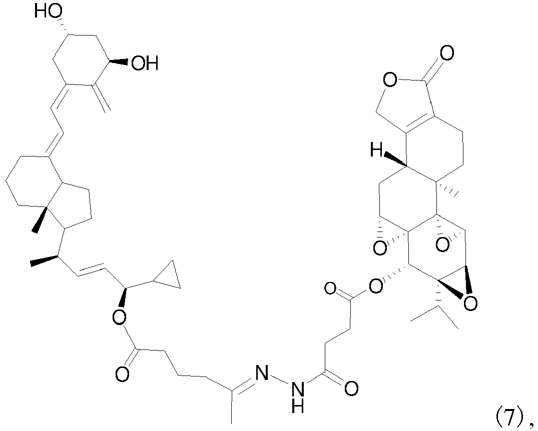

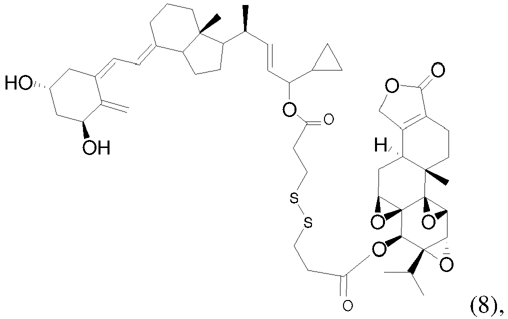

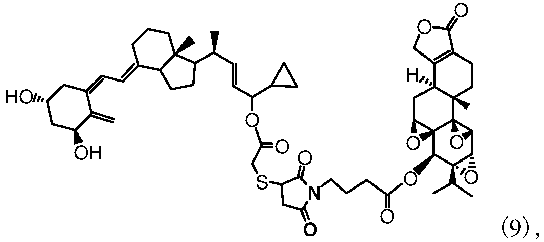

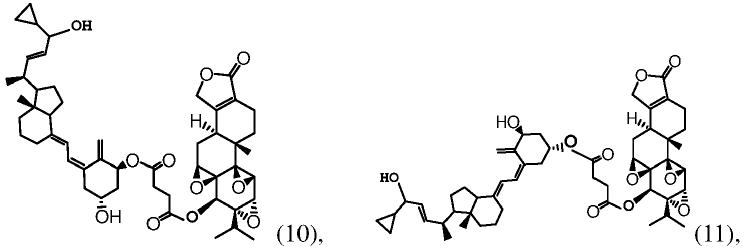

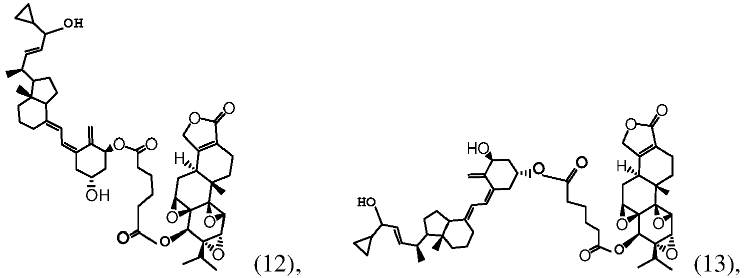

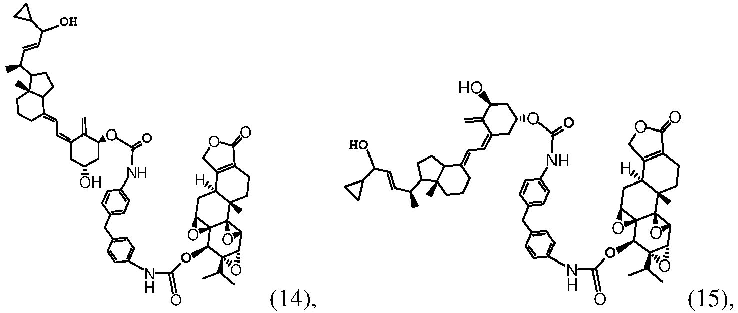

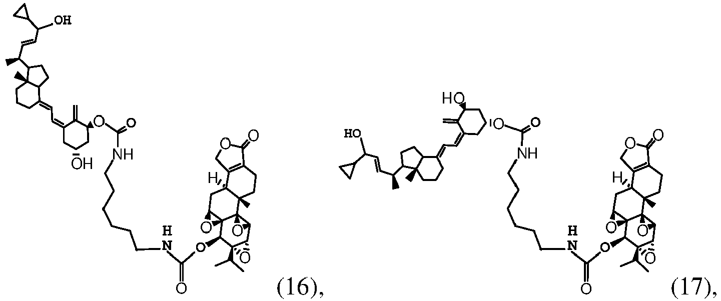

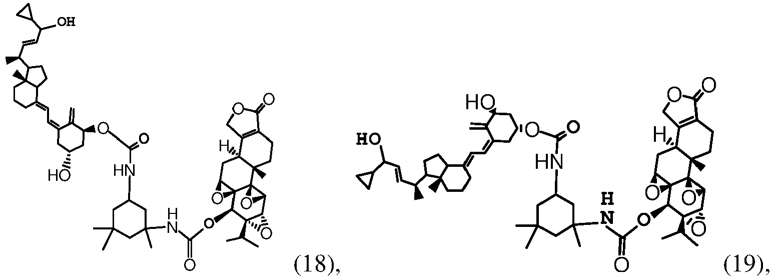

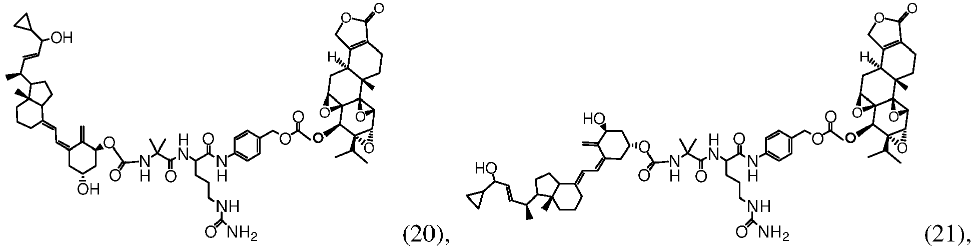

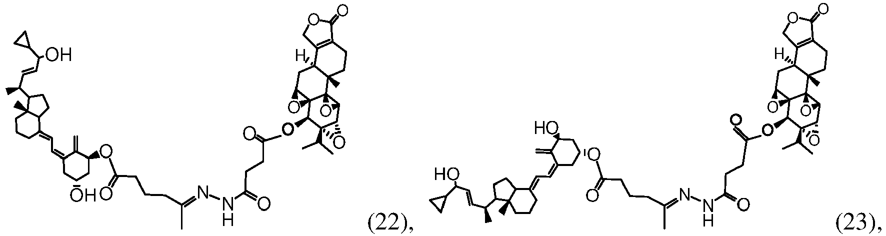

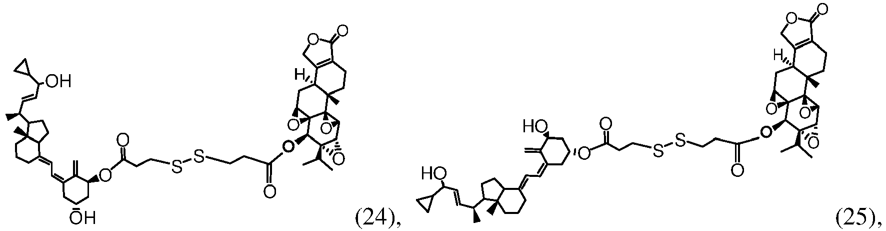

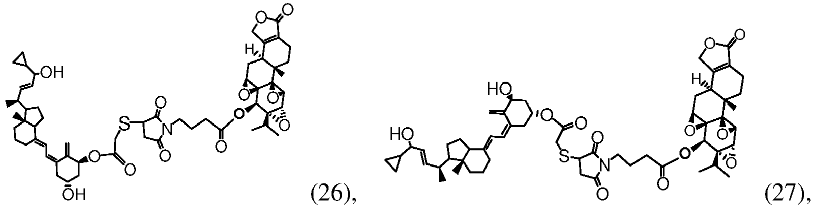

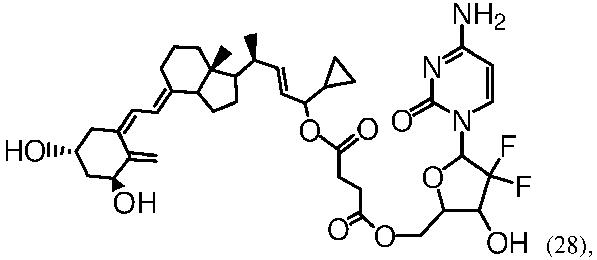

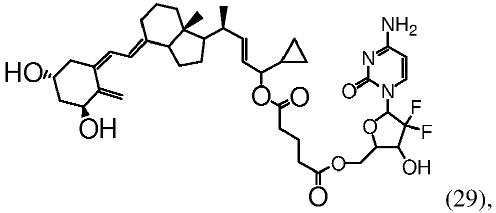

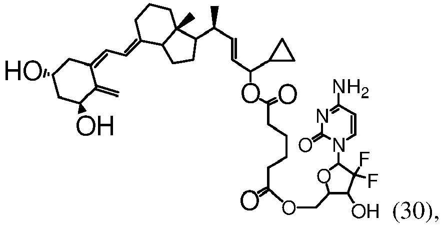

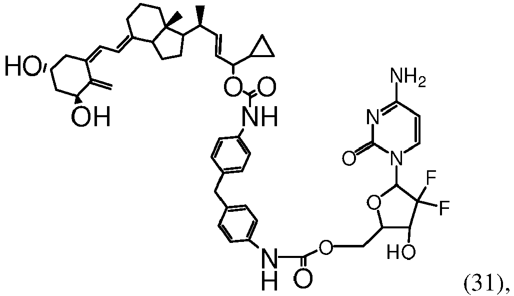

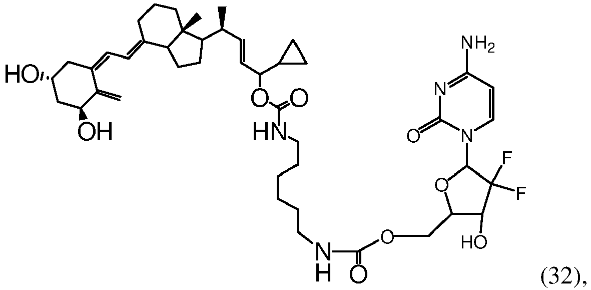

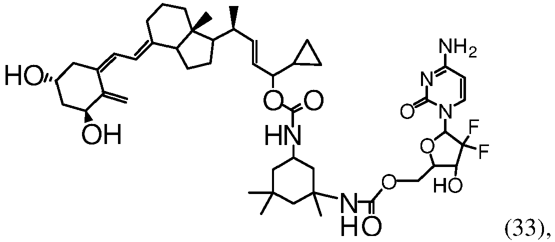

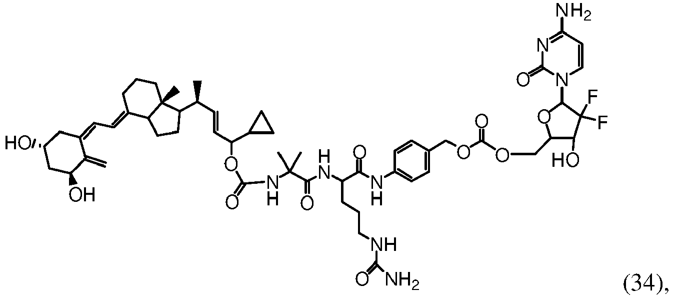

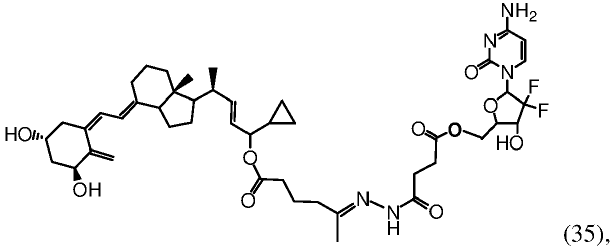

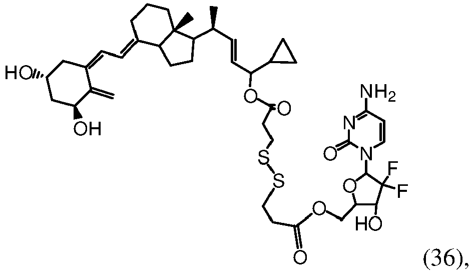

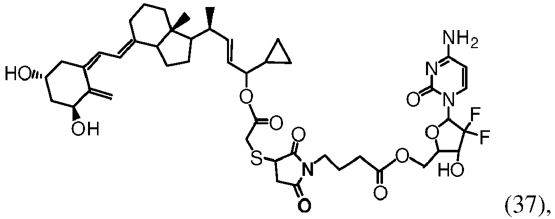

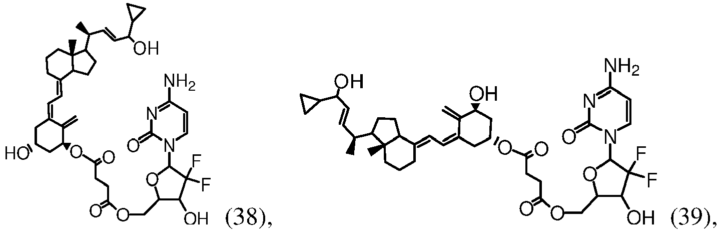

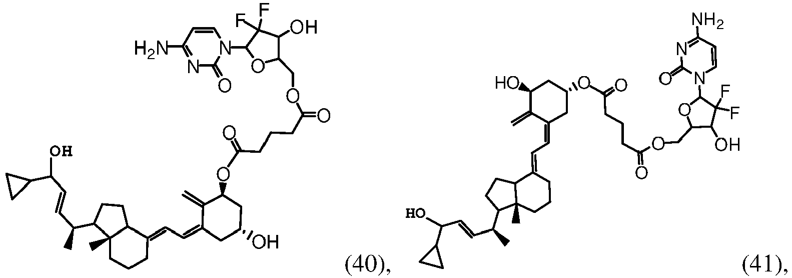

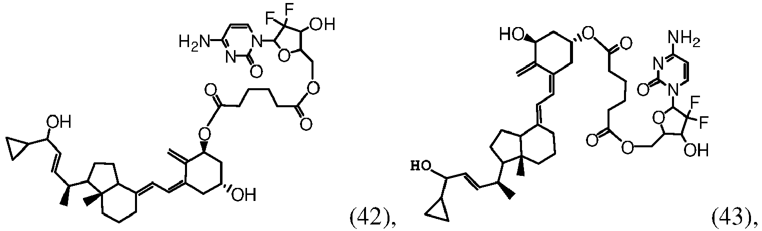

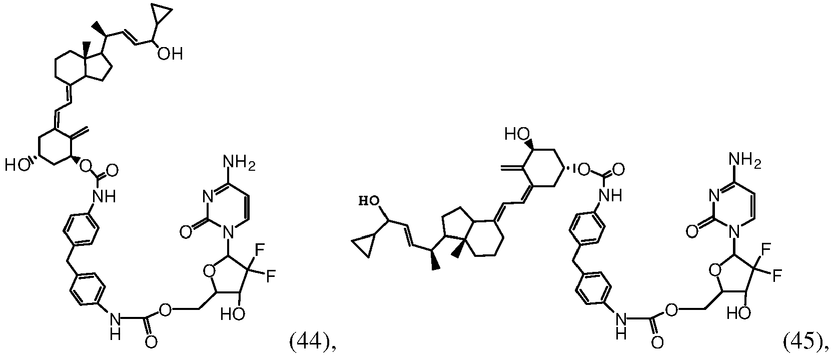

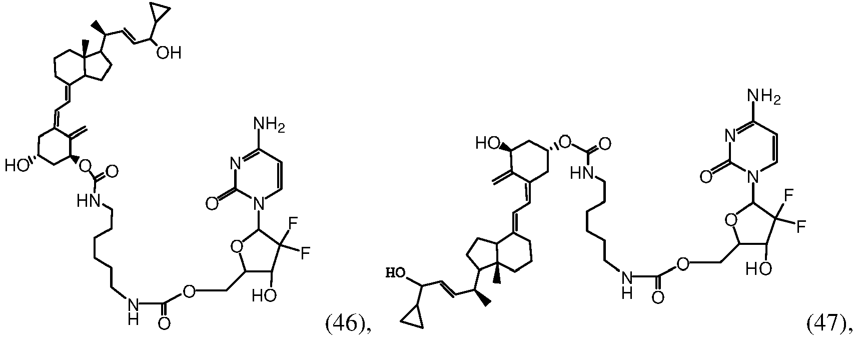

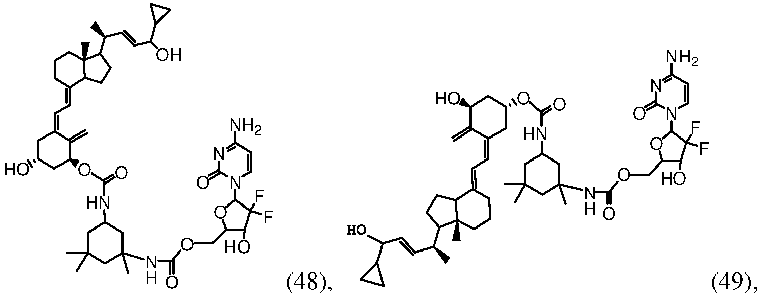

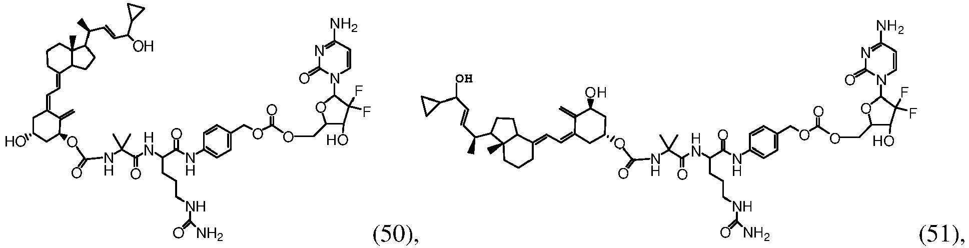

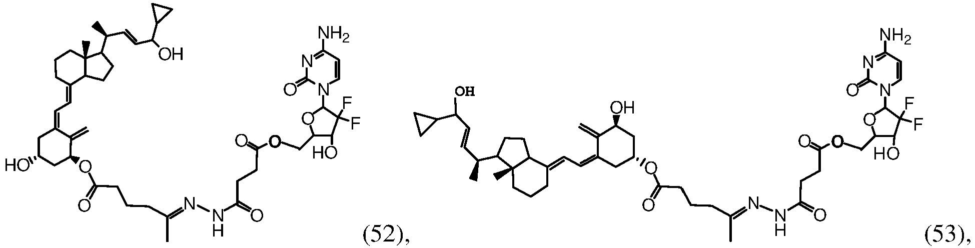

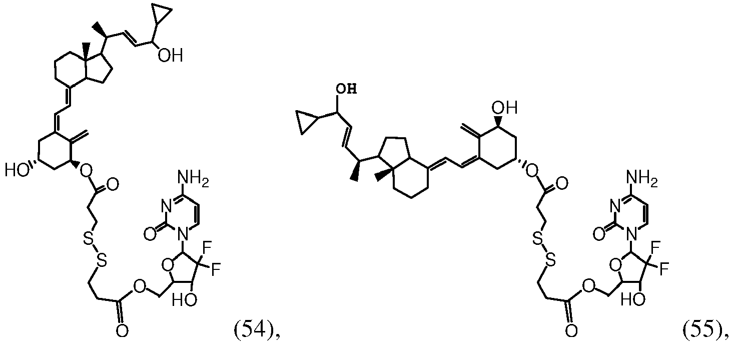

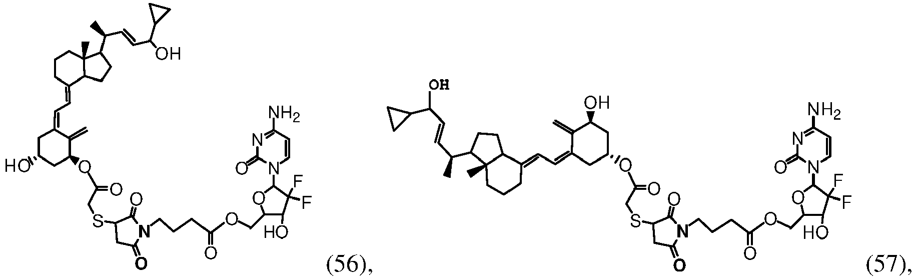

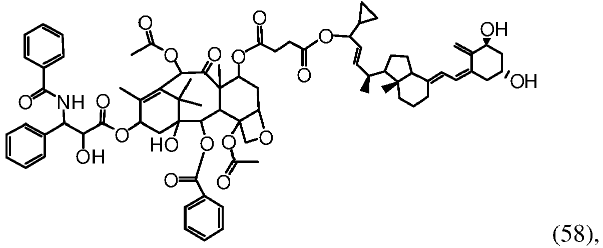

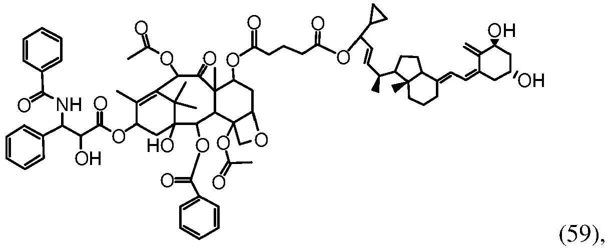

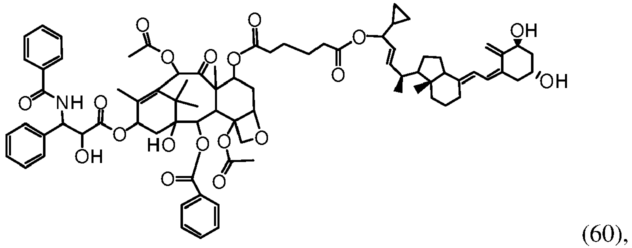

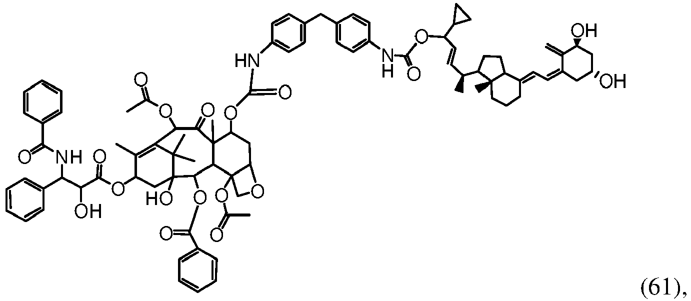

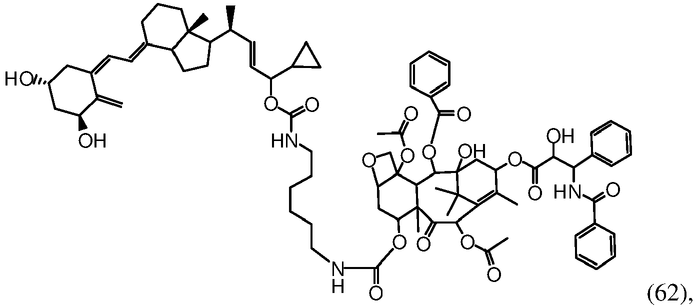

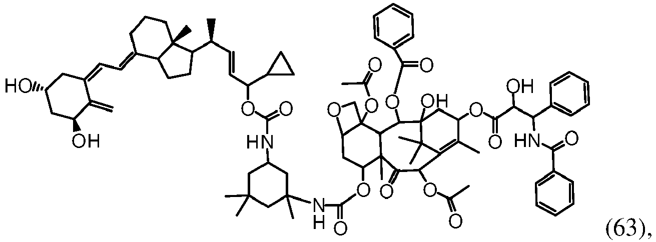

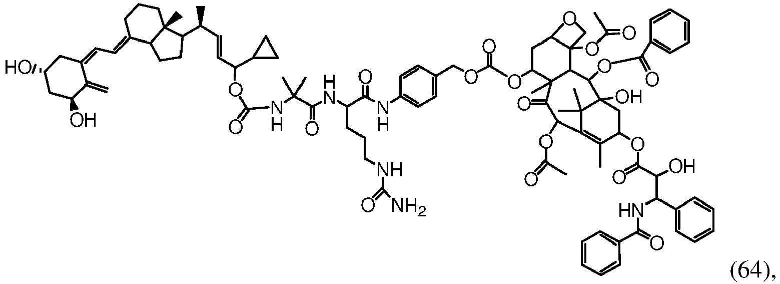

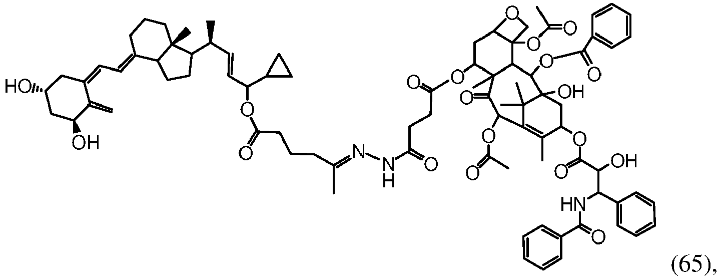

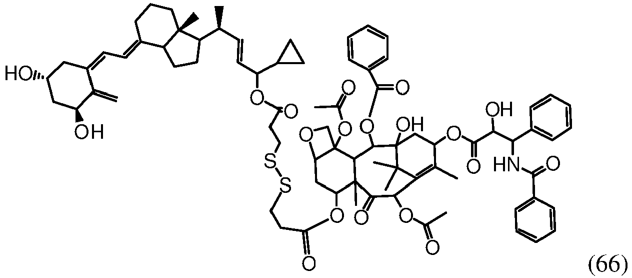

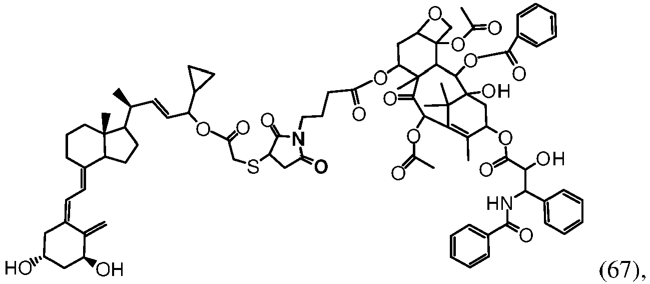

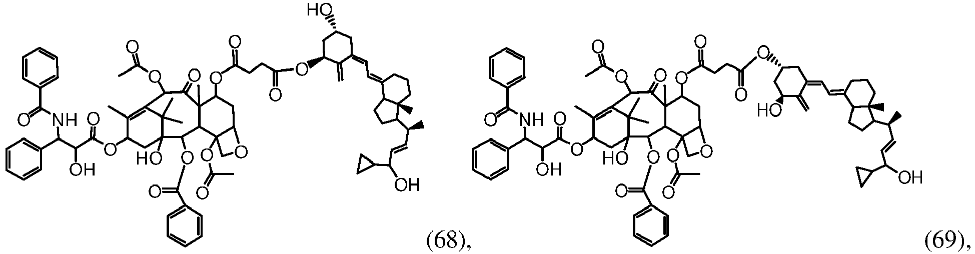

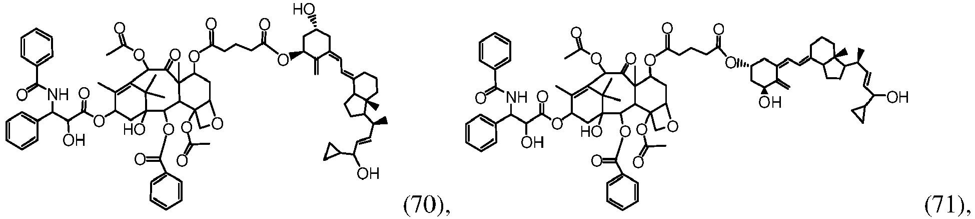

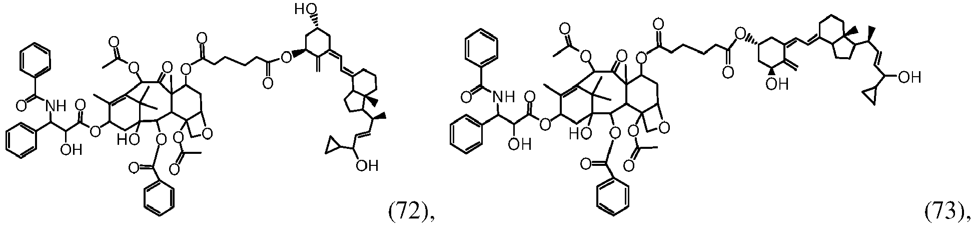

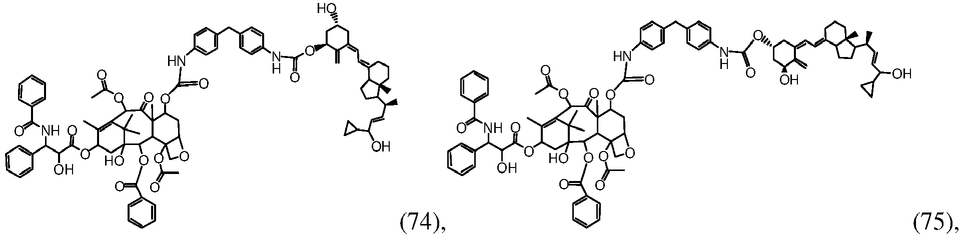

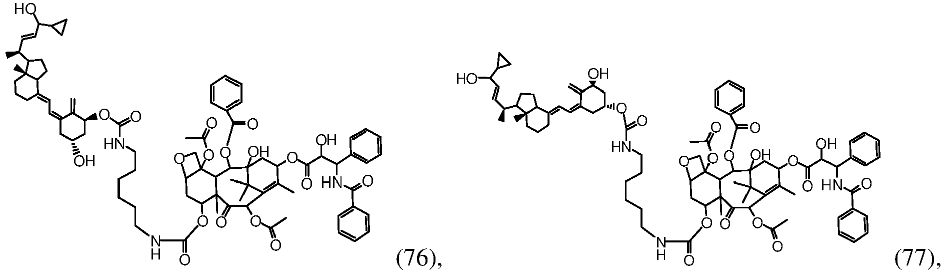

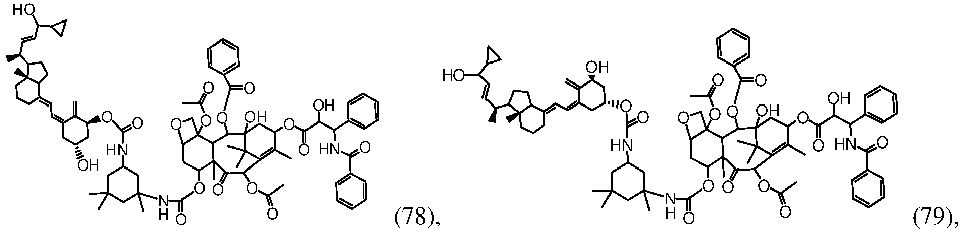

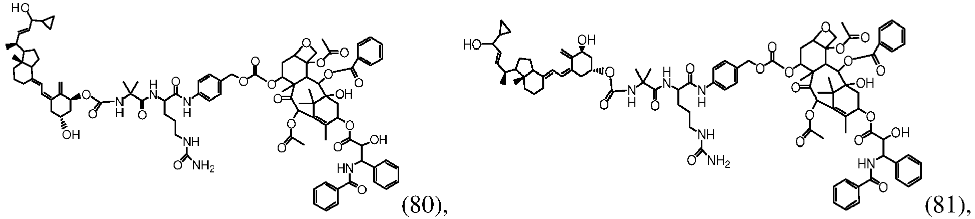

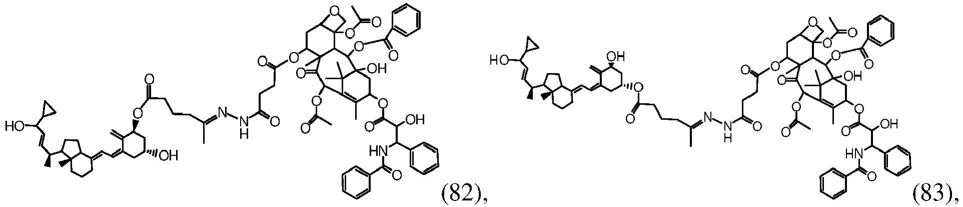

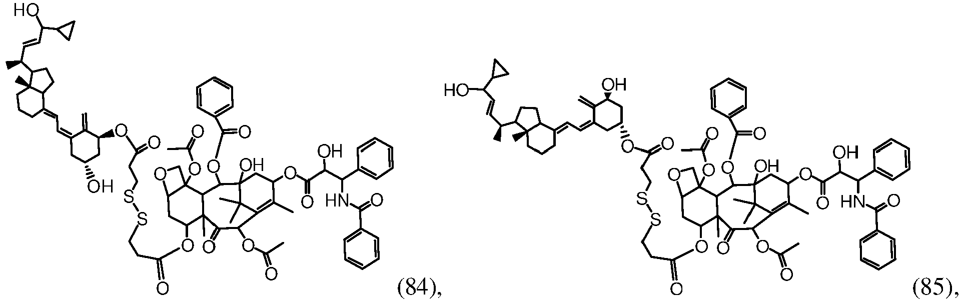

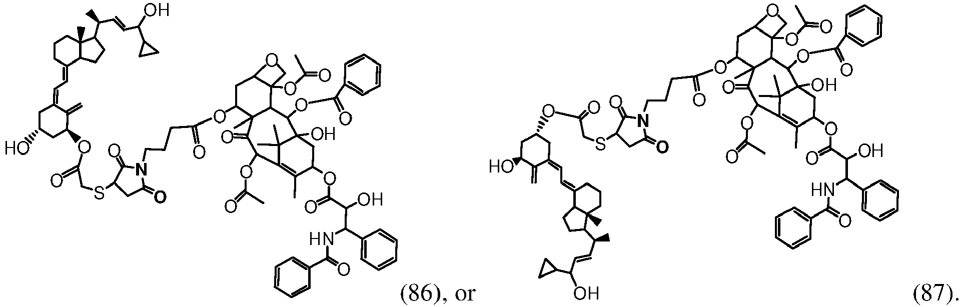

Compound (1) to compound (27) are coupling compounds formed by a coupling of calcipotriol and triptolide via an enzyme-degradable linker; compound (28) to compound (57) are coupling compounds formed by a coupling of calcipotriol and Gemcitabine via an enzyme-degradable linker; and compounds (58) to (87) are coupling compounds formed by a coupling of calcipotriol and paclitaxel via an enzyme-degradable linker.

The compound according to the embodiments of the present disclosure includes the tumor stroma-regulating group and the cytotoxic group that are coupled to each other. The tumor stroma-regulating group is used to regulate the tumor stroma, and the cytotoxic group is used to kill the tumor cells. The compound according to the embodiments of the present disclosure can act on the tumor stroma and the tumor cells at the same time, so as to achieve the purpose of eliminating or reducing the tumor stroma while killing tumor cells. The pharmacokinetic properties of the compound according to the embodiments of the present disclosure are improved, i.e., the pharmacokinetics of the tumor stroma-regulating group and the cytotoxic group are synchronized, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines.

In another aspect of the present disclosure, the present disclosure provides a compound. According to the embodiments of the present disclosure, the compound is a compound represented by formula (I), or the compound is an isomer (such as compound II, compound III), a stereoisomer, a geometric isomer, a tautomer, a nitrogen oxide, a hydrate, a solvate, a metabolite, a pharmaceutically acceptable salt or a prodrug of the compound represented by formula (I):

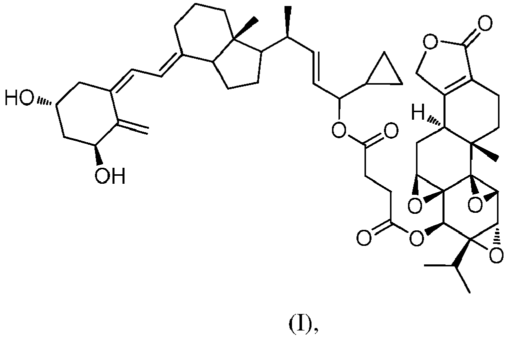

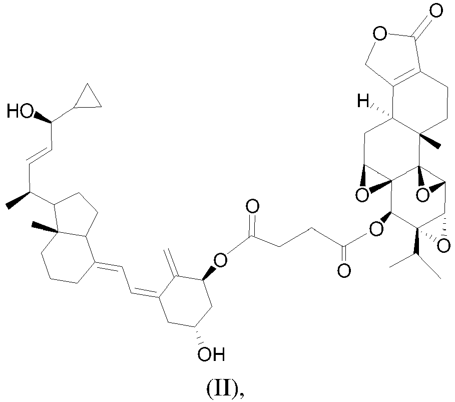

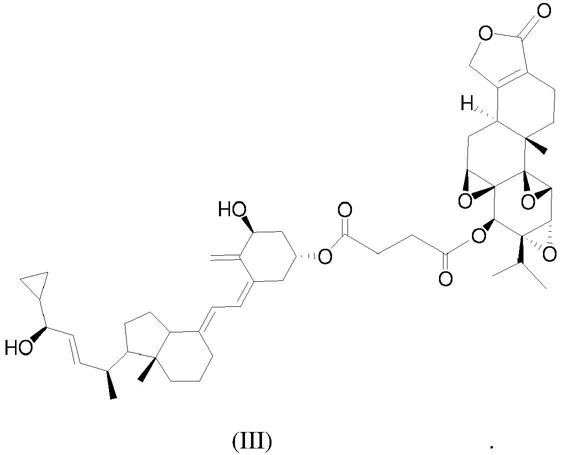

The above compound according to the embodiments of the present disclosure is formed by connecting triptolide and calcipotriol through an enzyme-degradable linker, so as to synthesize a brand new, dual-targeting coupling compound. Such a compound is named as "Callide" in the present disclosure. Compared with the original medicine TP, Callide has an increased molecular weight and a slower crystallization tendency, and it is more hydrophobic. Therefore, Callide can be easily loaded and delivered by means of a variety of nano-drug delivery systems (such as polymer micelles, albumin composite nanoparticles, liposomes, etc.), thereby optimizing pharmacokinetics of Callide and increasing a concentration of Callide at the tumor site, and reducing toxic and side effects of TP. Meanwhile, Callide can be only catalytically degraded by esterase in vivo to produce Cal and TP, such that the pharmacokinetics of Cal and TP can be better synchronized, thereby achieving the synergistic effects of promoting the PSC dormancy (by calcipotriol) and inhibiting the pancreatic cancer cells (by triptolide), and significantly reducing the toxic and side effects. The coupling compound according to the embodiments of the present disclosure can effectively control the formation of stroma in pancreatic cancer tissues and achieve effective aggregation of TP, thereby realizing a more effective treatment of pancreatic cancer. The compound according to the embodiments of the present disclosure has a longer half-life and therapeutic window as well as significantly reduced therapeutic toxicity compared to the existing drugs for cancer treatment.

"Isomers" refer to compounds that have the same molecular formula but different atomic arrangements, Simply, a phenomenon that compounds have the same molecular formula but different structures is called isomerism; and compounds having the same molecular formula but different structures are isomers to each other. Many isomers have similar properties. In organic chemistry, isomers can be the same type of substances (containing the same functional group) or different types of substances (containing different functional groups).

"Stereoisomers" refer to compounds that have the same chemical structure but different steric arrangements of atoms or groups. Stereoisomers include enantiomers, diastereomers, conformational isomers (rotamers), geometric isomers (cis/trans isomers), atropisomers, or the like.

"Chirality" refers to a property that a molecule is not overlapping with its mirror image. "Achirality" refers to a property that a molecule can overlap with its mirror image.

"Enantiomers" refer to two isomers of a compound that are each a mirror image of the other one and cannot overlap with one another.

"Diastereomers" refer to two stereoisomers of a compound that have two or more chiral centers and are each not a mirror image of the other one. Diastereomers have different physical properties, such as melting point, boiling point; different spectral properties and reactivity. A mixture of diastereomers can be separated by high-resolution analytical operations, for example, electrophoresis, and chromatography such as HPLC.

The definitions and rules of stereochemistry used in the present disclosure generally follow "McGraw-Hill Dictionary of Chemical Terms (1984)", S. P. Parker, Ed., McGraw-Hill Book Company, New York; and "Stereochemistry of Organic Compounds", Eliel, E. and Wilen, S., John Wiley & Sons, Inc., New York, 1994.

Many organic compounds exist in optically active forms, i.e., they are capable of rotating a plane of plane-polarized light. When describing optically active compounds, the prefixes *D* and *L,* or *R* and *S* are used to denote the absolute configurations of the molecule with respect to one or more chiral centers. The prefixes d and 1, or (+) and (-) are symbols used to specify a rotation of plane-polarized light caused by a compound, where (-) or 1 indicates that the compound is levorotatory, and the prefix (+) or d indicates that the compound is dextrorotatory. When specific stereoisomers are enantiomers, and a mixture of such isomers is called an enantiomeric mixture. A mixture of enantiomers in 50:50 is called a racemic mixture or a racemate, which may occur when there is no stereoselectivity or stereospecificity in a chemical reaction or process.

Any asymmetric atom (for example, carbon, etc.) of the compound of the present disclosure can present in a racemate- or enantiomer-enriched form, such as present in *(R)-, (S)-,* or *(R, S)*-configuration. In some embodiments, in terms of *(R)-,* or *(S)-*configuration, each asymmetric atom has an enantiomeric excess of at least 50%, an enantiomeric excess of at least 60%, an enantiomeric excess of at least 70%, an enantiomeric excess of at least 80%, an enantiomeric excess of at least 90%, an enantiomeric excess of at least 95%, or an enantiomeric excess of at least 99%.

In accordance with the selection of starting materials and methods, the compounds of the present disclosure may be present as one of the possible isomers or a mixture thereof, such as racemate and a mixture of diastereomers, which depends on a number of asymmetric carbon atoms. The optically active *(R)-* or *(S)*-isomers can be prepared using chiral synthons or chiral reagents, or resolved using conventional techniques. If the compound contains a double bond, the substituent may be in the *E* or *Z* configuration; and if the compound contains disubstituted cycloalkyl, the cycloalkyl substituent may have a *cis* or *trans* configuration.

Any obtained mixture of stereoisomers can be separated into pure or substantially pure stereoisomers, enantiomers, diastereomers, for example, by chromatography and/or fractional crystallization process.

The racemate of obtained end products or intermediates can be resolved into optical enantiomers by methods known to those skilled in the art, for example, by separating the obtained diastereomeric salts. Racemic products can also be separated by chiral chromatography, such as high-performance liquid chromatography (HPLC) using chiral adsorbents. Particularly, the enantiomers can be prepared by asymmetric synthesis, for example, referring to "Enantiomers, Racemates and Resolutions", Jacques, et al., Wiley Interscience, New York, 1981**;** "Principles of Asymmetric Synthesis", 2nd Ed. Robert E. Gawley, Jeffrey Aubé, Elsevier, Oxford, UK, 2012**;** "Stereochemistry of Carbon Compounds", Eliel, E.L., McGraw-Hill, NY, 1962**;** "Tables of Resolving Agents and Optical Resolutions", p. 268, Wilen, S.H., E.L. Eliel, Ed., Univ. of Notre Dame Press, Notre Dame, in 1972**;** and "Chiral Separation Techniques: A Practical Approach", Subramanian, G. Ed., Wiley-VCH Verlag GmbH & Co. KGaA, Weinheim, Germany, 2007**.**

The term "tautomer" or "tautomeric form" refers to structural isomers that have different energies and can be interconverted by a low energy barrier. If tautomerism is possible (for example, in solution), a chemical equilibrium of tautomers can be reached. For example, proton tautomers (also known as prototropic tautomers) include interconversion through proton migration, such as ketone-enol isomerization and imine-enamine isomerization. Valence tautomer includes interconversion through a recombination of some bond electrons. A specific example of ketone-enol tautomerization is an interconversion of pentane-2,4-dione and 4-hydroxypent-3-en-2-one tautomers. Another example of tautomerism is phenol-ketone tautomerization. A specific example of phenol-ketone tautomerization is an interconversion of pyridine-4-ol and pyridine-4(1H)-one tautomers. Unless otherwise indicated, all tautomeric forms of the compound of the present disclosure shall fall within the scope of the present disclosure.

The term "prodrug" used in the present disclosure indicates a compound that is capable of being converted into a compound represented by formula (I) in vivo. Such conversion is affected by a prodrug hydrolysis in blood or an enzymatic conversion into a parent structure in blood or tissue. The prodrug compounds of the present disclosure may be esters. In the present disclosure, the esters used as prodrugs include phenyl esters, aliphatic (C1-24) esters, acyloxymethyl esters, carbonic esters, carbamates and amino acid esters. For example, a compound in the present disclosure contains a hydroxyl group, which can be acylated to obtain a compound in the form of a prodrug. Other forms of the prodrug include phosphate esters, for example, the phosphate ester compounds obtained by phosphorylation of the hydroxyl group on the parent structure. For a full discussion of prodrugs, please refer to the following literatures: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

"Metabolite" refers to a product obtained by metabolizing a specific compound or its salt in vivo. The metabolite of a compound can be identified by techniques well known in the art, and its activity can be characterized by assays as described in the present disclosure. Such products may be obtained through oxidation, reduction, hydrolysis, amidation, deamidation, esterification, de-esterification, or enzymatic cleavage of the administrated compound, or the like. Accordingly, the present disclosure includes the metabolites of the compound, including metabolites produced by fully contacting the compound of the present disclosure with a mammal for a period of time.

As used herein, a "pharmaceutically acceptable salt" refer to an organic and inorganic salt of the compound of the present disclosure. The pharmaceutically acceptable salts are well known to those in the art, as described in the literature: S. M. Berge et al., describe pharmaceutically acceptable salts in detail in J. Pharmaceutical Sciences, 1977, 66: 1-19. Salts formed by pharmaceutically acceptable non-toxic acids include, but are not limited to, inorganic acid salts formed by reacting with amino groups, including hydrochloride, hydrobromide, phosphate, sulfate, perchlorate; and organic acid salts such as acetate, oxalate, maleate, tartrate, citrate, succinate, malonate, or the salts obtained through other methods such as ion exchange described in the literature. Other pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecyl sulfate, esilate, formate, fumarate, glucoheptonate, glycerophosphate, gluconate, hemisulfate, enanthate, caproate, hydroiodide, 2-hydroxy-ethanesulfonate, Lacturonate, lactate, laurate, lauryl sulfate, malate, malonate, methanesulfonate, 2-naphthalenesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, valerate, etc. Salts obtained by suitable bases include alkali metal, alkaline earth metal, ammonium, and N+(C1-4 alkyl)4 salts. The present disclosure also contemplates quaternary ammonium salts formed by any compound with a group containing N. Water-soluble or oil-soluble or dispersed products can be obtained by quaternization. The alkali metal or alkaline earth metal salts include sodium, lithium, potassium, calcium, magnesium, and the like. The pharmaceutically acceptable salts further include suitable and non-toxic ammonium, quaternary ammonium salts as well as amine cations formed by counterions such as halides, hydroxides, carboxylates, sulfates, phosphates, nitrates, C1-8 sulfonates and aromatic sulfonates.

In the present disclosure, a "solvate" refers to an association compound formed by one or more solvent molecules and the compound of the present disclosure. The solvents for forming the association compound include, but are not limited to, water, isopropanol, ethanol, methanol, dimethyl sulfoxide, ethyl acetate, acetic acid, and aminoethanol. The term "hydrate" refers to an association compound formed by water as the solvent molecules.

For the term "treating" any disease or disorder as used herein, in some embodiments, "treating" or "treatment" refers to ameliorating the disease or disorder (i.e., slowing or preventing or alleviating the development of the disease or at least one of its clinical symptoms); in other embodiments, "treating" or "treatment" refers to alleviating or improving at least one physical parameter, including physical parameters that may not be perceived by the patient; in other embodiments, "treating" or "treatment" refers to regulating a disease or disorder physically (e.g., stabilizing perceptible symptoms) or physiologically (e.g., stabilizing the parameters of the body) or in both aspects; and in other embodiments, "treating" or "treatment" refers to preventing or delaying the onset, occurrence, or deterioration of a disease or disorder.

Pharmaceutically acceptable acid addition salts can be formed with inorganic and organic acids, such as acetate, aspartate, benzoate, benzenesulfonate, bromide/hydrobromide, bicarbonate/carbonate, bisulfate/sulfate, camphorsulfonate, chloride/hydrochloride, chlorotheophylline, citrate, ethanedisulfonate, fumarate, glucoheptonate, gluconate, glucuronate, hippurate, hydroiodide/iodide, isethionate, lactate, lacturonate, lauryl sulfate, malate, maleate, malonate, mandelate, methanesulfonate, methylsulfate, naphthoate, naphthalenesulfonate, nicotinate, nitrate, octadecate, oleate, palmitate, pamoate, pectate, phosphate/hydrogen phosphate/dihydrogen phosphate, polygalacturonate, propionate, stearate, succinate, sulfosalicylate, tartrate, tosylate, and trifluoroacetate.

Inorganic acids, from which salts can be derived, include, for example, hydrochloric acid, hydrobromic acid, sulfuric acid, nitric acid, phosphoric acid, and the like.

Organic acids, from which salts can be derived, include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, p-toluenesulfonic acid, sulfosalicylic acid, etc.

Pharmaceutically acceptable base addition salts can be formed with inorganic and organic bases.

Inorganic bases, from which salts can be derived, include, for example, ammonium salts and metals from Groups I to XII of the Periodic Table. In some embodiments, the salts are derived from sodium, potassium, ammonium, calcium, magnesium, iron, silver, zinc, and copper; particularly suitable salts include ammonium, potassium, sodium, calcium, and magnesium salts.

Organic bases, from which salts can be derived, include primary amines, secondary amines and tertiary amines, and substituted amines include naturally occurring substituted amines, cyclic amines, basic ion exchange resins, and the like. Certain organic amines include, for example, isopropylamine, benzathine, cholinate, diethanolamine, diethylamine, lysine, meglumine, piperazine, and tromethamine.

The pharmaceutically acceptable salts of the present disclosure can be synthesized from the parent compound, basic or acidic moieties using conventional chemical methods. In general, such salts can be prepared by reacting the compounds in free acid form with a stoichiometric amount of a suitable base (such as hydroxides, carbonates, or bicarbonates of Na, Ca, Mg or K, and the like), or by reacting the compounds in free base form with a stoichiometric amount of a suitable acid. Such reactions are usually carried out in water or organic solvents, or a mixture thereof. Generally, non-aqueous media such as diethyl ether, ethyl acetate, ethanol, isopropanol, or acetonitrile need to be used where appropriate. For example, a list of other suitable salts can be found in "Remington's Pharmaceutical Sciences", 20th edition, Mack Publishing Company, Easton, Pa., 1985; and " Handbook of Pharmaceutical Salts: Properties, Selection, and Use", Stahl and Wermuth (Wiley-VCH, Weinheim, Germany, 2002).

In addition, the compounds of the present disclosure, including the salts thereof, can also be obtained in the form of their hydrates or in the form containing solvents thereof (for example, ethanol, DMSO, etc.), for the crystallization thereof. The compounds of the present disclosure can form solvates inherently or by design with pharmaceutically acceptable solvents (including water). Therefore, the present disclosure is intended to include both solvated and unsolvated forms.

Any structural formulas given in the present disclosure are also intended to represent the forms in which these compounds are not isotopically enriched and isotopically enriched. The isotope-enriched compounds have the structure depicted by the general formula given in the present disclosure, except that one or more atoms are replaced with atoms having a selected atomic weight or mass number. Exemplary isotopes that can be incorporated into the compounds of the present disclosure include isotopes of hydrogen, carbon, nitrogen, oxygen, phosphorus, sulfur, fluorine, and chlorine, such as ²H, ³H, ¹¹C, ¹³C, ¹⁴C, ¹⁵N, ¹⁷O, ¹⁸O, ¹⁸F, ³¹P, ³²P, ³⁵S, ³⁶C1, and ¹²⁵I.

In another aspect, the compounds described in the present disclosure include isotopically enriched compounds defined in the present disclosure, for example, those in which radioactive isotopes such as ³H, ¹⁴C, and ¹⁸F are present, or in which non-radioactive isotopes such as ²H and ¹³C are present. Such isotope-enriched compounds can be used for metabolic studies (using ¹⁴C), reaction kinetics studies (for example, using ²H or ³H), detection or imaging techniques such as positron emission tomography (PET) or single photon emission computed tomography (SPECT) including determination of drugs or substrate tissue distribution, or can be used in radiotherapy of patients. ¹⁸F-enriched compounds are particularly ideal for PET or SPECT studies. The isotope-enriched compounds of formula (I) can be prepared by conventional techniques familiar to those skilled in the art or as described in the examples and preparation procedures of the present disclosure, using suitable isotope-labeled reagents instead of the previously used unlabeled reagents.

In addition, the substitution of heavier isotopes, especially deuterium (i.e., ²H or D), can provide certain therapeutic advantages that are caused by higher metabolic stability, for example, an increase in in vivo half-life, a decrease in dosage requirements, or an improvement in the therapeutic index. It should be understood that, deuterium in the present disclosure is regarded as a substituent of the compound represented by formula (I). A concentration of this type of heavier isotope, especially deuterium, can be defined by an isotope enrichment factor. As used herein, the term "isotope enrichment factor" refers to a ratio between an isotopic abundance and a natural abundance of a specified isotope. If the substituent of the compound of the present disclosure is designated as deuterium, for each designated deuterium atom, the compound has an isotope enrichment factor of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% of deuterium incorporation), at least 6466.7 (97% deuterium incorporation), at least 6600 (99% deuterium incorporation), or at least 6633.3 (99.5% deuterium incorporation). The pharmaceutically acceptable solvates of the present disclosure include those in which the crystallization solvent may be isotopically substituted, such as D2O, acetone-d6, or DMSO-d6.

### Pharmaceutical Composition

In a second aspect of the present disclosure, the present disclosure provides a pharmaceutical composition. According to the embodiments of the present disclosure, the pharmaceutical composition includes the above-described compound as the active ingredient. The pharmaceutical composition according to the embodiments of the present disclosure can effectively control the formation of stroma in cancer tissues and achieve more effective treatment of tumors. The pharmaceutical composition according to the embodiments of the present disclosure has a longer half-life and therapeutic window as well as significantly reduced therapeutic toxicity compared to the existing drugs for cancer treatment.

According to the embodiments of the present disclosure, the pharmaceutical composition further includes pharmaceutically acceptable excipients.

According to the embodiments of the present disclosure, the formulation of the pharmaceutical composition is not particularly limited, and those skilled in the art can flexibly choose according to the actual situation. According to the embodiments of the present disclosure, the pharmaceutical composition is formulated in a form of micelles, emulsion, capsules, pills, tablets, granules, oral liquid, oral ointment, aerosol, or spray, thereby facilitating the administration.

Polymer micelles, as drug carriers, are attracting more and more attention. The polymer micelles can form a unique core-shell structure, having an outer shell formed by a hydrophilic segment and an inner core formed by a hydrophobic segment, such that a hydrophobic drug can be wrapped in the inner core of the micelles and protect the loaded drug. The size of micelles is generally 10-100 nanometers, allowing them to escape the phagocytosis of the human reticuloendothelial system (RES) and the influence of other human internal environment, while increasing the high permeability and retention effect (EPR) of solid tumors. Therefore, according to the embodiments of the present disclosure, the pharmaceutical composition is in the form of micelles, and the carrier of the micelles is a degradable amphiphilic polymer, including polyethylene-glycol polylactic acid diblock copolymer (PEG-PLA); monomethoxy polyethylene-glycol (mPEG) polylactic acid (levorotatory, dextrorotatory, or racemic): mPEG-PLA; monomethoxy polyethylene-glycol (mPEG) polylactic acid-glycolic acid copolymer (in different ratios): mPEG-PLGA; monomethoxy polyethylene glycol (mPEG) polycaprolactone (PCL): mPEG-PCL; monomethoxy polyethylene glycol (mPEG) polytrimethylene carbonate (PTMC): mPEG-PTMC; or monomethoxy polyethylene glycol (mPEG) polyamino acids (polylysine, polyglutamic acid, polyaspartic acid, polyornithine, polyarginine, polyhistidine, etc.). Among them, the molecular weight of each block of each polymer can be designed and synthesized as needed. For example, polyethylene-glycol-polylactic acid diblock copolymer (PEG-PLA) has a molecular weight of 2000 to 2000. Those skilled in the art can use the following synthetic schemes: PEG-PLA is synthesized by using monohydroxy PEG as an initiator and lactide as polymerization monomer to form a block polymer through a ring-opening polymerization. If a molar amount of PEG keeps unchanged, a length of the PLA segment can be controlled by changing a molar concentration of the lactide monomer, so as to obtain polymers with a constant PEG chain length and different PLA chain lengths. Similarly, polymers with different PEG chain lengths and a constant PLA chain length can be designed and synthesized by adopting PEG of different molecular weights and then fixing a concentration of lactide. Therefore, the two segments of the amphiphilic block polymer can be obtained by chemical synthesis, adjusting the polymerization concentration and ratio thereof to obtain any molecular weight. In addition, studies have indicated that a drug-loading capacity, in vitro and in vivo stability of the formed micelles, rates of drug release in vivo and in vitro, etc. are varying with the different polymer chain lengths. If the formulation is optimized by adjusting the type and properties of the polymer on basis of the present disclosure, it is very likely that better in vivo and in vitro therapeutic effects can be achieved. For example, according to the embodiments of the present disclosure, PEG-PLA is synthesized by using monohydroxy PEG as an initiator and lactide as polymerization monomer to form a block polymer through a ring-opening polymerization. If a molar amount of PEG keeps unchanged, a length of the PLA segment can be controlled by changing a molar concentration of the lactide monomer, to obtain polymers with a constant PEG chain length and different PLA chain lengths. Similarly, polymers with different PEG chain lengths and a constant PLA chain length can be designed and synthesized by adopting PEG of different molecular weights and then fixing a concentration of lactide. Therefore, the two segments of the amphiphilic block polymer can be obtained by chemical synthesis, adjusting the polymerization concentration and ratio thereof to obtain any molecular weight. In addition, studies have indicated that a drug-loading capacity, in vitro and in vivo stability of the formed micelles, rates of drug release in vivo and in vitro, etc. are varying with the different polymer chain lengths. On basis of the present disclosure, the formulation is optimized by adjusting the type and properties of the polymer, such that better in vivo and in vitro therapeutic effects can be achieved, which also falls within the protection scope of the present disclosure. In this way, the active ingredient can be prevented from being cleared and degraded by the human reticuloendothelial system into non-toxic monomers and excreted out of the body, and the hydrophilic segment of PEG has the advantages of being easily soluble in water, easy to flow and low toxicity, which can achieve an effect of a long circulation. In addition, the micelle system can also effectively increase the drug loading and bioavailability of the active ingredient, thereby better exerting the efficacy of treating or preventing cancer.

According to an embodiment of the present disclosure, the pharmaceutical composition further includes additional anti-pancreatic cancer medicines. The additional anti-pancreatic cancer medicines include one or more of 5-fluorouracil, Gemcitabine, FOLFIRINOX, nanopaclitaxel/gemcitabine combination, and ONIVYDE™. By combining with other medicines for treating pancreatic cancer, the pharmaceutical composition has more significant therapeutic effect on pancreatic cancer.

The compound of the present disclosure can be produced and formulated into forms of racemic mixtures, isomers, enantiomers, diastereomers, rotamers, N-oxides, polymorphs, solvates, pharmaceutically acceptable salts, and active metabolites; and a pharmaceutical composition can also be produced, the pharmaceutical composition containing the compound represented by formula (I) or its metabolites, enantiomers, diastereomers, N-oxides, polymorphs, solvates, pharmaceutically acceptable salts, and active metabolites, as well as a pharmaceutically acceptable carrier and optionally an excipient.

The pharmaceutical composition of the present disclosure can be produced and administered in dosage units, and each unit contains a certain amount of at least one compound of the present disclosure and/or at least one physiologically acceptable addition salt thereof. The dosage can vary within a very wide range, as the compound is effective even at low dosage levels and is relatively non-toxic. The compound can be administered in a therapeutically effective low micromolar dosage, and as needed, the dosage can be increased to the maximum dosage that the patient can bear.

A therapeutically effective amount of the compound represented by formula (I) and a pharmaceutically acceptable salt thereof, when can be used for treatment, can be administered as an unprocessed chemical or as an active ingredient of a pharmaceutical composition. Accordingly, the present disclosure also provides a pharmaceutical composition, and the pharmaceutical composition includes a therapeutically effective amount of the compound represented by formula (I) or a pharmaceutically acceptable salt thereof, as well as one or more pharmaceutically acceptable carriers, diluents, or excipients.

In fact, according to conventional pharmaceutical compounding techniques, the compound of the present disclosure or a pharmaceutically acceptable salt thereof, as an active ingredient, can be combined with a pharmaceutical carrier in an intimately mixed manner. The carrier can have a wide variety of forms, depending on the form of formulation desired to be administered, such as oral or parenteral (including intravenous). Therefore, the pharmaceutical composition can be present as separate units suitable for oral administration, such as capsules, cachets, or tablets, each of which contains a predetermined amount of active ingredient. In addition, the composition can be in the following forms: powder, granules, solutions, suspensions in aqueous liquids, non-aqueous liquids, oil-in-water emulsions, or water-in-oil liquid emulsions. In addition to the common formulations mentioned above, the compound or pharmaceutically acceptable salts thereof can also be administered by controlled release means and/or delivery devices. The composition can be prepared by any method in the pharmaceutical industry. Generally, such methods include a step of combining the active ingredient with the carrier, which constitutes one or more necessary ingredients. Generally, the composition is prepared by uniformly and intimately mixing the active ingredient with a liquid carrier or a finely divided solid carrier or both. Then, the product can be conveniently prepared into the desired form.

The employed pharmaceutical carrier can be solid, liquid, or gas. Examples of solid carriers include lactose, gypsum powder, sucrose, talc, gelatin, agar, pectin, acacia, magnesium stearate, and stearic acid. Examples of liquid carriers are syrup, peanut oil, olive oil and water. Examples of gas carriers include carbon dioxide and nitrogen.

The term "therapeutically effective amount" as used in the present disclosure refers to a total amount of respective active ingredients enough to exhibit a meaningful patient benefit. When an individual active ingredient is administered alone, the term refers only to this ingredient. When the active ingredients used in combination, the term refers to a combined amount of the active ingredients that cause a therapeutic effect regardless of combination, sequential or simultaneous administration. The compound represented by formula (I) and the pharmaceutically acceptable salt thereof are as described above. The carriers, diluents or excipients must be acceptable in the sense of being compatible with the other ingredients of the formulation and not harmful to its recipient. According to another aspect of the present disclosure, a method for preparing a pharmaceutical formulation is also provided, and the method includes uniformly mixing the compound represented by formula (I) or a pharmaceutically acceptable salt thereof with one or more pharmaceutically acceptable carriers, diluents or excipients. The term "pharmaceutically acceptable" as used in the present disclosure refers to such compounds, raw materials, compositions and/or formulations that, within the scope of reasonable medical judgment, are suitable for contact with patient tissues without excessive toxicity, irritation, allergies or other problems and complications commensurate with a reasonable benefit/risk ratio, and can be effectively used for their intended purpose.

Generally, the compound of the present disclosure is administered in a therapeutically effective amount by any conventional manner of administration for substances that exert similar effects. A suitable dosage range is typically 1-500 mg per day, preferably 1-100 mg per day, and most preferably 1-30 mg per day, depending upon various factors such as the severity of the disease to be treated, the age and relative health of the subject, the efficacy of the compounds used, the route and form of administration, the indications targeted for administration, and the preferences and experience of the relevant medical practitioner. A person of ordinary skill in the field of treating the disease can determine the therapeutically effective amount of the compound of the present disclosure for a given disease relying on personal knowledge and the present disclosure, without undue experimentation.

The compound of the present disclosure can be administrated according to the needs of patients, for example, oral administration, nasal administration, parenteral administration (subcutaneous, intravenous, intramuscular, intrasternal and infusion), inhalation administration, transrectal administration, transvaginal administration, body surface administration, topical administration, transdermal administration, and transocular administration.

Various solid oral formulations can be used for administration of the compound of the present disclosure, for example, solid formulations of tablets, soft capsules, capsules, caplets, granules, lozenges, and bulk powders. The compounds of the present disclosure can be administered alone or in combination with various pharmaceutically acceptable carriers, diluents (e.g., sucrose, mannitol, lactose, starch) and excipients known in the art, including, but not limited to, suspending agents, solubilizers, buffers, binders, disintegrating agents, preservatives, coloring agents, flavoring agents, lubricants, etc. Time-controlled release capsules, tablets and gels are also advantageous for the administration of the compounds of the present disclosure.

Tablets can be prepared by compression or molding, optionally using one or more auxiliary ingredients or adjuvants. Compressed tablets can be prepared by compressing the active ingredient in a free-flowing form (e.g., powder or granules) in a suitable machine, optionally mixed with a binder, lubricant, inert diluent, surfactant, or dispersant. Molded tablets can be prepared by molding a mixture of powdered compounds moistened with an inert liquid diluent in a suitable machine. Each tablet preferably contains about 0.1 mg to about 500 mg of active ingredient; and each caplet or capsule preferably contains about 0.1 mg to about 500 mg of active ingredient. Accordingly, in the case of taking one or two tablets, caplets or capsules (once, twice or three times a day), the tablets, capsules or capsules conveniently contain 0.1mg, 1mg, 5mg, 25mg, 50mg, 100mg, 200mg, 300mg, 400mg, or 500mg of active ingredient.

The compound of the present disclosure can also be administered in a variety of liquid oral formulations, including aqueous and anhydrous solutions, emulsions, suspensions, syrups, and elixirs. Such formulations can also contain suitable inert diluents known in the art, such as water, and suitable excipients known in the art, such as preservatives, lubricants, sweeteners, flavoring agents, as well as agents for emulsifying and/or suspending the compounds of the present disclosure. The compound of the present disclosure can be administered by injection in a form of an isotonic sterile solution, for example, intravenous injection. Other preparations are also possible.

Suppositories for rectal administration of the compound of the present disclosure can be prepared by mixing the compound with suitable excipients such as cocoa butter, salicylate, and polyethylene glycol.

Formulations for vaginal administration can be in a form of creams, gels, pastes, foams, or sprays, and they contain suitable carriers known in the art, in addition to the active ingredients.

For topical administration, the pharmaceutical composition can be in forms of cream, ointment, liniment, lotion, emulsion, suspension, gel, solution, paste, powder, sprays and drops that are suitable for administration to skin, eyes, ears, or nose. The topical administration can also include transdermal administration by, for example, a transdermal patch.

For the treatment of respiratory diseases, the compound of the present disclosure is preferably administered by inhalation. In this regard, the compound can be administered directly as powders (preferably in a micronized form) or administered by spray solutions or suspensions containing the compound.

Inhalable preparations include inhalable powders, propellant-containing metered aerosols, or propellant-free inhalable preparations.

The powder compound of the present disclosure can be added with a diluent or carrier, which is generally non-toxic and chemically inert to the compound of the present disclosure, such as lactose or any other additive suitable for improving the respiration.

Inhalation aerosols containing gaseous propellants such as hydrofluoroalkanes may contain the compound of the present disclosure in a solution or dispersed form. The propellant-driven formulation can also contain other ingredients, such as co-solvents, stabilizers, and optionally other excipients.

The propellant-free inhalable formulations containing the compound of the present disclosure can be in a form of solution or suspension in aqueous media, alcoholic media, or aqueous alcohol media, and they can be delivered through jet nebulizers or ultrasonic nebulizers known in the art, or delivered though soft-mist nebulizers such as Respimat®.

The compound of the present disclosure can be administered as a single active ingredient or in combination with other pharmaceutically active ingredients, including those currently used for treating pancreatic cancer, for example, 5-fluorouracil, Gemcitabine, FOLFIRINOX, nanopaclitaxel/Gemcitabin combination, and ONIVYDE™.

Preferably, the compound represented by formula (I) administered alone or in combination with other active ingredients is used for preventing and/or treating cancer.

The term "used in combination with" or "combination" shall be understood as meaning that the respective ingredients can be administered simultaneously or more or less simultaneously, or they are administrated separately in a sequence. In some embodiments, one therapeutic agent/pharmaceutical active ingredient can be administered in the morning and the other one is administered later on the same day. In other embodiments, one therapeutic agent/pharmaceutical active ingredient can be administered once a day, and the other is administered once a week. It shall be understood that, if the ingredients are administered directly in succession, a delayed administration of the second ingredient should not cause a loss of the beneficial effect of the combination.

The simultaneous administration can be performed by any suitable route, and it is preferable to administrate the therapeutic agents to the subject in need, for example, by oral route, intravenous route, intramuscular route, or subcutaneous injection, such that the respective therapeutic agents can be administrated in a fixed ratio.

The more or less simultaneous or sequential administration of each therapeutic agent can be performed by any suitable route, including, but not limited to, oral route, intravenous route, intramuscular route, and absorption through mucosal tissue. The therapeutic agents can be administered by the same route or by different routes. For example, the combined therapeutic agents can be administered via the oral route.

The compound of the present disclosure can be included in a pharmaceutical composition. The pharmaceutical composition includes the compound described in the present disclosure or a pharmaceutically acceptable salt thereof as an active ingredient, and a pharmaceutically acceptable carrier; and optionally other therapeutic ingredients or adjuvants.

The composition includes compositions suitable for oral, rectal, topical, and parenteral (including subcutaneous, intramuscular, and intravenous) administration, although in a given case, the most suitable route depends on the specific host, and the properties and severity of the pathology of the host administrated with the active ingredient. Conveniently, the pharmaceutical composition can be presented in unit dosage form and prepared by using any method well known in the pharmaceutical field.

Creams, ointments, jellies, solutions, or suspensions containing the compound can be applied for topical use. For the purposes of the present disclosure, oral lotions and mouthwashes are included within the scope of topical use.

The pharmaceutical compositions suitable for parenteral administration can be prepared as solutions or suspensions of the active ingredients in water. A suitable surfactant may be included, such as hydroxypropyl cellulose. It is also possible to prepare dispersions in glycerin, liquid polyethylene glycol and mixtures thereof (in oil). In addition, preservatives may be included to prevent growth of harmful microorganisms.

The pharmaceutical compositions suitable for injection use include sterile aqueous solutions or dispersions. The compositions may be in a form of sterile powder, for an immediate preparation of such sterile injectable solutions or dispersions. In all cases, the final injectable form must be sterile and must be effectively fluid to allow it to be easily injected.

The pharmaceutical composition must be stable under the conditions of production and storage. Therefore, it should preferably be protected against a contaminating action of microorganisms such as bacteria and fungi. The carrier can be a solvent or dispersion medium, including, for example, water, ethanol, polyols (e.g., glycerin, propylene glycol, and liquid polyethylene glycol), vegetable oils, and suitable mixtures thereof.

The pharmaceutical composition may be in a form suitable for topical use, such as aerosols, creams, ointments, lotions, powders, etc. In addition, the composition can be in a form suitable for use in a transdermal device, and these preparations can be prepared by a conventional processing method using the compound or the pharmaceutically acceptable salt thereof. As an example, a cream or a ointment is prepared by mixing a hydrophilic material with water and about 5wt% to about 10wt% of the compound, thereby producing the cream or ointment with a desired consistency.

The present disclosure provides a method for treating pancreatic cancer of a patient in need of such a treatment, and the method includes co-administering to the patient a therapeutically effective amount of at least one compound represented by formula (I), or a pharmaceutically acceptable salt or solvate of the compound.

A dosage of the compound of the present disclosure depends on various factors, including the specific disease to be treated, the severity of symptoms, the route of administration, the frequency of the dose interval, the specific compound used, the efficacy of the compound, the toxicological characteristics, and the pharmacokinetics characteristics.

An amount of the active ingredient, which can be combined with a carrier material to produce a single dosage form, shall vary depending on the host to be treated and the specific route of administration. For example, formulations intended for oral administration to humans can conveniently contain about 0.5mg to about 5g of the active ingredient, which is compounded with a suitable and convenient amount of a carrier material (which may account for about 5% to about 95% of the total composition). The unit dosage forms can generally contain about 1 mg to about 1000 mg of the active ingredient, usually 25mg, 50mg, 100mg, 200mg, 300mg, 400mg, 500mg, 600mg, 800mg, or 1000mg.

For any particular patient, the specific dosage level shall depend on a series of factors, including age, weight, general health, gender, diet, timing of administration, route of administration, excretion rate, drug combination, and severity of the specific disease to be treated.

### Use of compound or pharmaceutical composition in preparing medicine

In a third aspect of the present disclosure, the present disclosure provides a use of the compound as described above or the pharmaceutical composition as described above in a preparation of a medicine for treating or preventing cancer. The active ingredient in the compound or the pharmaceutical composition according to the embodiments of the present disclosure can be effectively in vivo degraded into the tumor stroma-regulating group and the cytotoxic group drugs, the tumor stroma-regulating group can effectively inhibit the growth of tumor stroma, and the cytotoxic group exerts a cytotoxic effect to kill the tumor cells effectively. Therefore, the compound or pharmaceutical composition according to the embodiments of the present disclosure can be used to effectively treat or prevent cancer.

According to the embodiments of the present disclosure, the cancer is pancreatic cancer, liver cancer, breast cancer, skin cancer, prostate cancer, or fibroblastoma. The above-mentioned cancers have a large amount of tumor stroma in a certain course of disease. These cancers have activated stromal fibroblasts and thus have a complex tumor microenvironment similar to pancreatic cancer (lack of blood vessels, insensitivity or resistance to chemotherapy drugs, immunity inhibition, etc.), and the tumor cells and the stromal fibroblasts also have similar interactions. The compound according to the embodiments of the present disclosure can regulate the activated stroma fibroblasts of these tumors to reduce a content of stroma in the tumor tissues, allowing more of the second coupling component to enter the tumor cells and to achieve the killing effect on the tumor cells. Specific reports of the tumor microenvironments can be found in the literature (Margareta M. Mueller. Nature reviews, 2004).

Preferably, the cancer is pancreatic cancer. The compound or the pharmaceutical composition according to the embodiments of the present disclosure has more significant therapeutic effect on pancreatic cancer.

An "effective amount" or "effective dosage" of a compound of the present disclosure or a pharmaceutically acceptable composition refers to an effective amount to treat or alleviate the severity of one or more of the disorders mentioned in the present disclosure. According to the method of the present disclosure, the compounds and compositions can be used to effectively treat or alleviate the severity of the disease in any dosage and route of administration. The exact necessary amount may vary depending on the patient's conditions, depending on race, age, patient's general conditions, severity of infection, special factors, route of administration, etc. The compound or composition may be administered in combination with one or more other therapeutic agents, as discussed in the present disclosure.

### Method for preparing compound

In a fourth aspect of the present disclosure, the present disclosure provides a for preparing the compound as described above. According to the embodiments of the present disclosure, the method includes subjecting a first coupling component and a second coupling component to a ligation reaction. The first coupling component is configured to regulate tumor stroma, and the second coupling component is configured to kill tumor cells. The compound obtained by the above method according to the embodiments of the present disclosure can act on the tumor stroma and the tumor cells at the same time, so as to achieve the purpose of eliminating or reducing the tumor stroma while killing tumor cells. The compound obtained by the above method according to the embodiments of the present disclosure has the improved pharmacokinetic properties, synchronizing the pharmacokinetics of the tumor stroma-regulating group and the cytotoxic group, so as to have a good synergistic effect on the production of stroma and the inhibition of cancer cells, thereby improving the drug resistance environment of the tumor, significantly improving the effective killing of tumor cells, as well as greatly reducing the toxic and side effects of the medicines. According to the embodiments of the present disclosure, the ligation reaction is performed in the presence of a linker. The type of linker is not particularly limited, as long as it can successfully perform the ligation reaction of the first coupling component and the second coupling component and can be enzymatically hydrolyzed in vivo.

According to a specific embodiment of the present disclosure, the ligation reaction is performed under the condition that the first coupling component, the second coupling component and the linker coexist, and the linker is at least one of the compounds represented by formula (88) to formula (97):

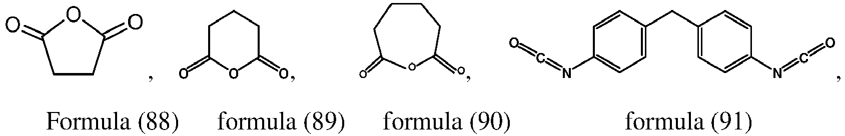

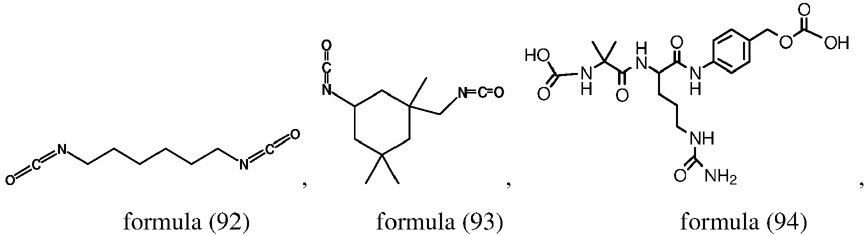

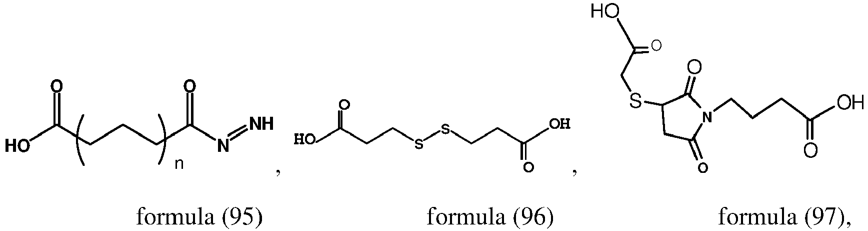

in which, n is 1 to 10. The compounds represented by formula (88) to formula (97) can be used as effective linkers to chemically couple the first coupling component with the second coupling component to form the above-mentioned stable coupling compound. According to yet another specific embodiment of the present disclosure, the first coupling component is Cal, and the second coupling component is TP. The compound represented by formula (A) is TP, and the compound represented by formula (B) is Cal:

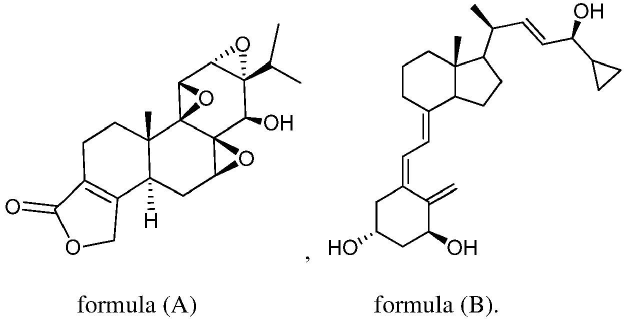

The ligation reaction adopting the compound represented by formula (88) as a linker has a high efficiency.

According to the embodiments of the present disclosure, a mass ratio of the compound represented by formula (A) and the compound represented by formula (B) is 1: 1. In this way, TP has a significant inhibitory effect on tumor cells, Cal has a significant inhibitory effect on the stroma formation of pancreatic cancer, and thus they have significant synergistic therapeutic effect.

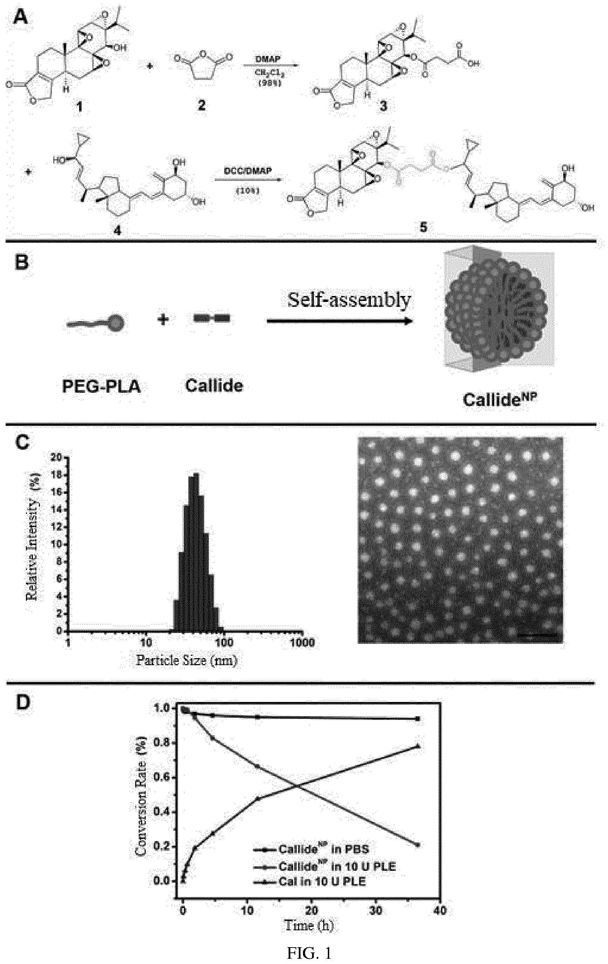

According to a specific embodiment of the present disclosure, the method for preparing the above-mentioned compound is shown in FIG. 1A and includes the following steps:
Step 1: Synthesis of TP-COOH: succinic anhydride (630mg, 6.3mmol), dimethylaminopyridine (DMAP, 765mg, 6.3mmol), and triethylamine (TEA, 783µL, 0.98mmol) were added to a solution of triptolide (324mg, 0.90mmol) in dichloromethane (CH₂Cl₂). The compound solution was stirred at room temperature for 24 hours, and the progress of the reaction was detected by column chromatography. After the reaction was completed, it was washed with dichloromethane and brine successively, and the organic phase was collected. The organic phase was dried with sodium sulfate, filtered, and distilled. The obtained crude product was purified with a silica gel column, using dichloromethane: methanol (100: 1) as the mobile phase, to obtain 380mg of white TP-COOH, with a yield of about 92%.
Step 2: Calcipotriol (Cal, 57mg, 0.14mmol), DMAP (84.3mg, 0.69mmol), and dicyclohexylcarbodicarbonate (DCC, 143mg, 0.69 mmol) were added to a solution of TP-COOH (64mg, 0.14mmol) in dichloromethane. The mixture solution was stirred at room temperature for 24 hours, and the progress of the reaction was detected by column chromatography. After the reaction, the mixture solution was diluted with an appropriate amount of dichloromethane, washed with 0.1M dilute hydrochloric acid solution and brine successively, and the organic phase was collected. The organic phase was dried with sodium sulfate, filtered, and distilled. The obtained crude product was purified with a silica gel column, using dichloromethane: methanol (100: 1) as the mobile phase, to obtain a white Callide solid 10mg, with a yield of about 15%.

In yet another aspect of the present disclosure, the present disclosure also provides a method for preparing micelles. According to the embodiments of the present disclosure, FIG. 1B is a schematic diagram of a synthesis method. The method includes the following steps:

1mg of Callide and 19mg of polyethylene glycol-polylactic acid block copolymer (PEG-PLA) were weighed, dissolved with 5ml acetonitrile in a 50ml round bottom flask, and sonicated for lmin to allow them to be fully dissolved. The solvent was spin-evaporated at 60 degrees Celsius in a water bath to obtain a transparent film. After the solvent was completely evaporated, the spin-evaporation continued for 30 minutes to remove the residual organic solvent. 5ml of pre-warmed physiological saline at 60 degrees Celsius was added to the roundbottom flask and hydrated by ultrasound, to obtain polymer micelles coating the drug, which is named as Callide^{NP}. A particle size of the polymer micelle solution was analyzed with a dynamic light scattering instrument, and an appearance thereof was analyzed with a transmission electron microscope. The results are shown in FIG. 1C.

Applicant has found that the method of the present disclosure can quickly and efficiently prepare and obtain the micelles, and the operation is simple and easy to be controlled, and has no special requirements for equipment, such that the method is suitable for large-scale production. In addition, TP is significantly aggregated, which can effectively inhibit TFIIAH, and Cal can effectively inhibit the formation of pancreatic cancer stroma, a half-life and a therapeutic window of the compound are significantly improved compared to TP, such that the compound has a more significant therapeutic effect on pancreatic cancer, and the toxic and side effects of the compound are reduced significantly. The micelles prepared by the method according to the embodiments of the present disclosure have good stability and can be effectively used for treating or preventing cancer, especially pancreatic cancer.

The embodiments of the present disclosure are described in detail below. The embodiments described below are illustrative and merely intended to explain the present disclosure, and they cannot be construed as limitations of the present disclosure. Technology or conditions that are not specifically indicated in the embodiments are those described in the literatures in the related art or in accordance with the product specification. All the reagents or instruments used without indicating the manufacturer are the conventional products that are commercially available.

### Example 1: In Vitro Degradation Experiment of Compound Callide

Experimental materials: phosphate buffer (PBS, Ph = 7.2 to 7.4), porcine liver esterase, high performance liquid chromatography (HPLC)

Experimental method: HPLC method

Experimental process: 5 microliters of 1mg/ml Callide (ethanol solution for solubilizing) in 5 ml of PBS buffer with or without porcine liver esterase, and stirred at 37 degrees Celsius for 1 min, 5 min, 10 min, 30 min, 1 h, 2 h, 4 h, 8 h, 12 h, 24 h, 48 h, respectively, 200 microliters of the solution were taken, filtered, and a concentration of Callide or calcipotriol in the solution was determined by HPLC.

Results: In the absence of porcine liver esterase, Callide could not be degraded, even if the time was extended to 48h, the concentration of Callide in the solution remained unchanged; while in the presence of porcine liver esterase, Callide was able to be degraded into a single calcipotriol, and the concentration of Callide decreased and the concentration of calcipotriol increased with time (experiment results are shown in FIG. 1D).

Conclusion: The new compound Callide synthesized in this study can be successfully degraded under the catalytic action of esterase.

### Example 2: Cytotoxicity Experiment of Callide

Experimental materials: four pancreatic cancer cell lines, i.e., MIA PaCa-2, PANC-1, SW1990, and SW1990-GEM. Whole cell culture medium, microplate reader

Experimental method: DNA assay method

Experimental process: pancreatic cancer cells were cultured, digested, resuspended into a cell suspension with medium, and seeded into 96-well plates, with 4000 cells per well. The experimental components are 5 groups, i.e., TP group, Cal group, TP and Cal physical mixed group (TP/Cal), Callide-DMSO without esterase group (Callide without PLE), Callide-DMSO with 10U esterase group (Callide with 10U PLE), Callide^{NP} without esterase group (Callide^{NP} without PLE), and Callide^{NP} with 10U esterase group (Callide^{NP} with 10U PLE). For each group, 8 concentrations of 5nM, 10nM, 20nM, 40nM, 80nM, 100nM, 200nM, and 400nM were prepared, and each concentration was repeated in six wells. After 24h of administration, the drug solution was removed by suction, washed with PBS twice, added Husted dye, analyzed by a microplate reader, and plotted a relative survival rate curve of cells to calculate an IC50 of each compound.

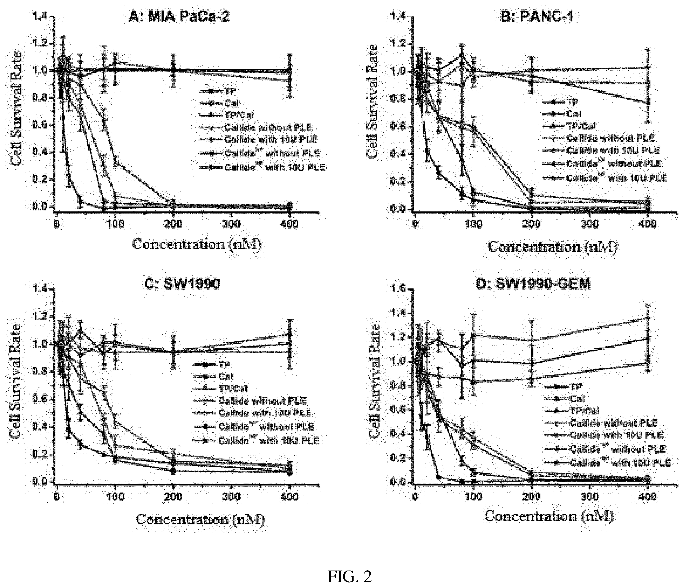

Results: as a traditional cytotoxic drug, TP has a high killing effect on all four types of pancreatic cancer cells, with IC₅₀ = 20nM. Cal, as a drug for clinical treatment of psoriasis, has no killing effect on cancer cells within the dosage range studied by Applicant. The compound Callide synthesized in the present disclosure had no toxic and side effects on cells without the addition of esterase. However, under the catalytic effect of porcine liver esterase, Callide can be degraded into TP (or TP-COOH) to produce cell killing effect. Compared to the pure compound Callide, a formulation of Callide micelles had similar cytotoxic effects. Callide^{NP} also did not produce any toxic and side effects on cells without the addition of esterase. However, under the catalytic effect of porcine liver esterase, Callide in Callide^{NP} can be degraded into TP (or TP-COOH) to produce cell killing effect and IC50 was increased by 4-7 times with respect to TP (experiment results are shown In FIG. 2).

Conclusion: Callide and Callide^{NP} prepared in the present disclosure can be successfully degraded under the catalytic degradation with esterase, and thus turn into an effective component to kill cancer cells. However, the micelles protect Callide from being degraded, so as to inhibit the release and degradation of Callide, such that Callide^{NP} has an increased IC50 value and a decreased cytotoxicity.

### Example 3: Rat PK Experiment of Callide^{NP}

Experimental materials: rats, strain SD, 6-9 weeks old. Analytical instruments: Agilent 1290/6460 triple quadrupole mass spectrometer, made in the United States.

Experimental method: chromatography-mass spectrometry

Experimental process: 12 SD rats were randomly divided into 4 groups, with 3 rats in each group. TP (0.3mg/kg), TP/Cal (0.3/0.35mg/kg), Callide^{NP} (0.3mg/kg), Callide^{NP} (0.6mg/kg) were administered respectively. 300µl of blood of each rat was collected through the orbit at 5 min, 15 min, 30 min, 1 h, 2 h, 4 h, and 8 h, respectively, centrifuged at 10000 rpm at 4°C, and the supernatant plasma was collected. The plasma was extracted with ethyl acetate/water, vortexed, sonicated, centrifuged at 4°C, the supernatant was evaporated to dryness under the protection of nitrogen, and then dissolved in mobile phase acetonitrile/water. After centrifugation, the supernatant was taken and injected.

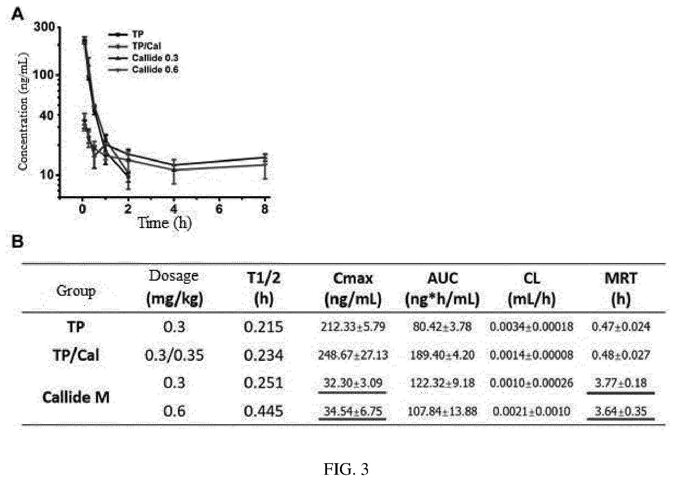

Results: TP, as a free drug, has a weak protein-binding ability in plasma. Through analysis and measurement, the concentration of free TP in plasma was high and the half-life was short, which is one of the reasons for the serious toxicity of TP in vivo. The physical mixing of TP and Cal failed to improve the pharmacokinetic parameters of TP, which is reason why the physical mixing of TP and Cal has the comparable toxicity to the free TP. Callide, as a prodrug of TP, produced a significantly reduced concentration of free TP in plasma, and has a prolonged plasma half-life and a prolonged average plasma residence time (experiment results are shown in FIG. 3).

Conclusion: Callide, as a prodrug of TP, can have significantly improved pharmacokinetic properties in vivo by delivering through polymer micelles, thereby reducing the serious side effects of the free TP, and increasing the anti-cancer effect of TP.

### Example 4: Toxicity Experiment of Callide^{NP} on Mice in Short- and Long-term Administration

Experimental material: BALB/C mice

Experimental method: BALB/C mice were divided into 5 groups, including a saline control group and four drug administration groups of TP, Cal, TP/Cal, and Callide^{NP} groups. 5 mice in the control group, and 5 mice for each of 3 dosages, i.e., high, medium and low dosages in each experimental group, for a total of 65 mice. Dosages of administration: TP (0.3mg/kg, 0.6mg/kg, 1.2mg/kg); Cal (0.6mg/kg, 1.2mg/kg, 1.8mg/kg); TP/Cal (0.6mg/kg, 1.2mg/kg, 1.8mg/kg); and Callide^{NP} (0.6mg/kg, 1.2mg/kg, 1.8mg/kg), were administered once a week via tail vein injection.

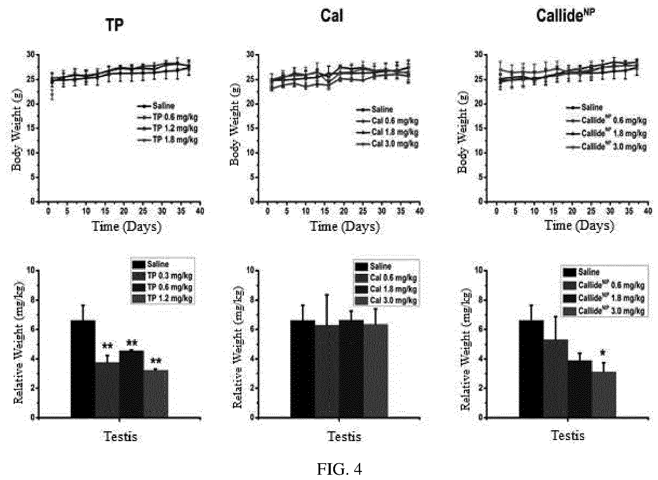

Results: The mice in the TP group were given 1.8 mg/kg in advance, and all mice died after a single administration. For this reason, the high, medium and low dosages of TP were designed as the above dosages. Even so, the mice in the TP group showed obvious toxic reactions. After the administration, the mice were sluggish and lethargic, especially the dosage of 1.2 mg/kg of TP. In addition to the mice 's poor mental status, severe skin ulceration appeared at the injection site on the tail of mice, and the mice lost their weight significantly. Anatomical results showed that the organs of the mice all had different degrees of necrosis, especially the testis. The relative weight of the testis was significantly reduced, and the morphology of spermatogonia and spermatocytes was severely damaged. The mice in the TP/Cal group had the similar states as described above, and the toxic and side effects caused by free TP had very adverse effects on the health of the mice. In contrast, none of the mice in the Cal group showed the above-mentioned adverse symptoms, and all the mice were in good health. In the Callide^{NP} group, the above-mentioned adverse states were significantly alleviated after the administration, except the mice in the high-dosage experimental group had poor mental state, but there was no significant change in body weight and various organs compared with the control group (experiment results are shown in FIG. 4).

Conclusion: compared with TP, the long-term toxicity of Callide is significantly reduced, which is the premise for Applicant to further verify the anti-tumor effect of Callide.

### Example 5: In Vitro Effect of Synergistic Effect of Callide^{NP} on Pancreatic Stellate Cells and Pancreatic Cancer Cells

Experimental materials: pancreatic stellate cells and pancreatic cancer cells cultured in transgenic mice. Among them, pancreatic cancer cells were transfected with luciferase gene by lentivirus transfection method.

Experimental method: in vitro co-culture, immunofluorescence histochemical analysis

Experimental process: the tumor tissues were taken from KPC mice that spontaneously developed with tumors, and the tumor tissues were dissected, and primary pancreatic stellate cells and pancreatic cancer cells were isolated by physical in vitro disruption and enzymatic hydrolysis, and cultured.

Pancreatic stellate cells alone were cultured in a 12-well plate, 100,000 cells per well, treated with TP (10nM), Cal (100nM), TP/Cal (10/100nM), Callide^{NP} (100nM) for 24 hours, and then contents of smooth actin and collagen of the cells in each well were analyzed with immunofluorescence staining.

Pancreatic stellate cells and pancreatic cancer cells were cultured in a 12-well plate at a ratio of 1: 1, with a total of 100,000 cells per well, using TP (10nM), Cal (100nM), treated with TP/Cal (10/100nM), and Callide^{NP} (100nM), and incubated for 24 hours, and then the contents of smooth actin and collagen of the cells in each well were analyzed with immunofluorescence staining.

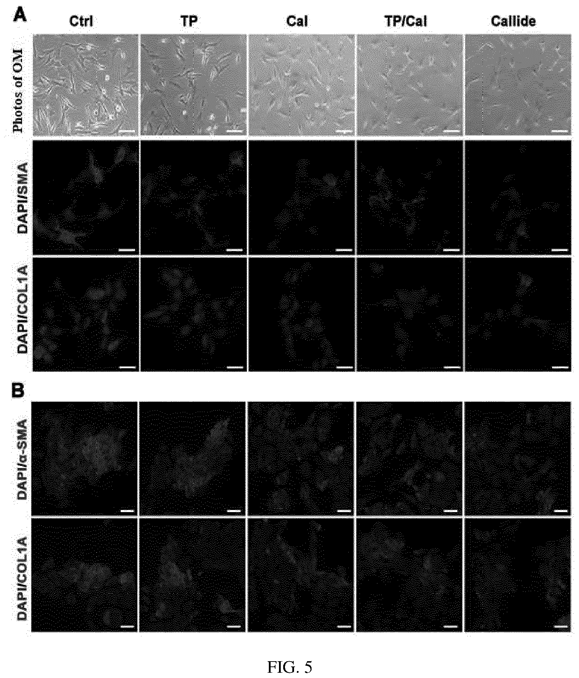

Results: for pancreatic stellate cells alone, the contents of smooth actin and collagen in TP-treated cells were not significantly different from those in the control cells; after Cal treatment, the expression levels of related proteins in the cells decreased, and the fluorescence intensity significantly decreased; TP/Cal and Callide^{NP} had the same trend. For the co-cultured pancreatic stellate cells and pancreatic cancer cells, compared with the pancreatic stellate cells alone, in the control group, the expression levels of related proteins between them increased significantly, and the expression levels of related proteins did not change significantly after TP treatment, while the expression levels of related proteins in the cells treated with Cal, TP/Cal and Callide^{NP} were significantly reduced (experiment results are shown in FIG. 5).

Conclusion: Callide can produce Cal under the catalytic degradation of esterase, such that the contents of smooth actin and collagen of cells can be reduced under the action of PSC activated by Cal reproducing, while the interaction between pancreatic stellate cells and pancreatic cancer cells can be weakened.

### Example 6: In Vivo Anti-tumor Effect Experiment of Callide^{NP} Based on Pancreatic Stellate Cells and Pancreatic Cancer Cells Co-implanted Orthotopic Model

Experimental materials: pancreatic stellate cells, pancreatic cancer cells transfected with luciferase; nude mice: strain Blab/C nude, 6-8 weeks old.

Experimental method: suspensions of pancreatic stellate cells and luciferase-transfected pancreatic cancer cells were mixed uniformly at a ratio of 1: 1, and inoculated into the pancreas at a density of 10,000 cells/40µL with an inoculation volume of 40µL.

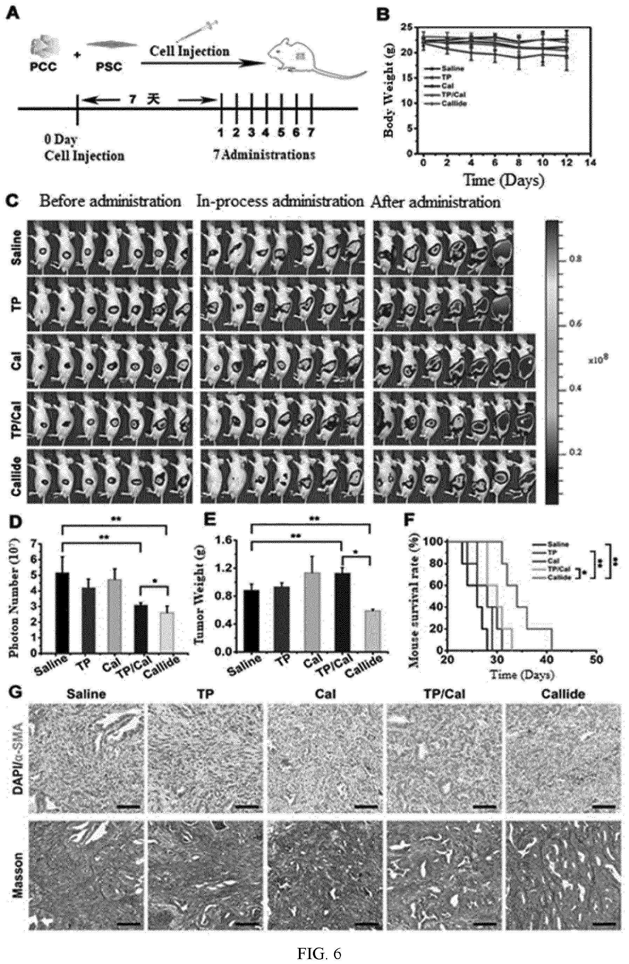

Experimental process: the two kinds of cells were digested and centrifuged, resuspended with PBS and mixed at a ratio of 1: 1, and then the cell mixture was mixed with Matrigel at a ratio of 1: 1 and pipetted uniformly. Nude mice underwent relevant operations under the premise of review by the Ethics Committee of Tsinghua University. 40µL of the uniformly pipetted cell suspension was injected into the pancreas with an insulin needle, the wound was sutured, and a sedative was injected subcutaneously. About one week after the cell injection, each mouse was intraperitoneally injected with 0.1ml of fluorescein sodium salt solution. After 10 min, the mice were anesthetized with isoflurane, and an intensity of the fluorescent signal in the pancreas of the mice was detected with a live imaging system for small animal. Subsequently, the mice were randomly divided into groups, 7 mice in each group, and TP (0.3mg/kg), Cal (0.35mg/kg), TP/Cal (0.3/0.35mg/kg), and Callide^{NP} (0.6 mg/kg) were administrated, respectively, once every two days for a total of 7 times. After each administration, the mice were weighed. After 4 times of administration, the intensity of the fluorescent signal in the pancreas of mice was detected with the live imaging system for small animal. After the administrations were all finished, the intensity of the fluorescent signal in the pancreas of the mice was detected again with the live imaging system for small animal, while recording a survival rate of mice. After the experiment, the tumor tissues of the mice were taken, and an expression of smooth actin and collagen in the tumor tissues was detected by tissue immunofluorescence staining (the experiment results are shown in FIG. 6).

Results: compared with the control group, the TP group had an insignificant tumor suppression effect, but an obvious toxicity. After seven administrations, the body weight of the mice was significantly reduced, and one mouse died; the Cal group had no tumor suppression effect, no significant difference in the fluorescent signal between the pancreatic cancer cells and the control group, no significant difference in the survival rate of the mice; in the TP/Cal group, the tumor cells had reduced fluorescent signal, but no significant difference in the change of tumor weight, the tumor suppression effect and the contribution to the survival rate of the mice were limited; in the Callide^{NP} group, the fluorescence signal of pancreatic cancer cells was significantly reduced, the survival rate of mice was significantly longer than that of the control group, and the content of smooth actin and collagen was reduced by immunofluorescence staining the tumor tissue, which is directly related to the tumor suppression effect of Callide^{NP}.

Conclusion: by synergistically suppressing pancreatic stellate cells and pancreatic cancer cells, Callide^{NP} can significantly inhibit a tumor size and prolong a survival time of mice in the co-implantation orthotopic model of pancreatic stellate cells and pancreatic cancer cells.

### Example 7: In Vivo Anti-tumor Effect Experiment of Callide^{NP} Based on Pancreatic Cancer MIA PaCa-2 Cell Line

Experimental material: MIA PaCa-2 cell line

Experimental method: establishing a mouse subcutaneous tumor model based on the MIA PaCa-2 cell line, and evaluating an inhibitory effect on tumor as well as toxic and side effect of each compound of the experimental group after administration.

Experimental process: 0.1ml of MIA PaCa-2 cell suspension was injected into axilla of the mice, and the growth of tumor was monitored. When the tumor had a size of about 100mm³, the mice were randomly divided into 5 groups, including a saline control group and four administration groups, i.e., TP, Cal, TP/Cal, Callide^{NP} group, 8 mice in each group. Dosages of administration: TP (0.3mg/kg), Cal (0.3mg/kg), TP/Cal (0.3/0.35mg/kg), Callide^{NP} (0.3mg/kg, administration dosage after it was theoretically completely degraded to TP), administered via tail vein injection once a week. The tumor size was measured every two days.

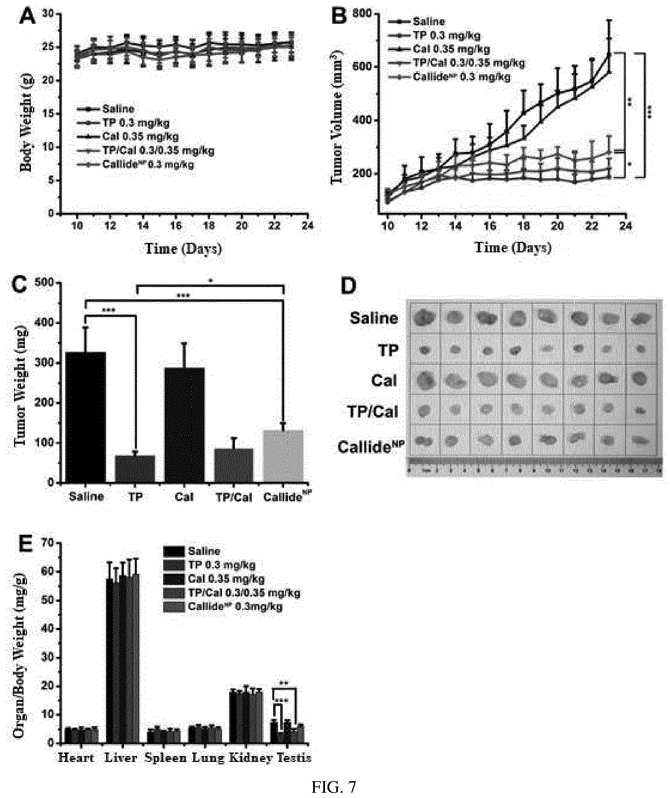

Results: as shown in FIG. 7, compared with the saline group, the Cal group had no anti-tumor effect. All other groups have a very significant effect of inhibiting tumor growth. After the mice were dissected, the tumors were isolated and weighed. The tumors in the TP, TP/Cal, and CallideNP groups had significantly less weight than the control group. It is worth noting that the tumor size of the TP group was smaller than that of the TP/Cal and Callide^{NP} groups, for the reason that TP/Cal is a physical mixture of TP physiological saline solution and Cal micelle preparations, an interaction between the these two reduced the function of TP. Callide^{NP} could only effect when it is degraded into TP. However, due to the uncertain distribution and concentration of esterase in the body, the degradation of Callide^{NP} is not complete, thereby limiting the degradation of Callide^{NP} and the anti-tumor effect of the degraded product TP to a certain extent. (Alternatively, Callide^{NP} changed the PK of TP, and the degraded product could be TP-COOH).

Conclusion: Callide^{NP} can effectively inhibit tumor growth without obvious toxic and side effects in the mouse subcutaneous tumor model based on the MIA PaCa-2 cell line. In addition, as Callide^{NP} cannot be completely degraded to TP, its antitumor effect is slightly weaker than pure TP.

### Example 8: In Vivo Anti-tumor Effect Experiment of Callide^{NP} Based on Humanized Pancreatic Cancer

Experimental material: humanized pancreatic cancer tissue

Experimental method: establishing a mouse subcutaneous tumor model based on humanized pancreatic cancer tissue

Experimental process: a mouse subcutaneous tumor model of humanized pancreatic cancer tissue was established, TP (0.3mg/kg) and Callide^{NP} (0.3mg/kg, administration dosage after it was theoretically completely degraded to TP) were administrated via tail vein injection, once a day. One week later, the dosage of Callide^{NP} was increased to 0.6 mg/kg.

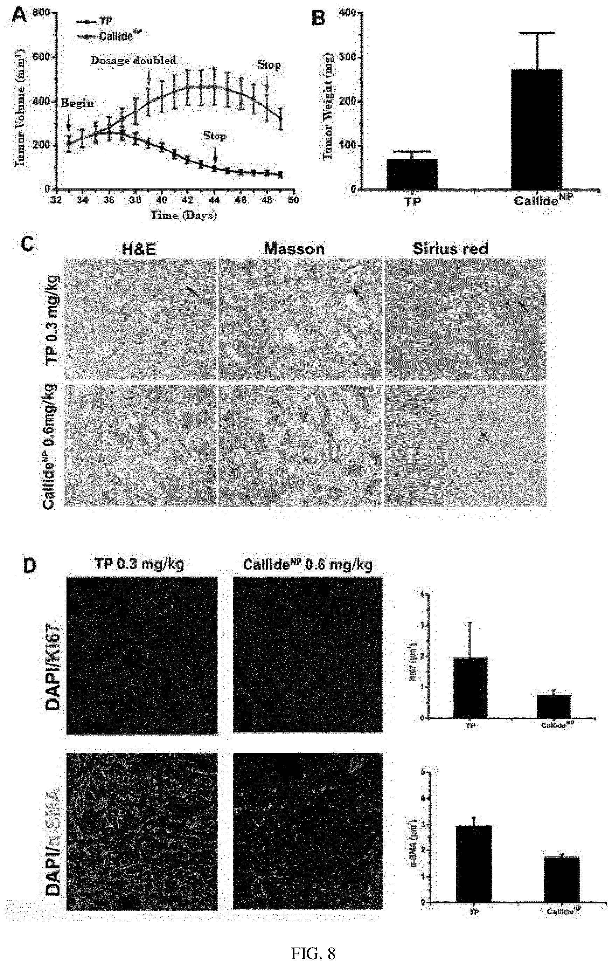

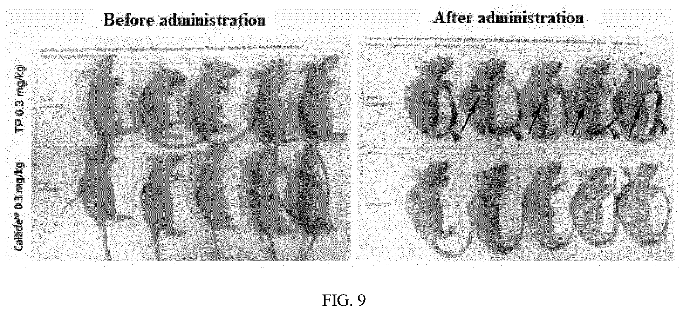

Results: Within the first week of the experiment, TP had a significant inhibitory effect on tumor growth, but Callide^{NP} did not. After the dosage was doubled, Callide^{NP} gradually began to exhibit a better tumor suppressing effect. The reason is in that, when Callide^{NP} was administrated with the dosage of 0.3mg/kg, the speed and degree of Callide degradation into TP in the body could not reach the effective therapeutic concentration of TP. After the dosage was doubled, the advantages of Callide could exhibit, the efficiency of tumor suppression gradually exceeded TP, and a slope of the tumor growth inhibition curve was greater than that of TP. It indicates that Callide^{NP}, under a dosage condition of an appropriate concentration, had a tumor suppression effect comparable to or better than that of TP (as shown in FIG. 5). Moreover, in view of the mental state and skin condition at the tail vein injection site of the mice, Callide^{NP} showed good safety (as shown in FIG. 9). Immunohistochemical staining results of the tumor tissues also indicated that Cal produced by the degradation of Callide had an effect on the tumor stroma, and the fibrin content of the tumor tissue was significantly reduced, accompanied by apoptosis of tumor cells (FIG. 8).

Conclusion: in the humanized mouse subcutaneous transplanted tumor model, Callide^{NP} exhibited good anti-tumor potential, and more preeminent safety than TP alone. The good anti-tumor effect is attributed to a dual mechanism of action, including a weakening effect on the tumor stroma and a killing effect on tumor cells.

In summary, under the catalytic action of porcine liver esterase, the new compound Callide prepared in the present disclosure can be degraded into Cal for regulating the tumor stroma and TP for killing the tumor cells; a long-term administration toxicity in vivo is greatly reduced compared to TP. In different mouse tumor models, Callide^{NP} has shown good anti-tumor effect. The pancreatic cancer can be effectively treated through the dual mechanism of action that acts synergistically on the tumor stroma and tumor cells.

In the description of this specification, the description referring to the term "one embodiment", "some embodiments", "an example", "specific examples", or "some examples" means specific features indicates that the specific features, structures, materials or characteristics described in conjunction with the embodiment or examples shall be included in at least one embodiment or example of the present disclosure. In this specification, the schematic expression of the above terms does not necessarily refer to the same embodiment or example. Moreover, the described specific features, structures, materials, or characteristics may be combined in any one or more embodiments or examples in any suitable manner. In addition, without contradicting each other, those skilled in the art may incorporate and combine different embodiments or examples and features of the different embodiments or examples described in the specification.

Although the embodiments of the present disclosure have been shown and described above, it should be understood that the above-mentioned embodiments are illustrative and shall not be construed as limitations to the present disclosure, and within the scope of the present disclosure, those skilled in the art can make changes, modifications, replacements and variations to the above embodiments.

## Claims

1. A compound, comprising a tumor stroma-regulating group and a cytotoxic group that are coupled to each other, wherein the tumor stroma-regulating group is configured to regulate tumor stroma, and the cytotoxic group is configured to kill tumor cells.

2. The compound according to claim 1, wherein the tumor stroma-regulating group comprises at least one selected from calcipotriol, cyclopamine, Ganciclovir, Fingolimod, all-trans retinoic acid, and hyaluronidase.

3. The compound according to claim 1, wherein the cytotoxic group comprises at least one selected from triptolide, paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, and Lapatinib;
optionally, the compound further comprises an enzyme-degradable linker; and optionally, the enzyme-degradable linker has at least one of the following structures:

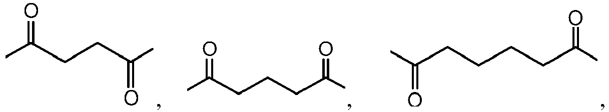

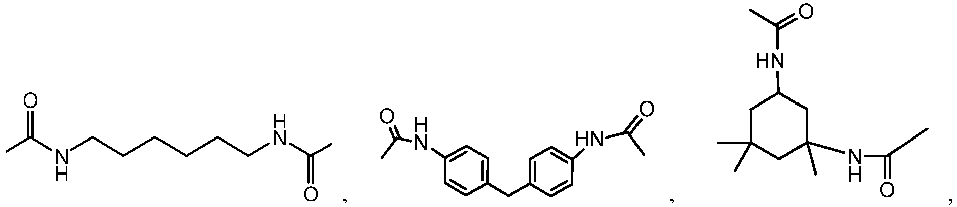

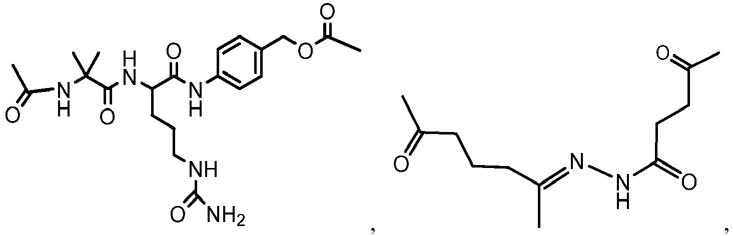

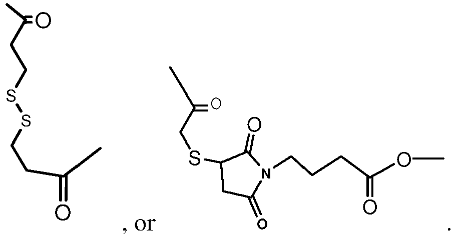

4. A compound, comprising one of the following structures, or comprising one of isomers, stereoisomers, geometric isomers, tautomers, nitrogen oxides, hydrates, solvates, metabolites, pharmaceutically acceptable salts or prodrugs of the following structures: 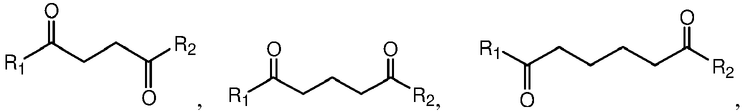 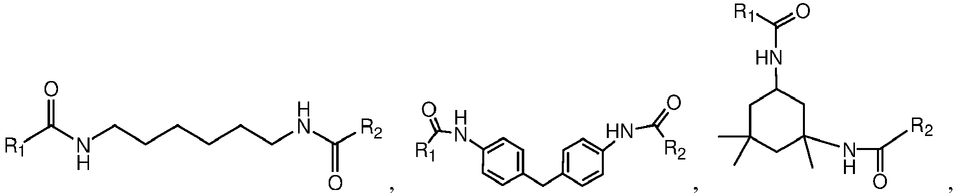 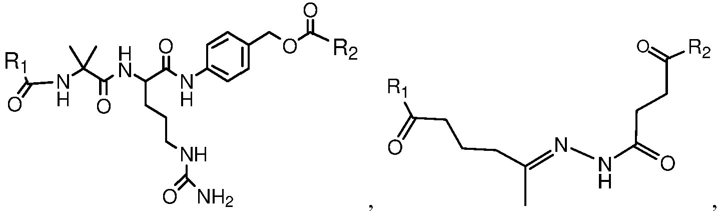 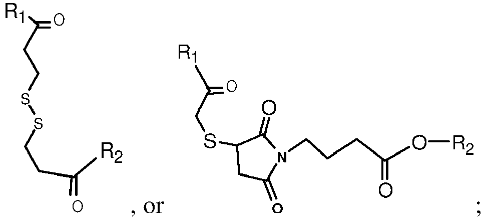 wherein R₁ is independently calcipotriol, cyclopamine, Ganciclovir, Fingolimod, all-trans retinoic acid or hyaluronidase; and
R2 is independently triptolide, paclitaxel, docetaxel, adriamycin, camptothecin, hydroxycamptothecin, 5-fluorouracil, Gemcitabine, Cisplatin, Irinotecan, Oxaliplatin, Pemetrexed, Capecitabine, Epirubicin, Sorafenib, Gefitinib, Erlotinib, Imatinib, Nilotinib, Dasatinib, Everolimus, Sunitinib, Ibrutinib, Crizotinib, Pazopanib, Carfilzomib, Tofacitinib, Axitinib, Regorafenib, Verofenib, Sirolimus, Ponatinib, Levatinib, Olaparib, Ceritinib, Romidepsin, Alectinib, Belinostat, Bosutinib, Vandetanib, Cabozantinib, Afatinib, Trametinib, Dabrafenib, or Lapatinib.

5. The compound according to claim 4, comprising one of the following structures:

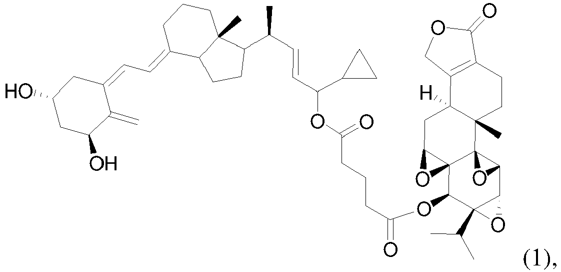

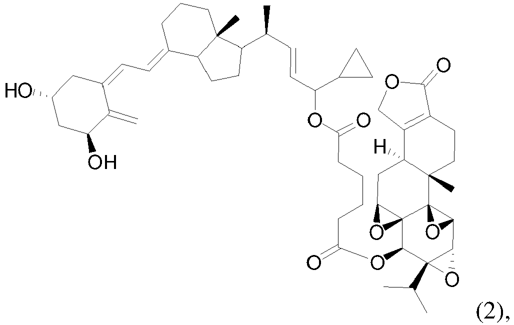

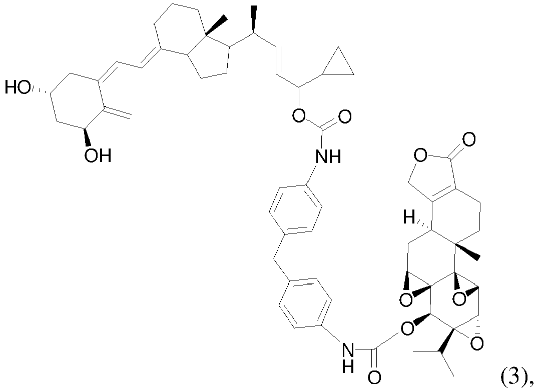

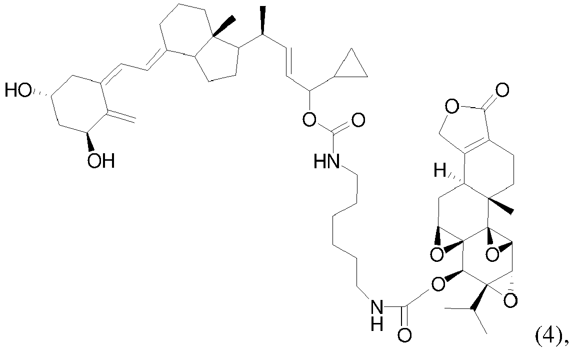

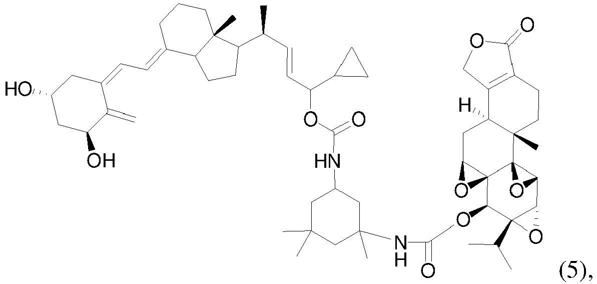

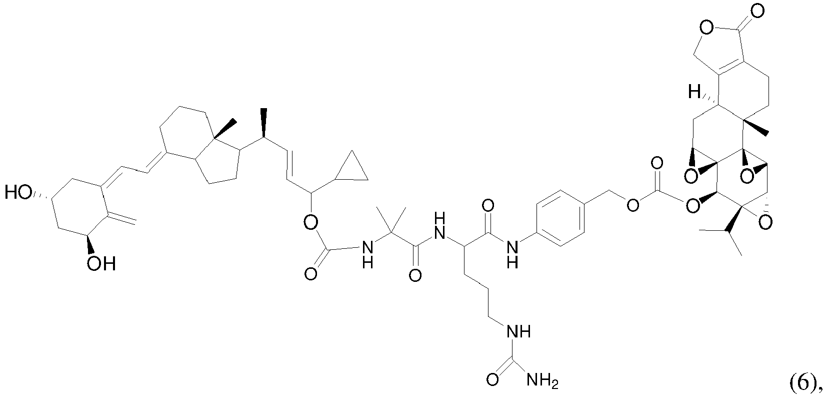

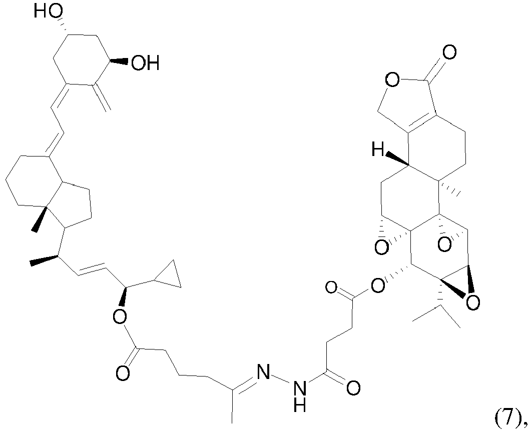

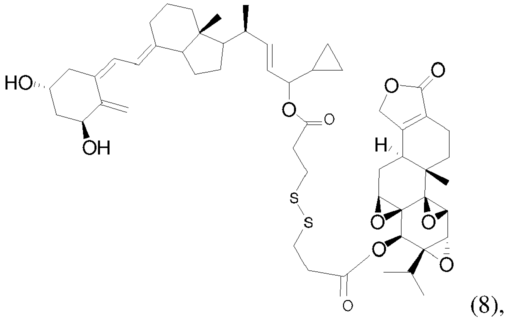

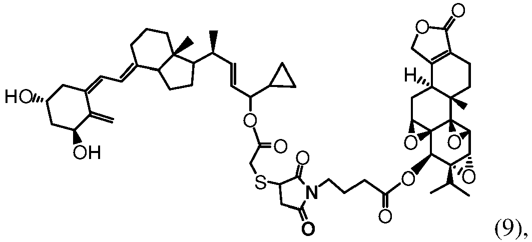

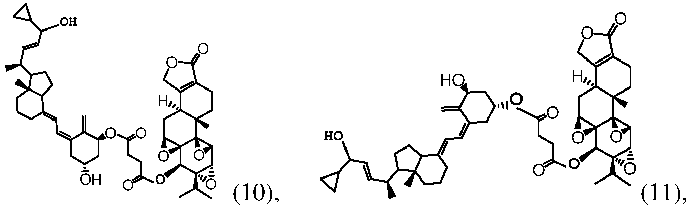

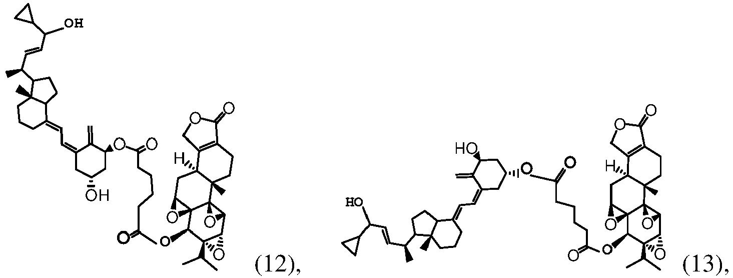

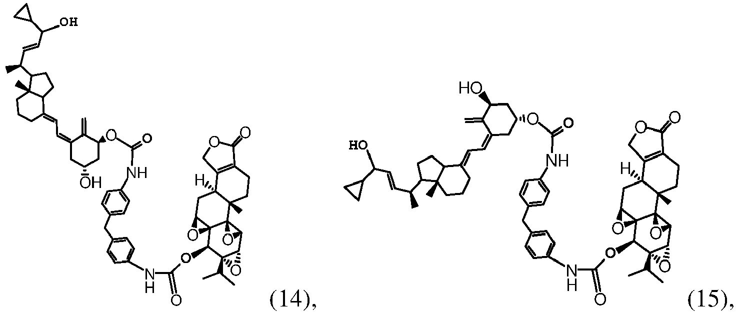

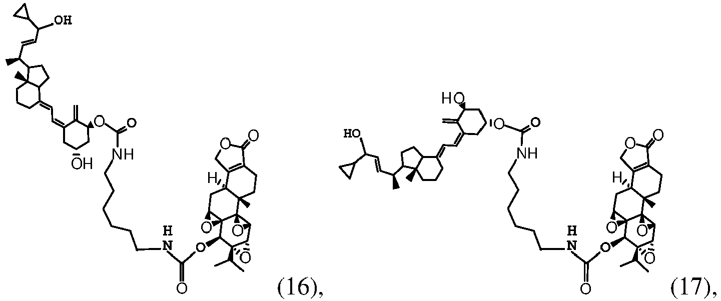

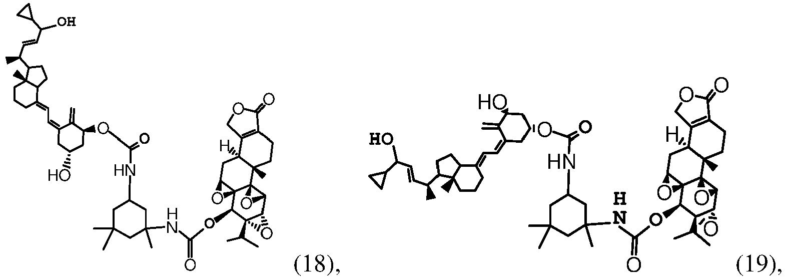

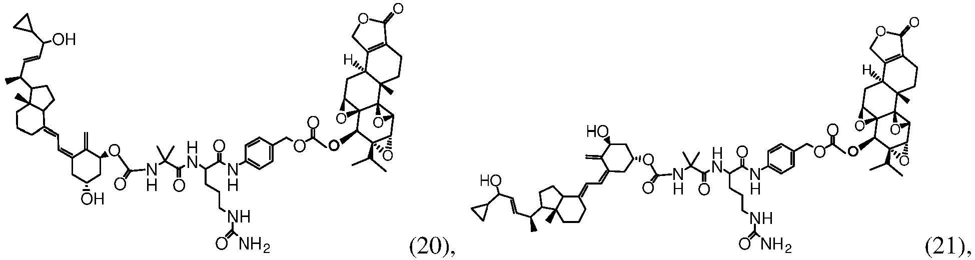

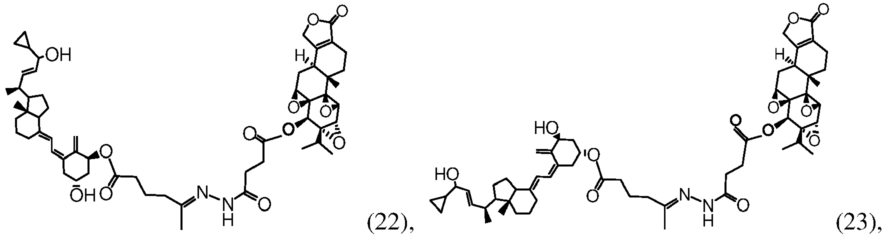

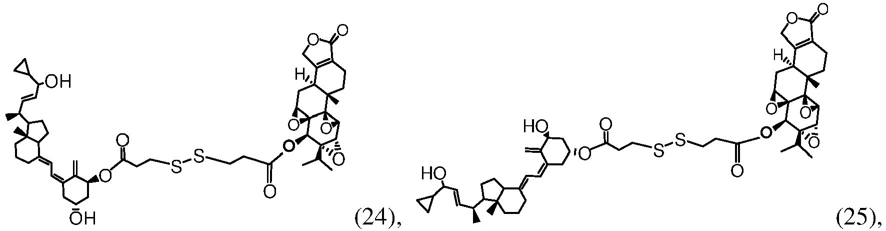

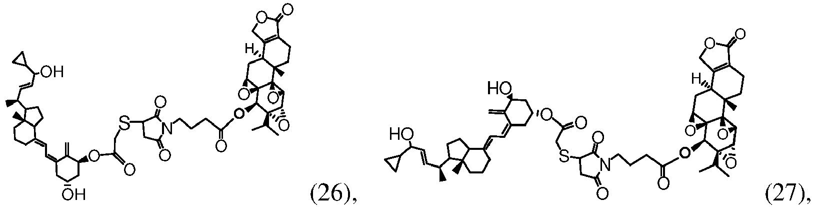

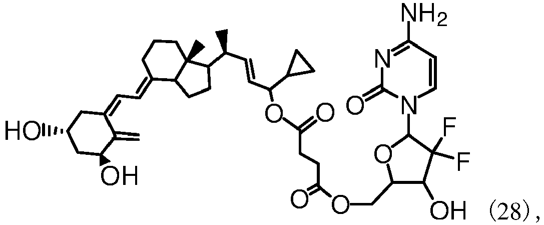

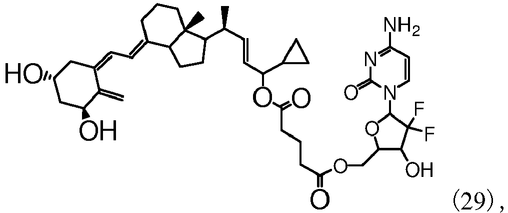

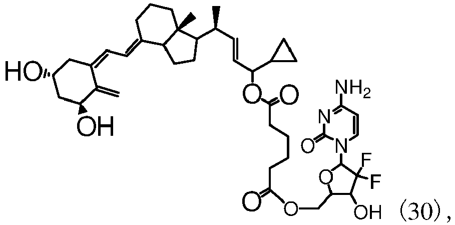

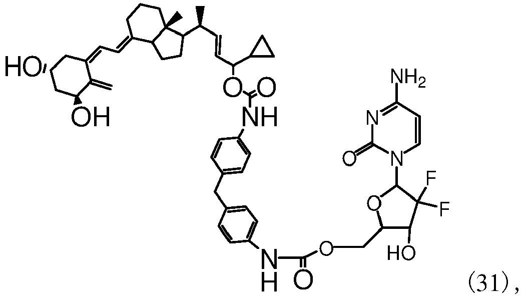

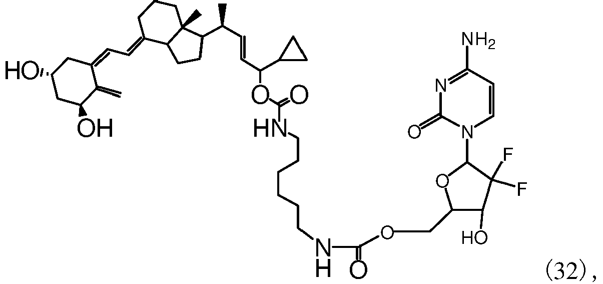

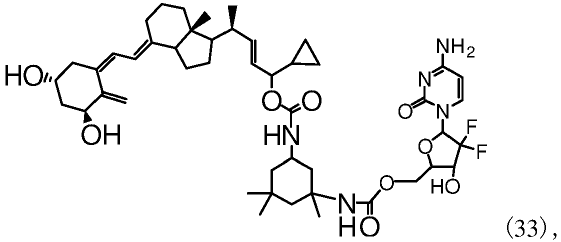

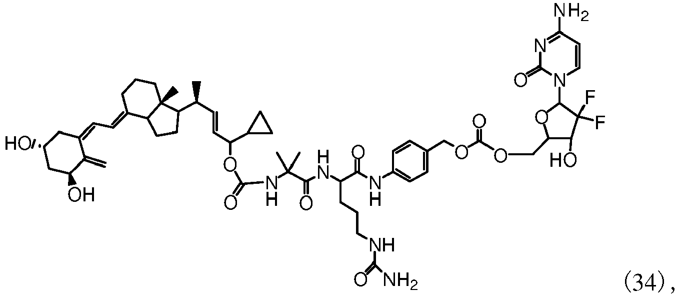

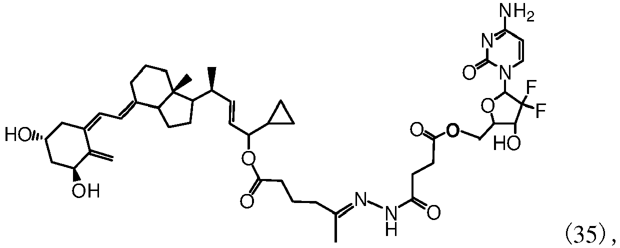

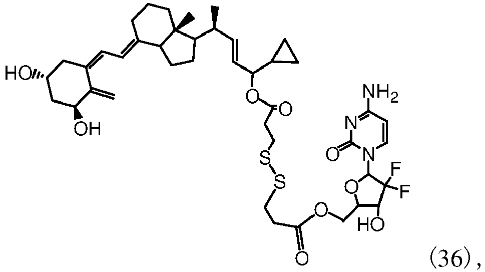

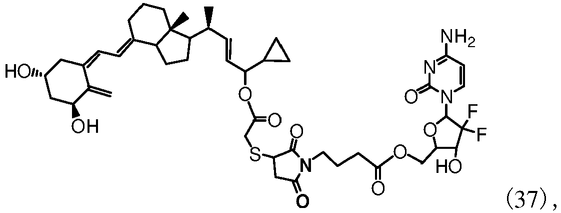

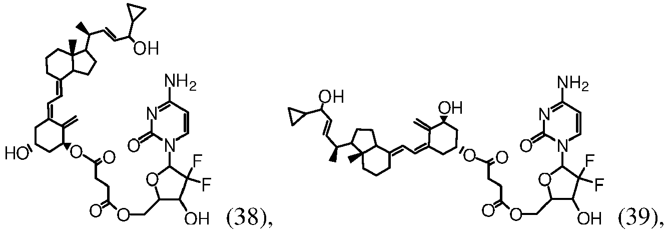

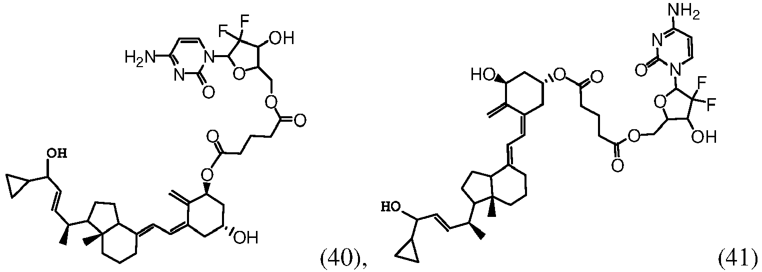

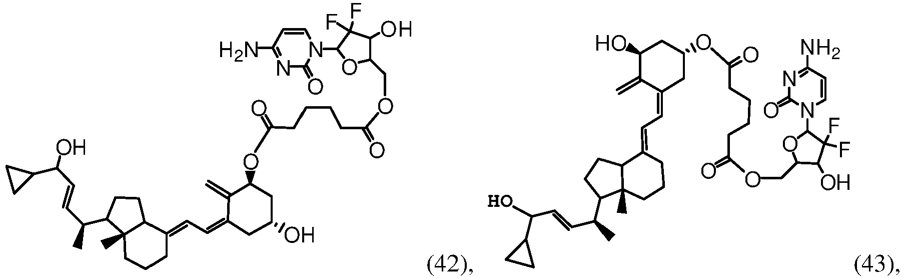

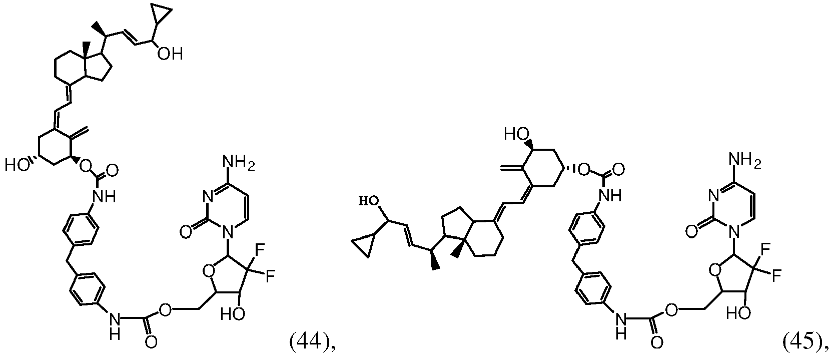

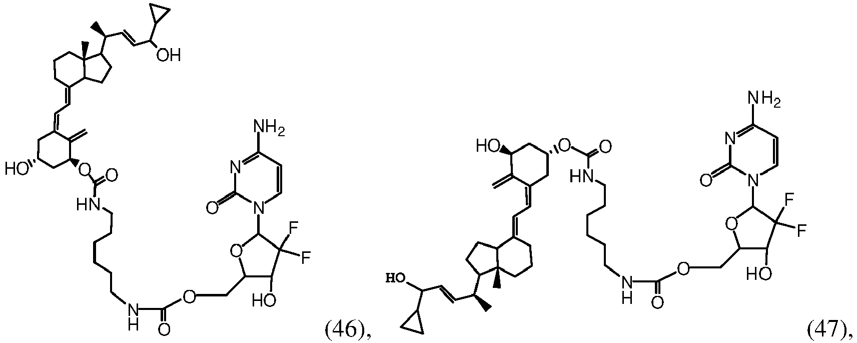

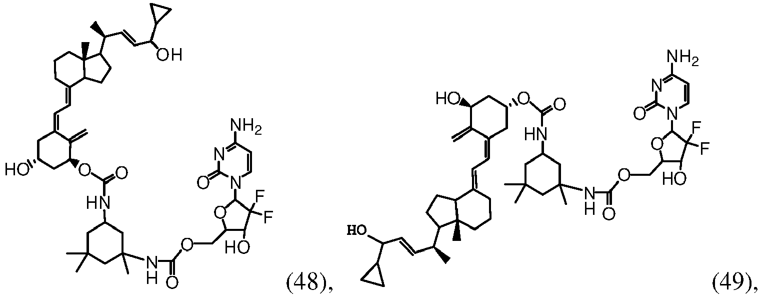

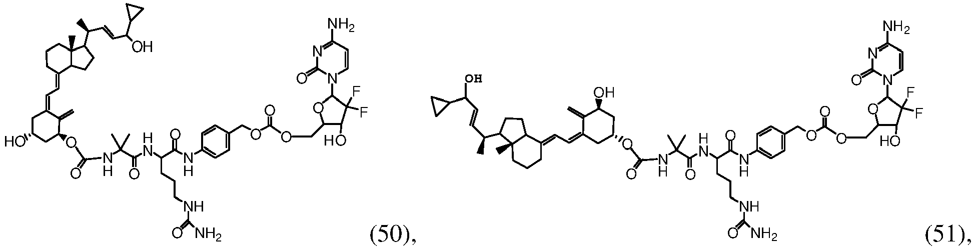

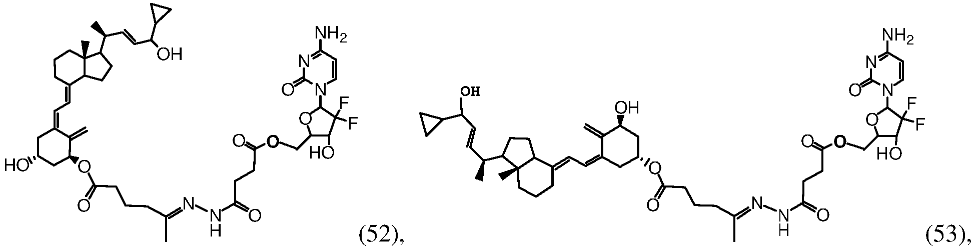

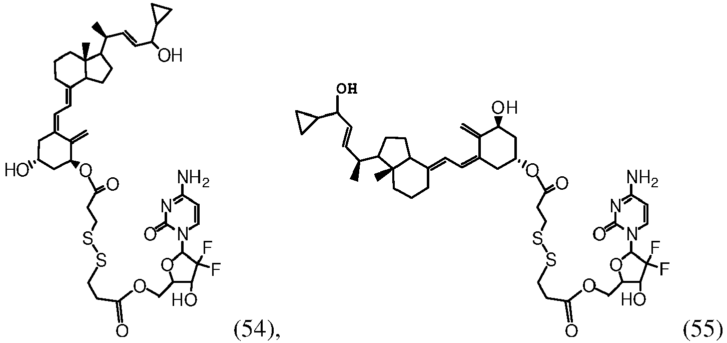

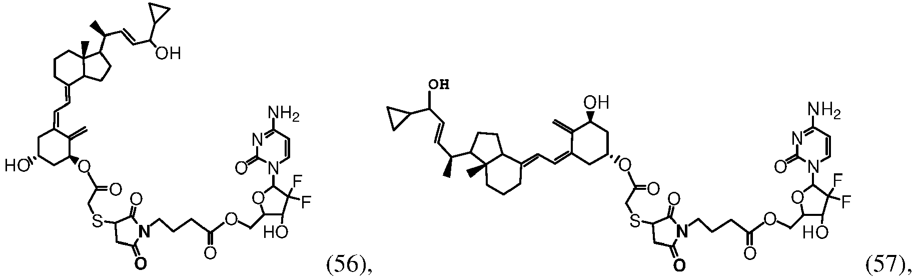

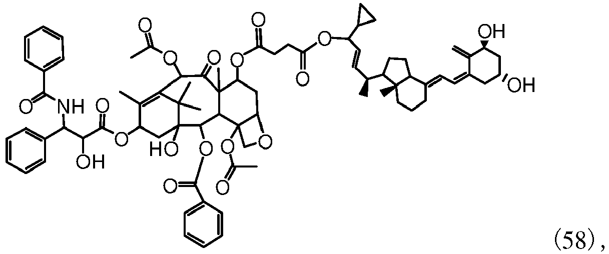

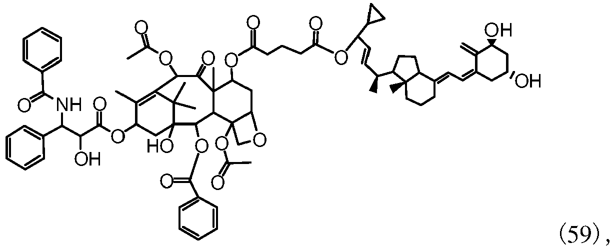

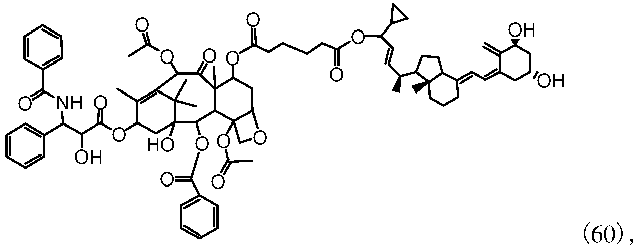

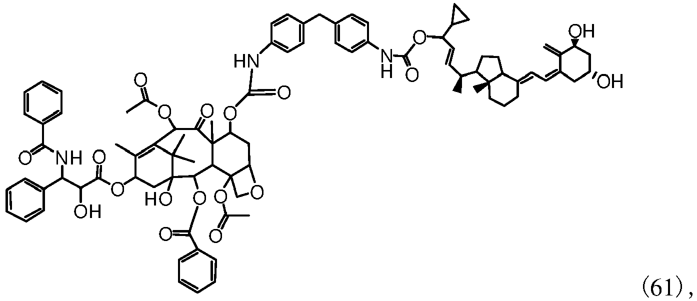

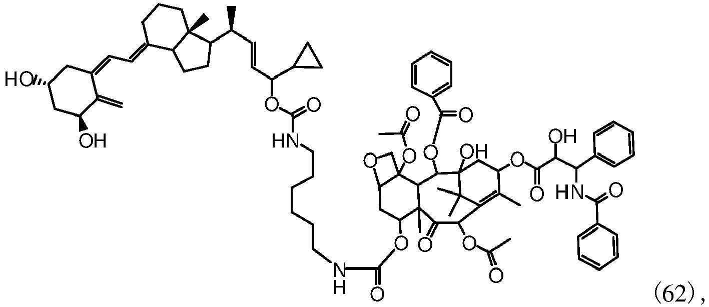

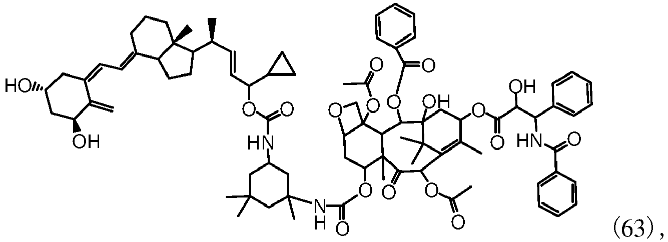

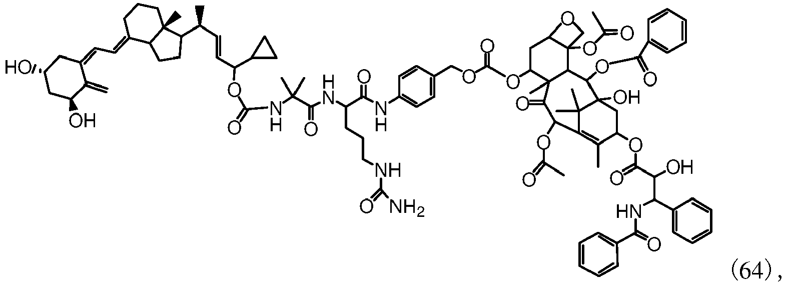

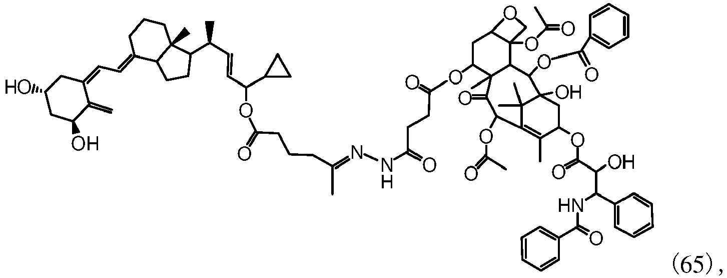

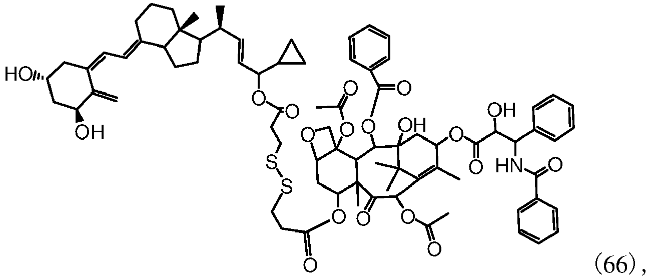

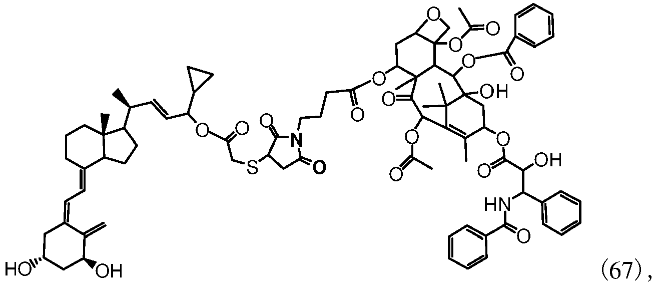

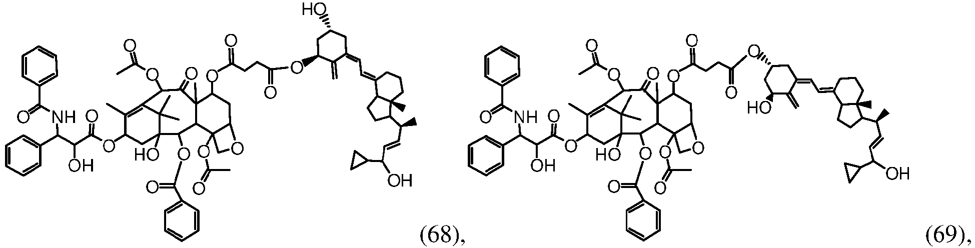

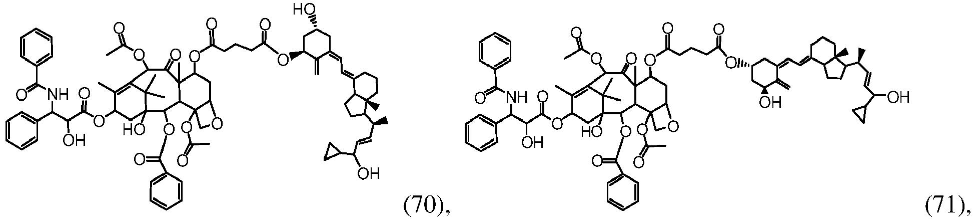

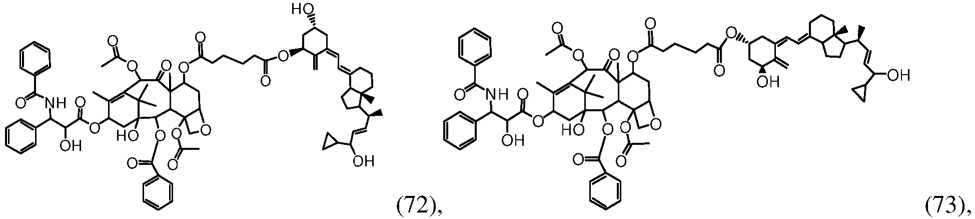

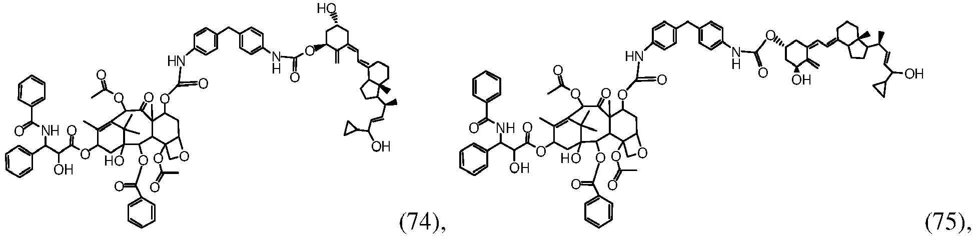

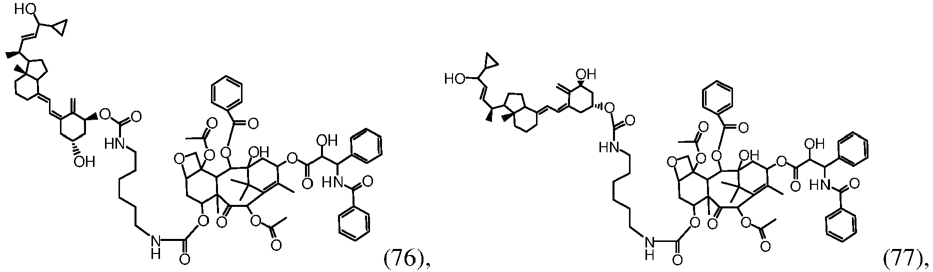

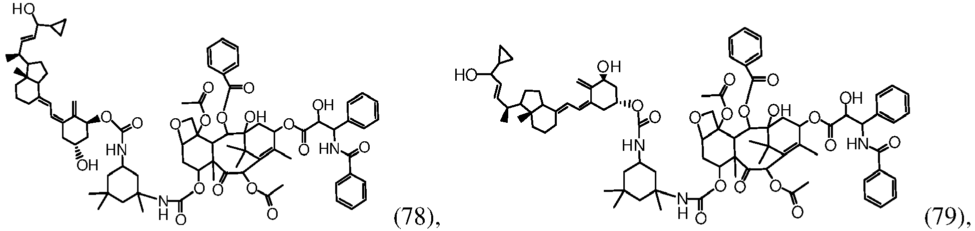

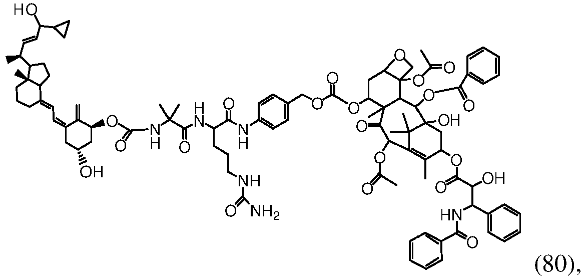

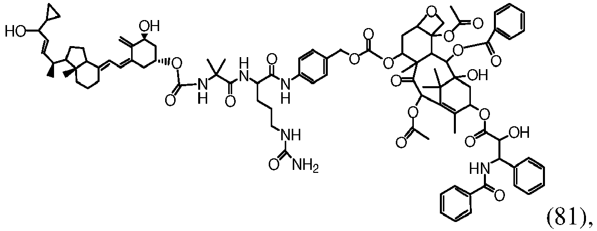

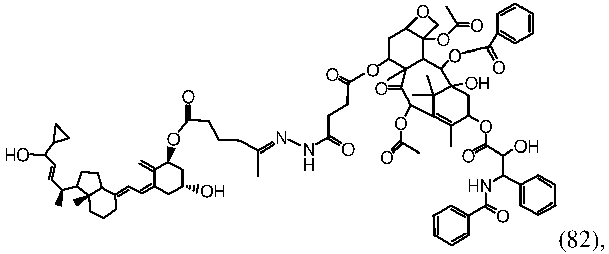

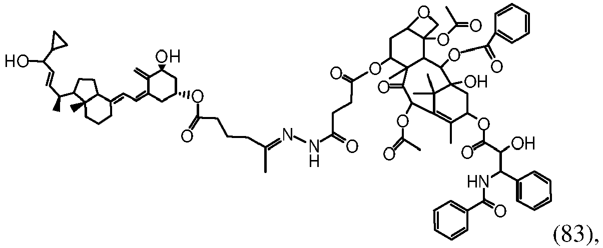

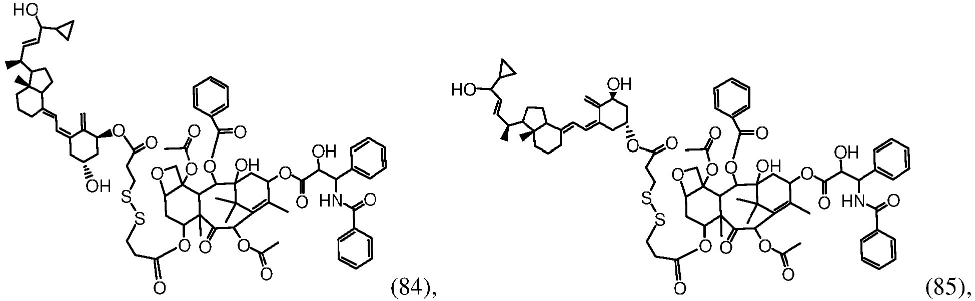

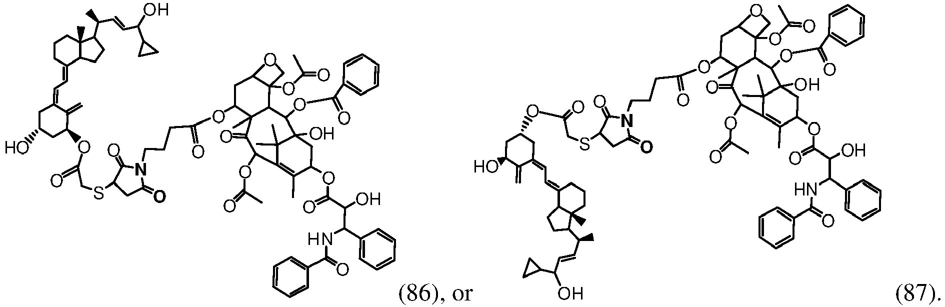

6. A compound, being a compound represented by formula (I), or being an isomer, a stereoisomer, a geometric isomer, a tautomer, a nitrogen oxide, a hydrate, a solvate, a metabolite, a pharmaceutically acceptable salt or a prodrug of the compound represented by formula (I),

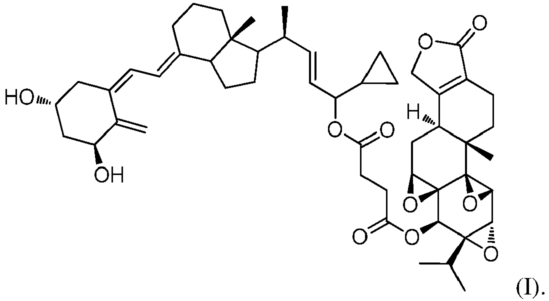

7. The compound according to claim 6, wherein the isomer of the compound represented by formula (I) has a structure represented by formula (II) or formula (III),

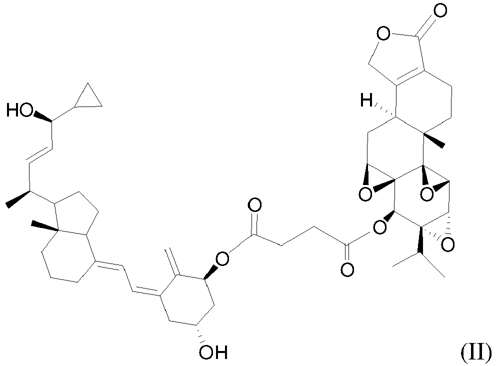

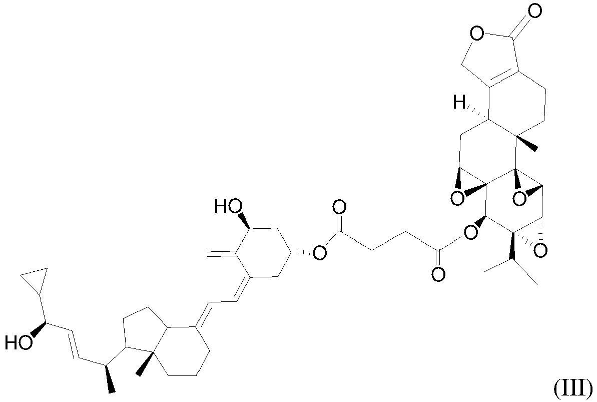

8. A pharmaceutical composition, comprising the compound according to any of claims 1 to 7 as an active ingredient.

9. The pharmaceutical composition according to claim 8, further comprising pharmaceutically acceptable excipients.

10. The pharmaceutical composition according to claim 8, wherein the pharmaceutical composition is formulated in a form of micelles, emulsion, albumin nanoparticles, liposomes, capsules, pills, tablets, granules, oral liquid, oral ointment, aerosol, or spray; and
preferably, the pharmaceutical composition is formulated in the form of micelles, and the micelles are formed by at least one of copolymer comprising polyethylene glycol-polylactic acid blocks, monomethoxy polyethylene glycol polylactic acid, monomethoxy polyethylene glycol polylactic acid glycolic acid copolymer, monomethoxy polyethylene glycol polycaprolactone, monomethoxy polyethylene glycol polytrimethylene carbonate, and monomethoxy polyethylene glycol polyamino acid.

11. The pharmaceutical composition according to claim 8, further comprising an additional anti-pancreatic cancer medicine, wherein the additional anti-pancreatic cancer medicine comprises one or more of 5-fluorouracil, Gemcitabine, FOLFIRINOX, nano-paclitaxel/Gemcitabine combination, and ONIVYDE™.

12. Use of the compound according to any one of claims 1 to 7 or the pharmaceutical composition according to any one of claims 8 to 11 in a preparation of a medicine for treating or preventing cancer.

13. The use according to claim 12, wherein the cancer is pancreatic cancer, liver cancer, breast cancer, skin cancer, prostate cancer, or fibroblastoma; and
preferably, the cancer is pancreatic cancer.

14. A method for preparing the compound according to any one of claims 1 to 7, the method comprising:
subjecting a first coupling component and a second coupling component to a ligation reaction,
wherein the first coupling component is configured to regulate tumor stroma, and the second coupling component is configured to kill tumor cells.

15. The method according to claim 14, wherein the ligation reaction is performed under a condition that the first coupling component, the second coupling component, and a linker coexist, and the linker is at least one of compounds represented by formula (88) to formula (97), 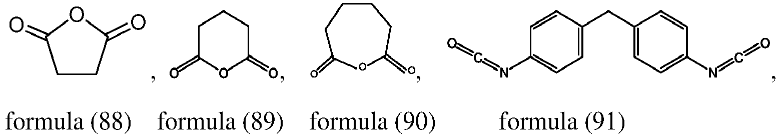 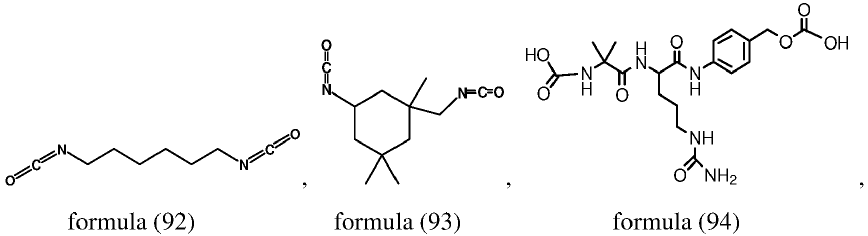 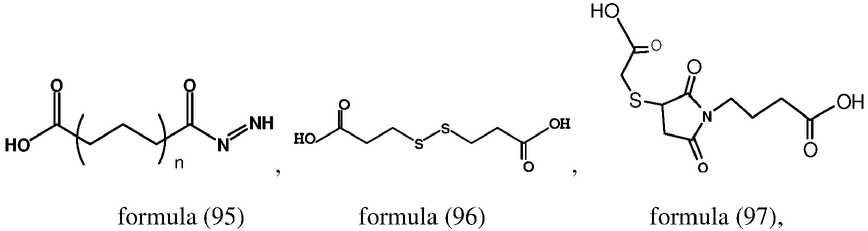 where, n is 1 to 10.

16. The method according to claim 15, wherein the first coupling component is calcipotriol, the second coupling component is triptolide, and the liner is the compound represented by formula (88).

17. The method according to claim 16, wherein a molar ratio of the first coupling component to the second coupling component is 1: 1.
